# EUROPEAN PATENT APPLICATION

(11) **EP 4 442 701 A1**
(43) Date of publication of application: **09.10.2024**
(21) Application number: 22897894.6
(22) Date of filing: 24.11.2022
(51) Int. Cl.: C07K 14/705, C07K 19/00, C12N 15/00, A61K 38/00, A61K 39/00, A61P 35/00

(54) **COMBINATION OF MOLECULAR SWITCH REGULATION TYPE CHIMERIC ANTIGEN RECEPTOR CELL AND ANTIBODY, AND USE THEREOF**

(30) Priority: 25.11.2021 CN 202111416497
(71) Applicant: Innovent Cells Pharmaceuticals (Suzhou) Co., Ltd., Suzhou, Jiangsu 215123 (CN)
(72) Inventor: XU, Wei, Suzhou, Jiangsu 215123 (CN); WEI, Huafeng, Suzhou, Jiangsu 215123 (CN); GUO, Shengjie, Suzhou, Jiangsu 215123 (CN); CHEN, Jianan, Suzhou, Jiangsu 215123 (CN); CHEN, Guozhi, Suzhou, Jiangsu 215123 (CN)
(74) Representative: Sagittarius IP
(86) International application number: PCT/CN2022/134109
(87) International publication number: WO 2023/093811

(57) **Abstract**

The present invention relates to a molecular switch regulation type chimeric antigen receptor polypeptide, containing a humanized anti-P329G mutant scFv sequence, a hinge region/spacer region, a transmembrane region, a costimulatory signal domain, and a stimulation signal domain; an immune effector cell engineered to express the molecular switch regulation type chimeric antigen receptor polypeptide; and a method for preparing the immune effector cell. The present invention further relates to a P329G mutant antibody capable of specifically binding CLDN18.2 molecules and a method for preparing the P329G mutant antibody. A pharmaceutical combination containing the immune effector cell and the P329G mutant antibody can be used for treating diseases related to CLDN18.2, such as cancer expressing or overexpressing CLDN18.2.

## Description

### TECHNICAL FIELD

The present invention relates generally to a combination of an immune effector cell (e.g., a T cell) engineered to express a molecular switch-regulated chimeric antigen receptor with an antibody and to use of the combination in the treatment of a disease related to CLDN18.2, such as a cancer expressing or overexpressing CLDN 18.2.

### BACKGROUND

Conventional chimeric antigen receptor T cells (CAR-T) achieve the purpose of recognizing and killing tumors by directly targeting surface antigens on tumor cells via chimeric antigen receptor (CAR) molecules on the T cells. The N-terminus of the chimeric antigen receptor comprises an antigen-binding domain that recognizes the antigen, such as a single-chain variable fragment (scFv) against the antigen. A drawback of the conventional chimeric antigen receptor T cells is that the CAR-T cells lack control over their activity and will continuously recognize and kill antigen-positive cells to which they are directed as long as these antigen-positive cells are present.

Since the majority of tumor antigens targeted by CAR-T cells are not tumor-specific, these tumor antigens are not only expressed on tumor cells but are also often expressed at low levels in many normal tissues, especially in important tissues and organs. The recognition and killing of the normal tissues by CAR-T cells leads to "On-target/off-tumor" toxicity, which may cause severe toxic side effects. For example, CAR-T cells targeting HER2, due to their recognition of lung epithelial cells expressing low levels of HER2, rapidly cause pulmonary edema and respiratory distress upon reinfusion into the body, leading to secondary multi-organ failure and even resulting in patient death (Morgan RA, Yang JC, Kitano M, et al., Case report of a serious adverse event following the administration of T cells transduced with a chimeric antigen receptor recognizing ERBB2[J] Mol Ther. 2010; 18(4): 843-851. doi: 10.1038/mt.2010.24). Similar pulmonary toxic side effects have also been reported in clinical trials of CAR-T cells targeting CEA, EGFRviii, and other targets. As another example, CAR-T cells targeting CD19 are used clinically to treat CD19-positive hematologic tumors. Due to the persistent presence of these CAR-T cells in the body, patients continue to experience a deficiency in their normal B cells (which express CD19) and their related humoral immune functions, even after the tumor cells have been cleared.

In order to reduce the "on-target/off-tumor" toxicity associated with CAR-T cells, the following strategies are generally employed in the prior art. One strategy is to design an antigen-binding domain contained in a CAR to target a target antigen that is highly expressed on the surface of tumor cells but not expressed or expressed at low levels in normal tissues, during the process of CAR design. Another strategy is to strictly control the dose of T cells administered, since excessive CAR-T cells, upon stimulation by an antigen, will proliferate exponentially and are more likely to cause on-target/off-tumor effects. Yet another strategy is to introduce an inducible suicide gene such as the inducible Caspase-9" (iCasp9) suicide gene into the construction of a CAR and, when on-target/off-tumor toxicity is observed in patients, administer AP1903 (a dimerizing chemical inducer that activates iCasp9) to cause apoptosis in CAR-T cells, thereby mitigating the toxicity (Zhang Huihui et al., Preclinical research on suicide gene as "safety switch" to control CAR-T cell toxicity, Chinese Journal of Cancer Biotherapy, 2021, 28(3): 225-231); there is also a report of using the herpes simplex virus thymidine kinase (HSV-TK) gene as a suicide gene to induce apoptosis in CAR-T cells through treatment with ganciclovir administration. Additionally, there is also a report of using an electroporation transfection method to make T cells transiently express CAR, exerting therapeutic effects through transient killing function (Kenderian SS et al., CD33-specific chimeric antigen receptor T cells exhibit potent preclinical activity against human acute myeloid leukemia[J] Leukemia. 2015; 29(8): 1637-1647).

Regarding the schemes reported in the prior art of using suicide genes as a "switch" to control the "on-target/off-tumor" toxicity of CAR-T cells, in the case of introducing iCasp9 into CAR-T cells, since AP1903 is an experimental drug, and experimenters must sign an agreement to obtain it from the supplier; in the case of introducing the HSV-TK gene into CAR-T cells, although ganciclovir has been used to control graft-versus-host disease in stem cell transplantation and has achieved certain clinical effects, the drug itself has immunogenicity, resulting in the elimination of cells other than the target cells (Traversari C et al., The potential immunogenicity of the TK suicide gene does not prevent full clinical benefit associated with the use of TK-transduced donor lymphocytes in HSCT for hematologic malignancies [J] Blood. 2007; 109(11): 4708-4715); furthermore, the mechanism of action of TK is to interfere with DNA synthesis during cell mitosis, acting only on dividing cells, and it takes several days or even weeks to achieve the desired effect. Therefore, there is still an urgent need in the art to develop other molecular switch-regulated chimeric antigen receptor cells for the treatment of cancer.

### SUMMARY

Through researches, the inventors have developed a novel set of molecular switch-regulated chimeric antigen receptor cells, and have constructed a CAR molecule by using an antibody with a Pro329Gly mutation (a mutation from proline to glycine at position 329 in an antibody Fc fragment according to the EU numbering, abbreviated as P329G) as a "molecular switch" or adaptor, wherein the CAR molecule is capable of specifically binding to an antibody containing a P329G mutated Fc domain but does not bind to an antibody lacking the P329G mutated Fc domain (also referred to herein as a "wild-type antibody"). Hence, an immune effector cell (e.g., a T cell) expressing said CAR is combined with the antibody as the "molecular switch" for the treatment of a disease related to CLDN18.2, such as a cancer expressing or overexpressing CLDN 18.2, for example, a CLDN18.2-positive solid tumor.

Therefore, in a first aspect, the present invention provides a molecular switch-regulated chimeric antigen receptor (CAR) polypeptide, comprising:
(1) a humanized anti-P329G mutation scFv sequence, wherein the scFv sequence comprises the following sequences that are capable of specifically binding to an antibody Fc domain comprising a P329G mutation, but not capable of specifically binding to an unmutated parent antibody Fc domain:
   (i) a heavy chain variable region, which comprises, according to the Kabat numbering,
      (a) a heavy chain complementarity determining region CDR H1 shown in the amino acid sequence RYWMN (SEQ ID NO: 54), or a variant of the CDR H1 with no more than 2 amino acid changes or no more than 1 amino acid change;
      (b) a CDR H2 shown in the amino acid sequence EITPDSSTINYAPSLKG (SEQ ID NO: 55), or a variant of the CDR H2 with no more than 2 amino acid changes or no more than 1 amino acid change; and
      (c) a CDR H3 shown in the amino acid sequence PYDYGAWFAS (SEQ ID NO: 56), or a variant of the CDR H3 with no more than 2 amino acid changes or no more than 1 amino acid change; and (ii) a light chain variable region, which comprises, according to the Kabat numbering,
      (d) a light chain complementarity determining region (CDR L) 1 shown in the amino acid sequence RSSTGAVTTSNYAN (SEQ ID NO: 57), or a variant of the CDR L1 with no more than 2 amino acid changes or no more than 1 amino acid change;
      (e) a CDR L2 shown in the amino acid sequence GTNKRAP (SEQ ID NO: 58), or a variant of the CDR L2 with no more than 2 amino acid changes or no more than 1 amino acid change; and
      (f) a CDR L3 shown in the amino acid sequence ALWYSNHWV (SEQ ID NO: 59), or a variant of the CDR L3 with no more than 2 amino acid changes or no more than 1 amino acid change;
   wherein the amino acid change is an addition, deletion or substitution of an amino acid;
(2) a hinge region/spacer region selected from:
   (i) a (G₄S)ₙ, (SG₄)ₙ or G₄(SG₄)ₙ peptide linker, wherein "n" is an integer of 1 to 10, such as an integer of 2 to 4, for example, the sequences set forth in SEQ ID NO: 4 and SEQ ID NO: 5;
   (ii) an IgG4 spacer region (SEQ ID NO: 6; ESKYGPPCPPCP), or a spacer region having at least 80% sequence identity thereto; and
   (iii) a CD28 hinge region (SEQ ID NO: 7; IEVMYPPPYLDNEKSNGTIIHVKGKHLCPSPLFPGPSKP), or a spacer region having at least 80% sequence identity thereto.
(3) a transmembrane region (TM) selected from:
   (i) a CD28 transmembrane domain or a variant thereof with 1-5 amino acid modifications, for example, the sequence set forth in SEQ ID NO: 8 or a variant thereof with 1-2 amino acid modifications; and
   (ii) a CD8 transmembrane domain or a variant thereof with 1-5 amino acid modifications, for example, the sequence set forth in SEQ ID NO: 9 or a variant thereof with 1-2 amino acid modifications;
(4) a co-stimulatory signaling domain (CSD) selected from:
   (i) a 4-1BB co-stimulatory domain or a variant thereof with 1-5 amino acid modifications, for example, the sequence set forth in SEQ ID NO: 11 or a variant thereof with 1-2 amino acid modifications; and
   (ii) a CD28 co-stimulatory domain or a variant thereof with 1-5 amino acid modifications, for example, the sequence set forth in SEQ ID NO: 10 or a variant thereof with 1-2 amino acid modifications, e.g., the sequence set forth in SEQ ID NO: 12; and
(5) a stimulatory signaling domain (SSD), which is a CD3ζ signaling domain or a variant thereof with 1-10 amino acid modifications, for example, the sequence set forth in SEQ ID NO: 13 or a variant thereof with 1-10 or 1-5 amino acid modifications;
   wherein the amino acid modification is an addition, deletion or substitution of an amino acid.

In some embodiments, the present invention provides a molecular switch-regulated chimeric antigen receptor (CAR) polypeptide, comprising:
(1) a humanized anti-P329G mutation scFv sequence, wherein the scFv sequence comprises the following sequences that are capable of specifically binding to an antibody Fc domain comprising a P329G mutation, but not capable of specifically binding to an unmutated parent antibody Fc domain:
   (i) a heavy chain variable region, which comprises, according to the Kabat numbering,
      (a) a CDR H1 shown in the amino acid sequence RYWMN (SEQ ID NO: 54);
      (b) a CDR H2 shown in the amino acid sequence EITPDSSTINYAPSLKG (SEQ ID NO: 55); and
      (c) a CDR H3 shown in the amino acid sequence PYDYGAWFAS (SEQ ID NO: 56); and
   (ii) a light chain variable region, which comprises, according to the Kabat numbering,
      (d) a CDR L1 shown in the amino acid sequence RSSTGAVTTSNYAN (SEQ ID NO: 57);
      (e) a CDR L2 shown in the amino acid sequence GTNKRAP (SEQ ID NO: 58); and
      (f) a CDR L3 shown in the amino acid sequence ALWYSNHWV (SEQ ID NO: 59);
   for example, (i) a heavy chain variable region comprising the sequence of SEQ ID NO: 2 or a sequence having at least 90%, 91%, 92%, 93%, 94%, 95%, 96%, 97%, 98% or 99% identity thereto, and
   (ii) a light chain variable region comprising the sequence of SEQ ID NO: 3 or a sequence having at least 90%, 91%, 92%, 93%, 94%, 95%, 96%, 97%, 98% or 99% identity thereto;
   for example, (i) a heavy chain variable region comprising the sequence of SEQ ID NO: 2, and
   (ii) a light chain variable region comprising the sequence of SEQ ID NO: 3;
(2) a hinge region/spacer region selected from:
   (i) a (G₄S)ₙ, (SG₄)ₙ or G₄(SG₄)ₙ peptide linker, wherein "n" is an integer of 1 to 10, such as an integer of 2 to 4, for example, the sequences set forth in SEQ ID NO: 4 and SEQ ID NO: 5;
   (ii) an IgG4 spacer region (SEQ ID NO: 6; ESKYGPPCPPCP), or a spacer region having at least 90% sequence identity thereto; and
   (iii) a CD28 hinge region (SEQ ID NO: 7; IEVMYPPPYLDNEKSNGTIIHVKGKHLCPSPLFPGPSKP), or a spacer region having at least 90% or at least 95% sequence identity thereto.
(3) a transmembrane region (TM) selected from:
   (i) a CD28 transmembrane domain set forth in SEQ ID NO: 8 or a variant thereof with 1 amino acid modification; and
   (ii) a CD8 transmembrane domain set forth in SEQ ID NO: 9 or a variant thereof with 1 amino acid modification;
(4) a co-stimulatory signaling domain (CSD) selected from:
   (i) a 4-1BB co-stimulatory domain set forth in SEQ ID NO: 11 or a variant thereof with 1 amino acid modification; and
   (ii) a CD28 co-stimulatory domain set forth in SEQ ID NO: 10 or a variant thereof with 1 amino acid modification, for example, the sequence set forth in SEQ ID NO: 12; and
(5) a stimulatory signaling domain (SSD), which is a CD3ζ signaling domain set forth in SEQ ID NO: 13 or a variant thereof with 1 amino acid modification;
   wherein the amino acid modification is an addition, deletion or substitution of an amino acid.

In some embodiments, the molecular switch-regulated CAR polypeptide of the present invention further comprises a signal peptide sequence located at the N-terminus, such as the signal peptide sequence set forth in SEQ ID NO: 1.

In some embodiments, the molecular switch-regulated CAR polypeptide of the present invention has the amino acid sequence set forth in SEQ ID NO: 21, 23 or 25.

In some embodiments, the molecular switch-regulated CAR polypeptide of the present invention further comprises a membrane-proximal intracellular domain located between the transmembrane region (TM) and the co-stimulatory signaling domain (CSD), for example, a CD3ε chain membrane-proximal intracellular signaling domain, such as a CD3ε chain membrane-proximal intracellular signaling domain as set forth in SEQ ID NO: 14. In a second aspect, the present invention provides a nucleic acid encoding the molecular switch-regulated CAR polypeptide as described herein, a vector comprising the nucleic acid encoding the CAR polypeptide described herein, and a cell comprising the CAR nucleic acid molecule or vector described herein, or a cell expressing the CAR polypeptide described herein, wherein preferably, the cell is an autologous T cell or an allogeneic T cell.

In some embodiments, the nucleic acid molecule encoding the molecular switch-regulated CAR polypeptide of the present invention comprises the nucleotide sequence set forth in SEQ ID NO: 22, 24, or 26.

In one embodiment, the present invention utilizes a human PBMC to prepare a primary P329G CAR-T cell. The CAR-T cell transduced with the CAR molecule of the present invention has an effector function *in vitro* and has sustained killing activity against target cells *in vitro.*

In a third aspect, the present invention provides a method for producing a cell, such as an immune effector cell, comprising introducing (e.g., transducing) an immune effector cell with a nucleic acid molecule (e.g., an RNA molecule, such as an mRNA molecule) encoding the CAR polypeptide described herein, or a vector comprising the nucleic acid molecule encoding the CAR polypeptide described herein.

In some embodiments, the immune effector cell is a T cell; for example, the T cell is an autologous T cell or an allogeneic T cell; for example, the immune effector cell is prepared from a T cell isolated from a human PBMC.

In a fourth aspect, the present invention provides a pharmaceutical combination, comprising (i) an immune effector cell (e.g., a T cell or an NK cell) expressing the CAR polypeptide according to the first aspect of the present invention, the nucleic acid molecule encoding the CAR polypeptide or the vector according to the second aspect, or any combination thereof, and (ii) an antibody or an antigen-binding fragment specifically binding to a CLDN18.2 molecule and comprising a P329G mutation (also referred to as a "P329G mutated antibody", a "PG antibody", or a "PG Ab"), wherein, for example, the P329G mutated antibody comprises a mutated Fc domain, wherein the amino acid at position P329 according to the EU numbering is mutated to glycine (G), and the binding of the mutated Fc domain to an Fcy receptor is reduced compared to the binding of an unmutated parent antibody Fc domain to the Fcy receptor; as well as, optionally, a pharmaceutically acceptable supplementary material.

In some embodiments, the antibody or the antigen-binding fragment specifically binding to the CLDN18.2 molecule described herein comprises a heavy chain variable region and a light chain variable region, wherein
(a) the heavy chain variable region comprises, according to the Kabat numbering, a CDR H1 shown in the amino acid sequence SYVMS (SEQ ID NO: 60), or a variant of the CDR H1 with no more than 2 amino acid changes or no more than 1 amino acid change; a CDR H2 shown in the amino acid sequence TISHSGGSTYYADSVKG (SEQ ID NO: 61), or a variant of the CDR H2 with no more than 2 amino acid changes or no more than 1 amino acid change; and a CDR H3 shown in the amino acid sequence DAPYYDILTGYRY (SEQ ID NO: 62), or a variant of the CDR H3 with no more than 2 amino acid changes or no more than 1 amino acid change; and the light chain variable region comprises, according to the Kabat numbering, a CDR L1 shown in the amino acid sequence RASQSISSWLA (SEQ ID NO: 63), or a variant of the CDR L1 with no more than 2 amino acid changes or no more than 1 amino acid change; a CDR L2 shown in the amino acid sequence KASSLES (SEQ ID NO: 64), or a variant of the CDR L2 with no more than 2 amino acid changes or no more than 1 amino acid change; and a CDR L3 shown in the amino acid sequence QQYNSYSYT (SEQ ID NO: 65), or a variant of the CDR L3 with no more than 2 amino acid changes or no more than 1 amino acid change;
(b) the heavy chain variable region comprises, according to the Kabat numbering, a CDR H1 shown in the amino acid sequence SYNIH (SEQ ID NO: 66), or a variant of the CDR H1 with no more than 2 amino acid changes or no more than 1 amino acid change; a CDR H2 shown in the amino acid sequence YIAPFQGDSRYNQKFKG (SEQ ID NO: 67), or a variant of the CDR H2 with no more than 2 amino acid changes or no more than 1 amino acid change; and a CDR H3 shown in the amino acid sequence LNRGNSLDY (SEQ ID NO: 68), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 69), or a variant of the CDR L1 with no more than 2 amino acid changes or no more than 1 amino acid change; a CDR L2 shown in the amino acid sequence WASTRES (SEQ ID NO: 70), or a variant of the CDR L2 with no more than 2 amino acid changes or no more than 1 amino acid change; and a CDR L3 shown in the amino acid sequence QNNYIYPLT (SEQ ID NO: 71), or a variant of the CDR L3 with no more than 2 amino acid changes or no more than 1 amino acid change;
(c) the heavy chain variable region comprises, according to the Kabat numbering, a CDR H1 shown in the amino acid sequence SYNIH (SEQ ID NO: 72), or a variant of the CDR H1 with no more than 2 amino acid changes or no more than 1 amino acid change; a CDR H2 shown in the amino acid sequence YIAPFQGDARYNQKFKG (SEQ ID NO: 73), or a variant of the CDR H2 with no more than 2 amino acid changes or no more than 1 amino acid change; and a CDR H3 shown in the amino acid sequence LNRGQSLDY (SEQ ID NO: 74), or a variant of the CDR H3 with no more than 2 amino acid changes or no more than 1 amino acid change; and the light chain variable region comprises, according to the Kabat numbering, a CDR L1 shown in the amino acid sequence KSSQSLFNAGNQRNYLT (SEQ ID NO: 75), or a variant of the CDR L1 with no more than 2 amino acid changes or no more than 1 amino acid change; a CDR L2 shown in the amino acid sequence WASTRES (SEQ ID NO: 76), or a variant of the CDR L2 with no more than 2 amino acid changes or no more than 1 amino acid change; and a CDR L3 shown in the amino acid sequence QNNYIYPLT (SEQ ID NO: 77), or a variant of the CDR L3 with no more than 2 amino acid changes or no more than 1 amino acid change;
(d) the heavy chain variable region comprises, according to the Kabat numbering, a CDR H1 shown in the amino acid sequence SYNIH (SEQ ID NO: 78), or a variant of the CDR H1 with no more than 2 amino acid changes or no more than 1 amino acid change; a CDR H2 shown in the amino acid sequence YIAPFQGDARYNQKFKG (SEQ ID NO: 79), or a variant of the CDR H2 with no more than 2 amino acid changes or no more than 1 amino acid change; and a CDR H3 shown in the amino acid sequence LNRGNALDY (SEQ ID NO: 80), or a variant of the CDR H3 with no more than 2 amino acid changes or no more than 1 amino acid change; and the light chain variable region comprises, according to the Kabat numbering, a CDR L1 shown in the amino acid sequence KSSQSLFQSGNQRNYLT (SEQ ID NO: 81), or a variant of the CDR L1 with no more than 2 amino acid changes or no more than 1 amino acid change; a CDR L2 shown in the amino acid sequence WASTRES (SEQ ID NO: 82), or a variant of the CDR L2 with no more than 2 amino acid changes or no more than 1 amino acid change; and a CDR L3 shown in the amino acid sequence QNNYIYPLT (SEQ ID NO: 83), or a variant of the CDR L3 with no more than 2 amino acid changes or no more than 1 amino acid change;
(e) the heavy chain variable region comprises, according to the Kabat numbering, a CDR H1 shown in the amino acid sequence SYNIS (SEQ ID NO: 84), or a variant of the CDR H1 with no more than 2 amino acid changes or no more than 1 amino acid change; a CDR H2 shown in the amino acid sequence YIAPFQGDARYNQKFKG (SEQ ID NO: 85), or a variant of the CDR H2 with no more than 2 amino acid changes or no more than 1 amino acid change; and a CDR H3 shown in the amino acid sequence LNRGNSLDY (SEQ ID NO: 86), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 87), or a variant of the CDR L1 with no more than 2 amino acid changes or no more than 1 amino acid change; a CDR L2 shown in the amino acid sequence WASTRES (SEQ ID NO: 88), or a variant of the CDR L2 with no more than 2 amino acid changes or no more than 1 amino acid change; and a CDR L3 shown in the amino acid sequence QNNYIYPLT (SEQ ID NO: 89), or a variant of the CDR L3 with no more than 2 amino acid changes or no more than 1 amino acid change; or
(f) the heavy chain variable region comprises, according to the Kabat numbering, a CDR H1 shown in the amino acid sequence TYWMH (SEQ ID NO: 90), or a variant of the CDR H1 with no more than 2 amino acid changes or no more than 1 amino acid change; a CDR H2 shown in the amino acid sequence LIDPSDSETRLNQKFKD (SEQ ID NO: 91), or a variant of the CDR H2 with no more than 2 amino acid changes or no more than 1 amino acid change; and a CDR H3 shown in the amino acid sequence WGQGTLVTVSS (SEQ ID NO: 92), or a variant of the CDR H3 with no more than 2 amino acid changes or no more than 1 amino acid change; and the light chain variable region comprises, according to the Kabat numbering, a CDR L1 shown in the amino acid sequence KSSQSLLNSGNQKNYLT (SEQ ID NO: 93), or a variant of the CDR L1 with no more than 2 amino acid changes or no more than 1 amino acid change; a CDR L2 shown in the amino acid sequence WASTRES (SEQ ID NO: 94), or a variant of the CDR L2 with no more than 2 amino acid changes or no more than 1 amino acid change; and a CDR L3 shown in the amino acid sequence QNDYSYPLT (SEQ ID NO: 95), or a variant of the CDR L3 with no more than 2 amino acid changes or no more than 1 amino acid change;
wherein the amino acid change is an addition, deletion or substitution of an amino acid.

In some embodiments, the present invention provides an antibody or an antigen-binding fragment specifically binding to a CLDN18.2 molecule, comprising a heavy chain variable region and a light chain variable region, wherein
(a) the heavy chain variable region comprises, according to the Kabat numbering, a CDR H1 shown in the amino acid sequence SYVMS (SEQ ID NO: 60); a CDR H2 shown in the amino acid sequence TISHSGGSTYYADSVKG (SEQ ID NO: 61); and a CDR H3 shown in the amino acid sequence DAPYYDILTGYRY (SEQ ID NO: 62); and the light chain variable region comprises, according to the Kabat numbering, a CDR L1 shown in the amino acid sequence RASQSISSWLA (SEQ ID NO: 63); a CDR L2 shown in the amino acid sequence KASSLES (SEQ ID NO: 64); and a CDR L3 shown in the amino acid sequence QQYNSYSYT (SEQ ID NO: 65);
(b) the heavy chain variable region comprises, according to the Kabat numbering, a CDR H1 shown in the amino acid sequence SYNIH (SEQ ID NO: 66); a CDR H2 shown in the amino acid sequence YIAPFQGDSRYNQKFKG (SEQ ID NO: 67); and a CDR H3 shown in the amino acid sequence LNRGNSLDY (SEQ ID NO: 68); and the light chain variable region comprises, according to the Kabat numbering, a CDR L1 shown in the amino acid sequence KSSQSLFNSGNQRNYLT (SEQ ID NO: 69); a CDR L2 shown in the amino acid sequence WASTRES (SEQ ID NO: 70); and a CDR L3 shown in the amino acid sequence QNNYIYPLT (SEQ ID NO: 71);
(c) the heavy chain variable region comprises, according to the Kabat numbering, a CDR H1 shown in the amino acid sequence SYNIH (SEQ ID NO: 72); a CDR H2 shown in the amino acid sequence YIAPFQGDARYNQKFKG (SEQ ID NO: 73); and a CDR H3 shown in the amino acid sequence LNRGQSLDY (SEQ ID NO: 74); and the light chain variable region comprises, according to the Kabat numbering, a CDR L1 shown in the amino acid sequence KSSQSLFNAGNQRNYLT (SEQ ID NO: 75); a CDR L2 shown in the amino acid sequence WASTRES (SEQ ID NO: 76); and a CDR L3 shown in the amino acid sequence QNNYIYPLT (SEQ ID NO: 77);
(d) the heavy chain variable region comprises, according to the Kabat numbering, a CDR H1 shown in the amino acid sequence SYNIH (SEQ ID NO: 78); a CDR H2 shown in the amino acid sequence YIAPFQGDARYNQKFKG (SEQ ID NO: 79); and a CDR H3 shown in the amino acid sequence LNRGNALDY (SEQ ID NO: 80); and the light chain variable region comprises, according to the Kabat numbering, a CDR L1 shown in the amino acid sequence KSSQSLFQSGNQRNYLT (SEQ ID NO: 81); a CDR L2 shown in the amino acid sequence WASTRES (SEQ ID NO: 82); and a CDR L3 shown in the amino acid sequence QNNYIYPLT (SEQ ID NO: 83);
(e) the heavy chain variable region comprises, according to the Kabat numbering, a CDR H1 shown in the amino acid sequence SYNIS (SEQ ID NO: 84); a CDR H2 shown in the amino acid sequence YIAPFQGDARYNQKFKG (SEQ ID NO: 85); and a CDR H3 shown in the amino acid sequence LNRGNSLDY (SEQ ID NO: 86); and the light chain variable region comprises, according to the Kabat numbering, a CDR L1 shown in the amino acid sequence KSSQSLFNSGNQRNYLT (SEQ ID NO: 87); a CDR L2 shown in the amino acid sequence WASTRES (SEQ ID NO: 88); and a CDR L3 shown in the amino acid sequence QNNYIYPLT (SEQ ID NO: 89); or
(f) the heavy chain variable region comprises, according to the Kabat numbering, a CDR H1 shown in the amino acid sequence TYWMH (SEQ ID NO: 90); a CDR H2 shown in the amino acid sequence LIDPSDSETRLNQKFKD (SEQ ID NO: 91); and a CDR H3 shown in the amino acid sequence NRWLLG (SEQ ID NO: 92); and the light chain variable region comprises, according to the Kabat numbering, a CDR L1 shown in the amino acid sequence KSSQSLLNSGNQKNYLT (SEQ ID NO: 93); a CDR L2 shown in the amino acid sequence WASTRES (SEQ ID NO: 94); and a CDR L3 shown in the amino acid sequence QNDYSYPLT (SEQ ID NO: 95).

In some embodiments, the present invention obtains an antibody or an antigen-binding fragment specifically binding to a CLDN18.2 molecule, comprising a heavy chain variable region and a light chain variable region, wherein
(a) the heavy chain variable region comprises the sequence of SEQ ID NO: 34 or a sequence having at least 90%, 91%, 92%, 93%, 94%, 95%, 96%, 97%, 98% or 99% identity thereto, and the light chain variable region comprises the sequence of SEQ ID NO: 35 or a sequence having at least 90%, 91%, 92%, 93%, 94%, 95%, 96%, 97%, 98% or 99% identity thereto;
(b) the heavy chain variable region comprises the sequence of SEQ ID NO: 36 or a sequence having at least 90%, 91%, 92%, 93%, 94%, 95%, 96%, 97%, 98% or 99% identity thereto, and the light chain variable region comprises the sequence of SEQ ID NO: 37 or a sequence having at least 90%, 91%, 92%, 93%, 94%, 95%, 96%, 97%, 98% or 99% identity thereto;
(c) the heavy chain variable region comprises the sequence of SEQ ID NO: 44 or a sequence having at least 90%, 91%, 92%, 93%, 94%, 95%, 96%, 97%, 98% or 99% identity thereto, and the light chain variable region comprises the sequence of SEQ ID NO: 45 or a sequence having at least 90%, 91%, 92%, 93%, 94%, 95%, 96%, 97%, 98% or 99% identity thereto;
(d) the heavy chain variable region comprises the sequence of SEQ ID NO: 46 or a sequence having at least 90%, 91%, 92%, 93%, 94%, 95%, 96%, 97%, 98% or 99% identity thereto, and the light chain variable region comprises the sequence of SEQ ID NO: 47 or a sequence having at least 90%, 91%, 92%, 93%, 94%, 95%, 96%, 97%, 98% or 99% identity thereto;
(e) the heavy chain variable region comprises the sequence of SEQ ID NO: 48 or a sequence having at least 90%, 91%, 92%, 93%, 94%, 95%, 96%, 97%, 98% or 99% identity thereto, and the light chain variable region comprises the sequence of SEQ ID NO: 49 or a sequence having at least 90%, 91%, 92%, 93%, 94%, 95%, 96%, 97%, 98% or 99% identity thereto; or
(f) the heavy chain variable region comprises the sequence of SEQ ID NO: 42 or a sequence having at least 90%, 91%, 92%, 93%, 94%, 95%, 96%, 97%, 98% or 99% identity thereto, and the light chain variable region comprises the sequence of SEQ ID NO: 43 or a sequence having at least 90%, 91%, 92%, 93%, 94%, 95%, 96%, 97%, 98% or 99% identity thereto.

In some embodiments, the present invention obtains an antibody or an antigen-binding fragment specifically binding to a CLDN18.2 molecule, comprising a heavy chain variable region and a light chain variable region, wherein
(a) the heavy chain variable region comprises the sequence of SEQ ID NO: 34, and the light chain variable region comprises the sequence of SEQ ID NO: 35;
(b) the heavy chain variable region comprises the sequence of SEQ ID NO: 36, and the light chain variable region comprises the sequence of SEQ ID NO: 37;
(c) the heavy chain variable region comprises the sequence of SEQ ID NO: 44, and the light chain variable region comprises the sequence of SEQ ID NO: 45;
(d) the heavy chain variable region comprises the sequence of SEQ ID NO: 46, and the light chain variable region comprises the sequence of SEQ ID NO: 47;
(e) the heavy chain variable region comprises the sequence of SEQ ID NO: 48, and the light chain variable region comprises the sequence of SEQ ID NO: 49; or
(f) the heavy chain variable region comprises the sequence of SEQ ID NO: 42, and the light chain variable region comprises the sequence of SEQ ID NO: 43;
in some embodiments, the antibody specifically binding to the CLDN18.2 molecule disclosed herein is an IgG1, IgG2, IgG3 or IgG4 antibody, preferably an IgG1 or IgG4 antibody, more preferably an IgG1 antibody.

In some embodiments, the antigen-binding fragment of the antibody specifically binding to the CLDN18.2 molecule disclosed herein is a Fab, a Fab', a F(ab')₂, an Fv, a single-chain Fv, a single-chain Fab, or a diabody. For the antibody or the antigen-binding fragment specifically binding to the CLDN18.2 molecule, an antibody having a mutated Fc domain is obtained by mutating the amino acid at position P329 according to the EU numbering to glycine (G), wherein the binding of the mutated Fc domain to an Fcy receptor is reduced compared to the binding of an unmutated parent antibody Fc domain to the Fcy receptor.

In some embodiments, the mutated Fc domain is a mutated Fc domain of an IgG1, IgG2, IgG3 or IgG4 antibody, preferably a mutated Fc domain of an IgG1 or IgG4 antibody, more preferably a mutated Fc domain of an IgG1 antibody;
in some embodiments, the antibody or the antigen-binding fragment specifically binding to the CLDN18.2 molecule comprises the heavy chain constant region sequence set forth in SEQ ID NO: 39 or a sequence having at least 90%, 91%, 92%, 93%, 94%, 95%, 96%, 97%, 98% or 99% identity thereto, wherein the amino acid at position P329 according to the EU numbering is mutated to G;
for example, the antibody or the antigen-binding fragment comprises the heavy chain constant region sequence set forth in SEQ ID NO: 39 or a sequence having at least 90%, 91%, 92%, 93%, 94%, 95%, 96%, 97%, 98% or 99% identity thereto, wherein the amino acid at position P329 according to the EU numbering is mutated to G; and the light chain constant region sequence set forth in SEQ ID NO: 40 or a sequence having at least 90%, 91%, 92%, 93%, 94%, 95%, 96%, 97%, 98%, or 99% identity thereto.

In some embodiments, (i) the immune effector cell is a T cell expressing the molecular switch-regulated CAR polypeptide according to the first aspect of the present invention prepared from an autologous T cell or an allogeneic T cell, for example, the immune effector cell is a T cell expressing the molecular switch-regulated CAR polypeptide according to the first aspect of the present invention prepared from a T cell isolated from a human PBMC; (ii) the P329G mutated antibody is an HB37A6 PG Ab (also referred to herein simply as an "A6 PG Ab", an "A6 PG antibody", or a "P329G A6 antibody"), an Hz69H9 PG Ab (also referred to herein simply as an "H9 PG Ab" or an "H9 PG antibody"), an Hz69H9-1.2 PG Ab (also referred to herein simply as an "H9-1.2 PG Ab" or an "H9-1.2 PG antibody"), an Hz69H9-2.1 PG Ab (also referred to herein simply as an "H9-2.1 PG" or an "H9-2.1 PG antibody"), an Hz69H9-SA PG Ab (also referred to herein simply as an "H9-SA PG Ab" or an "H9-SA PG antibody"), and/or an Hz3G3 PG Ab (also referred to herein simply as a "G3 PG Ab" or a "G3 PG antibody").

In one embodiment, the present invention used primary P329G CAR-T cells prepared from human PBMCs and utilized a P329G mutated anti-CLDN18.2 humanized antibody to evaluate the effector function of the P329G CAR-T cells against a Claudin18.2 (also known as CLDN18.2) tumor cell antigen exerted through antibody dependence in an *in-vitro* co-culture system; based on this, the P329G mutated anti-CLDN18.2 antibody can act as a "molecular switch" to regulate the recognition and killing activity of the P329G CAR-T cells against CLDN18.2-positive tumor cells; this *in-vitro* effector function is comparable to that of conventional CAR-T cells directly targeting CLDN18.2-positive tumor cells, but the activity of the conventional CAR-T cells does not depend on the P329G mutated antibody.

In other embodiments, the present invention validated the anti-tumor effect of the P329G CAR-T cells in combination with the P329G mutated antibody in immunodeficient mice inoculated with tumors derived from human tumor cell lines positive for CLDN18.2 expression, and explored the antibody administration dose, interval, etc.; although increasing the dose of the P329G mutated antibody induced a toxic response, the mice were able to tolerate the toxic response, and the body weight of the mice returned to normal after discontinuation of the antibody administration; in contrast, the corresponding conventional CAR-T cells induced severe toxic side effects at the same dose of cell reinfusion, leading to the death of the mice within 1 week.

In still other embodiments, the present invention monitored the *in-vivo* persistence of the P329G CAR-T cells in immunodeficient mice inoculated with tumors derived from human tumor cell lines positive for CLDN18.2 expression using flow cytometry. The pharmaceutical combination of the P329G CAR-T cells combined with the P329G mutated antibody disclosed herein is capable of significantly expanding the P329G CAR-T cells *in vivo* and exhibits a slight antibody dose-dependence.

In some embodiments, primary CAR-T cells expressing the CAR molecules of the present invention (e.g., HuR968B CAR and HuR9684M CAR) in combination with a P329G mutated anti-CLDN18.2 humanized antibody demonstrated significantly better performance than CAR molecules in the prior art in *in-vitro* repeated killing experiments; the primary CAR-T cells expressing the CAR molecules of the present invention (e.g., HuR968B CAR and HuR9684M CAR) possess the ability to continuously kill tumor cells while maintaining the specificity of the CAR molecules; in the co-culture experiments with tumor cells, the CAR-T cells of the present invention were able to be activated, proliferate, secrete effector cytokines and produce a killing effect on the tumor cells only in the presence of the P329G mutated antibody, whereas WT antibodies without the P329G mutation were unable to stimulate the killing effect function of the P329G CAR-T cells on the tumor cells.

In some embodiments, the pharmaceutical combination of the present invention utilizes P329G mutated antibody molecules with different affinities; by detecting the functions of P329G CAR-T cells, it was found that the affinity of the P329G mutated antibody can affect the specific recognition effect and the "on-target/off-tumor" effect of the P329G CAR-T cells.

In some embodiments, the use of an affinity-reduced anti-CLDN18.2 antibody in the pharmaceutical combination of the present invention is capable of maintaining the anti-tumor effect while reducing the toxic side effects caused by "on-target/off-tumor" recognition by CAR-T cells.

In some embodiments, (i) in the pharmaceutical combination of the present invention is administered intravenously at a dose of 1 × 10⁶-1 × 10¹² immune effector cells, preferably 5 × 10⁶-1 × 10¹¹ immune effector cells, more preferably 1 × 10⁷-1 × 10¹⁰ immune effector cells, such as 5 × 10⁷ immune effector cells, 2.5 × 10⁸ cells, 7.5 × 10⁸ immune effector cells, or 1.25 × 10⁹ immune effector cells, either in a single administration or in multiple administrations; and
(ii) is administered in the form of a dose unit of 0.1-10 mg/kg, preferably 0.3 mg/kg, 0.5 mg/kg, 1 mg/kg, 3 mg/kg, 5 mg/kg, 7 mg/kg, or 9 mg/kg, preferably as a parenteral dosage form, more preferably as an intravenous dosage form.

In some embodiments, (i) and (ii) in the pharmaceutical combination of the present invention are administered separately, simultaneously, or sequentially.

In some embodiments, (i) and (ii) in the pharmaceutical combination of the present invention are administered separately; for example, (i) is administered intravenously on the first day, (ii) is administered on the second day, and then (ii) is administered multiple times at a certain frequency, while an *in-vivo* concentration of (i) and a desired therapeutic efficacy endpoint are monitored to determine whether to administer (i) multiple times; or (ii) is administered on the first day, (i) is administered intravenously on the second day, and then (ii) is administered multiple times at a certain frequency, while an *in-vivo* concentration of (i) and a desired therapeutic efficacy endpoint are monitored to determine whether to administer (i) multiple times.

In some embodiments, (i) and (ii) in the pharmaceutical combination of the present invention are each administered once, and then (ii) is administered multiple times at a frequency of once every 3-4 days, once a week, once every two weeks, once every three weeks, or once every four weeks, while the *in-vivo* concentration of (i) and the desired therapeutic efficacy endpoint are detected to determine whether to administer (i) multiple times.

In a fifth aspect, the present invention provides use of the pharmaceutical combination of the present invention in the treatment of a disease related to CLDN18.2 in a subject, comprising administering to the subject a therapeutically effective amount of the pharmaceutical combination as defined above in the fourth aspect, wherein the disease related to CLDN18.2 is, for example, a cancer expressing or overexpressing CLDN 18.2, such as a CLDN18.2-positive solid tumor.

In a sixth aspect, the present invention provides use of the pharmaceutical combination of the present invention in the preparation of a medicament for treating a disease related to CLDN18.2, wherein the disease related to CLDN18.2 is, for example, a cancer expressing or overexpressing CLDN 18.2, such as a CLDN18.2-positive solid tumor.

In a seventh aspect, the present invention provides a method for treating a disease related to CLDN18.2, comprising administering to a subject a therapeutically effective amount of the pharmaceutical combination of the present invention, wherein the disease related to CLDN18.2 is, for example, a cancer expressing or overexpressing CLDN 18.2, such as a CLDN18.2-positive solid tumor.

In an eighth aspect, the present invention provides a kit of parts, comprising the pharmaceutical combination as defined above in the fourth aspect, wherein preferably, the kit of parts is in the form of a pharmaceutical dose unit. In a ninth aspect, the present invention provides a pharmaceutical complex, formed by
(i) an immune effector cell (e.g., a T cell) expressing the molecular switch-regulated CAR polypeptide according to the first aspect of the present invention; and
(ii) an antibody or an antigen-binding fragment specifically binding to a CLDN18.2 molecule and comprising a P329G mutation (also referred to as a P329G mutated antibody), wherein, for example, the P329G mutated antibody comprises a mutated Fc domain, wherein the amino acid at position P329 according to the EU numbering is mutated to glycine (G), and the binding of the mutated Fc domain to an Fcy receptor is reduced compared to the binding of an unmutated parent antibody Fc domain to the Fcy receptor;

wherein the complex is formed by binding of the humanized anti-P329G mutation scFv sequence in the extracellular domain of the CAR polypeptide in the immune effector cell to the Fc domain of the P329G mutated antibody;
for example, the immune effector cell is a T cell expressing the molecular switch-regulated CAR polypeptide according to the first aspect of the present invention prepared from an autologous T cell or an allogeneic T cell, e.g., the immune effector cell is a T cell expressing the molecular switch-regulated CAR polypeptide according to the first aspect of the present invention prepared from a T cell isolated from a human PBMC;
for example, the P329G mutated antibody is an HB37A6 PG Ab, an Hz69H9 PG Ab, an Hz69H9-1.2 PG Ab, an Hz69H9-2.1 PG Ab, an Hz69H9-SA PG Ab, and/or an Hz3G3 PG Ab.

The present invention further provides use of the pharmaceutical complex in the treatment of a disease related to CLDN18.2 in a subject, wherein preferably, the disease related to CLDN18.2 is, for example, a cancer expressing or overexpressing CLDN 18.2, such as a CLDN18.2-positive solid tumor.

Thus, the present invention, for the first time, demonstrates that the pharmaceutical combination and the pharmaceutical complex of the present invention are applicable to primary CAR-T cells and are therefore capable of regulating the functions of CAR-T cells *in vitro* and *in vivo,* clarifies that the pharmaceutical combination and the pharmaceutical complex of the present invention are capable of endowing CAR-T cells with optimal *in-vitro* and *in-vivo* functions, demonstrates that the pharmaceutical combination and the pharmaceutical complex of the present invention are applicable to target therapeutic target CLDN18.2 to treat diseases, and obtains the impact of the characteristics (e.g., affinity) of the P329G mutated antibody molecule serving as the "molecular switch" on the functions of CAR-T cells.

### BRIEF DESCRIPTION OF THE DRAWINGS

The preferred embodiments of the present invention described in detail below will be better understood when read in conjunction with the following drawings. For the purpose of illustrating the present invention, currently, preferred embodiments are shown in the drawings. However, it should be understood that the present invention is not limited to the precise arrangements and means of the embodiments shown in the drawings.
FIG. 1 shows the structures of 4 CAR constructs. In the figure, "SP" represents a signal peptide; "TMD" represents a transmembrane domain; "ICD" represents an intracellular domain; "CSD" represents a co-stimulatory signaling domain; and "SSD" represents a stimulatory signaling domain. In each of the constructs, the extracellular domain comprises an antigen-binding portion capable of specifically binding to a mutated Fc domain containing a P329G mutation, and the antigen-binding portion comprises a heavy chain variable region (VH) and a light chain variable region (VH).
FIG. 2 shows a map of a pRK lentiviral expression vector.
FIG. 3 shows electrophoresis identification results of the lentiviral expression vector after insertion of each CAR construct and digestion with a restriction endonuclease.
FIG. 4A shows the expression of CAR in subpopulations of CD3⁺ cells, CD8⁻ (CD4⁺) T cells and CD8⁺ T cells as detected by flow cytometry after transduction of T cells with each of 7 types of CAR.
FIG. 4B shows the expression of CD45RA and CCR7 in CAR-T cells as detected by flow cytometry after transduction of T cells with each of 7 types of CAR.
FIG. 4C shows the expression of CD27 and CD28 in CAR-T cells as detected by flow cytometry after transduction of T cells with each of 7 types of CAR.
FIG. 5 shows the expression of CLDN18.2 on cells from gastric cancer cell lines AGS, SNU601, SNU620 and NUGC4 and on cells from a human pancreatic cancer cell line DAN-G18.2 stably transfected to express CLDN18.2, as detected by flow cytometry.
FIG. 6A shows the binding abilities of an A6 antibody (i.e., HB37A6 antibody), an H9 antibody (i.e., Hz69H9 antibody), and a Zmab antibody to CHO-GS cells stably expressing human, rhesus monkey, mouse and rat CLDN18.1 and CLDN18.2; and the binding abilities of WT and P329G mutated A6, H9 and Zmab antibodies at different concentrations to CLDN18.2 expression-positive tumor cells SUN601, NUGC4, and DAN-G18.2. In the figure, Con IgG represents a control IgG, which is an IgG that does not bind to CLDN18.2 or CLDN18.1 molecules.
FIG. 6B shows the binding of the WT A6 antibody (i.e., HB37A6 antibody) and the P329G mutated A6 antibody (i.e., HB37A6 PG antibody) to P329G CAR-T cells.
FIG. 6C shows whether the WT A6 antibody (i.e., HB37A6 antibody) and the P329G mutated A6 antibody (i.e., HB37A6 PG antibody) mediate PBMCs to exert a cell killing function through ADCC.
FIG. 6D shows whether the WT A6 antibody (i.e., HB37A6 antibody) and the P329G mutated A6 antibody (i.e., HB37A6 PG antibody) mediate a complement to exert a cell killing function through CDC.
FIG. 7A shows the experimental results of repeated killing of SNU601 cells by HuR968B, HuR9684M and Hu28HR968 CAR-T cells prepared from donor 3 (PCH20201100004:allcells, cat: PB004F-C, 39-year-old female) at an E:T ratio of 1:5.
FIG. 7B shows the experimental results of repeated killing of SNU601 cells by HuR968B, HuR9684M and Hu28HR968 CAR-T cells prepared from donor 3 (PCH20201100004:allcells, cat: PB004F-C, 39-year-old female) at an E:T ratio of 1:10.
FIG. 8A shows the killing effects of HuR968B and HuR9684M CAR-T cells prepared from donor 3 (PCH20201100004:allcells, cat: PB004F-C, 39-year-old female) on DAN-G18.2 tumor cells.
FIGs. 8B-8D show the results of the release of effector cytokines IFN-γ, IL-2 and TNF-α from HuR968B and HuR9684M CAR-T cells co-cultured with tumor cells from donor 3 (PCH20201100004:allcells, cat: PB004F-C, 39-year-old female), respectively.
FIG. 8E shows the killing effects of HuR968B CAR-T cells on DAN-G18.2 and SNU601 tumor cells at different concentrations of P329G A6 antibody.
FIG. 8F shows the proliferation of HuR9684M CAR-T cells after stimulation by a plate-bound WT or P329G A6 antibody.
FIG. 8G shows the proliferation of HuR968B CAR-T cells after stimulation by a plate-bound WT or P329G A6 antibody.
FIG. 9A shows sampling time points for an A6 antibody pharmacokinetic experiment in mice and a schematic diagram of an A6 antibody ELISA in mice.
FIG. 9B shows the results of an A6 antibody pharmacokinetic experiment in mice.
FIG. 10A shows the therapeutic effects of HuR968B and HuR9684M CAR-T cells in immunodeficient tumor-bearing mice inoculated with human DAN-G18.2 tumor cells.
FIG. 10B shows the changes in mouse body weight induced by HuR968B and HuR9684M CAR-T cells in immunodeficient tumor-bearing mice inoculated with human DAN-G18.2 tumor cells.
FIG. 10C shows the *in-vivo* expansion of HuR968B and HuR9684M CAR-T cells in immunodeficient tumor-bearing mice inoculated with human DAN-G18.2 tumor cells.
FIG. 11A shows the therapeutic effects of HuR968B CAR-T cells in combination with different doses of P329G A6 antibody in immunodeficient tumor-bearing mice inoculated with human DAN-G18.2 tumor cells.
FIG. 11B shows the changes in mouse body weight induced by HuR968B CAR-T cells in combination with different doses of P329G A6 antibody in immunodeficient tumor-bearing mice inoculated with human DAN-G18.2 tumor cells.
FIG. 11C shows the *in-vivo* expansion of HuR968B CAR-T cells induced by HuR968B CAR-T cells in combination with different doses of P329G A6 antibody in immunodeficient tumor-bearing mice inoculated with human DAN-G18.2 tumor cells.
FIG. 12A shows the therapeutic effects of HuR968B CAR-T cells in combination with multiple doses of P329G antibody in immunodeficient tumor-bearing mice inoculated with human DAN-G18.2 tumor cells.
FIG. 12B shows the changes in mouse body weight induced by HuR968B CAR-T cells in combination with multiple doses of P329G antibody in immunodeficient tumor-bearing mice inoculated with human DAN-G18.2 tumor cells.
FIGs. 13A-13B show the anti-tumor effects produced by different administration sequences of HuR968B CAR-T cells and a P329G A6 antibody in immunodeficient tumor-bearing mice inoculated with human DAN-G18.2 tumor cells.
FIG. 14A shows the binding abilities of 7 different anti-CLDN18.2 antibodies to CLDN18.2 based on cytological experiments.
FIG. 14B shows affinity profiles of 6 antibodies for a recombinant human CLDN18.2 protein as determined using surface plasmon resonance (SPR).
FIGs. 15A-15C show the results of 7 different antibodies inducing HuR968B CAR-T cells to release effector cytokines IL-2, IFN-γ and TNF-α in co-culture experiments with DAN-G18.2 or SNU601 tumor cells, respectively.
FIG. 15D shows the killing effects of HuR968B CAR-T cells on DAN-G18.2 tumor cells induced by 7 different antibodies.
FIG. 15E shows the results of dynamic killing experiments comparing the killing effects of HuR968B CAR-T cells on tumor cells with high CLDN18.2 expression induced by high- or low-affinity antibodies.
FIG. 15F shows the results of dynamic killing experiments comparing the killing effects of HuR968B CAR-T cells on tumor cells with low CLDN18.2 expression induced by high- or low-affinity antibodies.
FIG. 16A shows the therapeutic effects of HuR968B CAR-T cells in combination with different CLDN18.2 antibodies in immunodeficient tumor-bearing mice inoculated with human DAN-G18.2 tumor cells.
FIG. 16B shows the changes in mouse body weight induced by HuR968B CAR-T cells in combination with different CLDN18.2 antibodies in immunodeficient tumor-bearing mice inoculated with human DAN-G18.2 tumor cells.
FIG. 17A shows a flow chart of a membrane proteome array (MPA) study.
FIG. 17B shows a schematic diagram of the structure of a test PG-Fc fusion protein used for the MPA study.
FIG. 17C shows the results of the first round of high-throughput screening for the binding of the test PG-Fc fusion protein to membrane proteins.
FIG. 17D shows the results of the second round of experiment on the binding of the test PG-Fc fusion protein to GALNT1 and GPR149 proteins.
FIG. 17E shows the expression of GALNT1 and GPR149 proteins in CHO GS cells overexpressing off-target genes as detected by IHC, with GALNT1 primarily expressed in the cytoplasm and GPR149 primarily expressed on the cell membrane.
FIG. 17F shows the expression of GALNT1 and GPR149 proteins in 293T cells overexpressing off-target genes as detected by IHC, with GALNT1 primarily expressed in the cytoplasm and GPR149 primarily expressed on the cell membrane.
FIG. 17G shows the expression of off-target genes as detected by flow cytometry, with CH2-PG-expressing cells as a positive control.
FIG. 17H shows the activating effects of HuR968B CAR-T cells on different target cells.
FIG. 17I shows the results of the release of effector cytokines IL-2, TNF-α and IFN-y from P329G CAR-T cells co-cultured with different target cells.
FIG. 17J shows the results of sustained killing of different target cells by P329G CAR-T cells at E:T ratios of 6.7:1, 13:1 and 20:1, respectively.

### DETAILED DESCRIPTION

Unless otherwise defined, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### I. Definitions

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting. The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "Claudins" is a family of integrin membrane proteins that are present in epithelial and endothelial tight junctions and are an important component of tight junctions, discovered in 1998 by Shoichiro Tsukita et al. This family has 24 members. The human Claudin 18 gene has two alternative exon 1 options, thus giving rise to two protein isoforms Claudin 18.1 (also referred to herein as "CLDN18.1") and Claudin 18.2 (also referred to herein as "CLDN18.2") that differ by only 7 amino acid residues in the sequence of about 50 amino acids in the first extracellular domain.

There is a significant difference in expression of Claudin 18.2 between cancer tissues and normal tissues, which may result from the fact that the CREB binding site in the Claudin18.2 promoter region is highly methylated in CpG in normal tissues, while the level of CpG methylation is reduced during canceration of cells, thereby allowing CREB to participate in the activation of Claudin 18.2 transcription.

The terms "CLDN18.2 antibody", "antibody directed against CLDN18.2", "antibody specifically binding to CLDN18.2", "antibody specifically targeting CLDN18.2", and "antibody specifically recognizing CLDN18.2" as used herein are used interchangeably and mean an antibody capable of specifically binding to the Claudin protein CLDN18.2. In particular, in some specific embodiments, these terms mean an antibody specifically binding to human CLDN18.2, especially an antibody specifically binding to human CLDN18.2 but not to human CLDN18.1. The term "antibody" is used herein in the broadest sense, refers to a protein comprising an antigen-binding site, and encompasses natural and artificial antibodies with various structures, including, but not limited to, monoclonal antibodies, polyclonal antibodies, multispecific antibodies (e.g., bispecific antibodies), single-chain antibodies, intact antibodies, and antibody fragments. Preferably, the antibody of the present invention is a single-domain antibody or a heavy-chain antibody.

"Antibody fragment" and "antigen-binding fragment" are used interchangeably herein and refer to a molecule different from an intact antibody, which comprises a portion of the intact antibody and binds to an antigen to which the intact antibody binds. Examples of the antibody fragment include, but are not limited to, Fab', F(ab')₂, Fv, single-chain Fv, single-chain Fab, and diabodies.

The term "scFv" refers to a fusion protein comprising at least one antibody fragment containing a light chain variable region and at least one antibody fragment containing a heavy chain variable region, wherein the light chain variable region and the heavy chain variable region are continuously linked, optionally via a flexible short polypeptide linker, and are capable of being expressed as a single-chain polypeptide, with the scFv retaining the specificity of the intact antibody from which it is derived. Unless otherwise indicated, the scFv, as used herein, may have VL and VH variable regions in any order (e.g., relative to the N-terminus and C-terminus of the polypeptide), and may comprise VL-linker-VH or VH-linker-VL.

"Complementarity determining region" or "CDR" or "highly variable region" is a region in an antibody variable domain that is highly variable in sequence and forms a structurally defined loop ("hypervariable loop") and/or comprises antigen-contacting residues ("antigen-contacting sites"). CDRs are primarily responsible for binding to antigen epitopes. CDRs of heavy and light chains are generally referred to as CDR1, CDR2, and CDR3, and are numbered sequentially from the N-terminus. CDRs located in a heavy chain variable domain of an antibody are referred to as CDR H1, CDR H2, and CDR H3, whereas CDRs located in a light chain variable domain of the antibody are referred to as CDR L1, CDR L2, and CDR L3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

Unless otherwise stated, the term "CDR" or "CDR sequence" used herein encompasses CDR sequences determined by any one of the schemes described above.

CDRs can also be determined based on having the same Kabat numbering positions as a reference CDR sequence (e.g., any of the exemplary CDRs of the present invention). In the present invention, the numbering positions of antibody variable regions and specific CDR sequences (including heavy chain variable region residues) are based on the Kabat numbering system.

Although CDRs vary from antibody to antibody, only a limited number of amino acid positions within the CDRs are directly involved in antigen binding. The smallest overlapping region can be determined using at least two of the Kabat, Chothia, AbM, and Contact schemes, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the minimal binding unit may remain unchanged, while the remaining CDR residues defined according to Kabat or Chothia or AbM may be substituted with conservative amino acid residues. The term "chimeric antibody" is an antibody molecule in which: (a) a constant region or a portion thereof is modified, substituted, or exchanged such that antigen-binding sites are linked to constant regions of different or modified classes and/or species, or disparate molecules imparting new properties (e.g., enzymes, toxins, hormones, growth factors, and drugs) to chimeric antibodies, etc.; or (b) a variable region or a portion thereof is modified, replaced, or exchanged by variable regions with different or modified antigen-binding specificities. For example, a murine antibody may be modified by substituting its constant region with a constant region from a human immunoglobulin. Due to the substitution with a human constant region, the chimeric antibody may retain its specificity for recognizing antigens, while having reduced antigenicity in humans as compared to the original murine antibody.

"Humanized" antibody refers to a chimeric antibody comprising amino acid residues from non-human CDRs and amino acid residues from human FRs. In some embodiments, in the humanized antibody, all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized.

"Human antibody" refers to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

The term "Fc region" is used herein to define a C-terminus region of an immunoglobulin heavy chain, which comprises at least a portion of a constant region. The term includes Fc regions of native sequences and variant Fc regions. In certain embodiments, a human IgG heavy chain Fc region extends from Cys226 or Pro230 to the carbonyl terminus of the heavy chain. However, the C-terminal lysine (Lys447) of the Fc region may or may not be present. Unless otherwise stated, the numbering of amino acid residues in the Fc region or constant region is based on the EU numbering scheme, which is also referred to as EU index, as described in Kabat et al., Sequences of Proteins of Immunological Interest, 5th Ed., Public Health Service, National Institutes of Health, Bethesda, MD, 1991.

The term "effector function" refers to biological activities attributed to an immunoglobulin Fc region that vary with immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (such as B-cell receptors), and B-cell activation.

The term "antibody-dependent cell-mediated cytotoxicity (ADCC)" is one of the major mechanisms by which certain cytotoxic effector cells (e.g., natural killer (NK) cells) mediate the killing of target cells and foreign host cells. In some embodiments, the ADCC effect is exerted by the activation of NK cells following the binding of the antibody Fc region to the Fc receptor FcyRIIIA (i.e., CD16a) expressed on, for example, NK cells. CD16a is a transmembrane receptor member of immunoglobulin superfamily, and according to the allelic polymorphism difference at position 158 of its N-terminus, CD16a exhibits differential expression of valine or phenylalanine at position 158, so that the isoforms of CD16a-158V/V (about 15%), CD16a-158V/F (about 25%), and CD16a-158F/F (about 60%) exist in the population. In some other embodiments, the chimeric antigen receptor of the present invention provides antibody-dependent cytotoxicity of T lymphocytes and enhances antibody-dependent cytotoxicity of NK cells. The chimeric antigen receptor of the present invention, by binding to an antibody (or other anti-tumor molecule comprising an Fc portion) that binds to tumor cells, induces the activation, sustained proliferation, and exertion of specific cytotoxicity against target cancer cells mediated by the antibody (or other anti-tumor molecule comprising the Fc portion) in T cells expressing the chimeric antigen receptor.

The term "complement-dependent cytotoxicity (CDC)" refers to the lysis of target cells in the presence of complement. Activation of the classical complement pathway is initiated by the binding of the first component of the complement system (C1q) to an antibody (of an appropriate subclass) that binds to its homologous antigen. To assess complement activation, a CDC assay can be performed, for example, by the method described in Gazzano-Santoro et al., J. Immunol. Methods 202: 163 (1996).

The term "variable region" or "variable domain" refers to a domain of a heavy chain or light chain of an antibody involved in the binding of the antibody to an antigen. Variable domains of heavy chains and light chains of natural antibodies typically have similar structures, wherein each domain comprises four conserved framework regions (FRs) and three complementarity determining regions (CDRs). (See, e.g., Kindt et al., Kuby Immunology, 6th ed., W.H. Freeman and Co., p. 91 (2007)). A single VH or VL domain may be sufficient to provide antigen-binding specificity.

As used herein, the term "binding" or "specific binding" means that the binding effect is selective for antigens and may be distinguished from unwanted or non-specific interactions. The ability of an antibody to bind to a particular antigen can be determined by an enzyme-linked immunosorbent assay (ELISA), SPR or bio-layer interferometry or a conventional binding assay known in the art.

The term "stimulation" refers to a primary response induced by the binding of a stimulatory molecule (e.g., a TCR/CD3 complex) to its corresponding ligand, which then mediates signal transduction events, such as, but not limited to, signal transduction by means of the TCR/CD3 complex. Stimulation may mediate altered expression of certain molecules, such as the down-regulation of TGF-β and/or the reorganization of the cytoskeletal structure, etc.

The term "stimulatory molecule" refers to a molecule expressed by a T cell that provides a primary cytoplasmic signaling sequence that regulates primary activation of the TCR complex in a stimulatory manner in at least some aspect of the T cell signaling pathway. In one embodiment, the primary signal is initiated, for example, by binding of the TCR/CD3 complex to peptide-loaded MHC molecules and results in mediation of T cell responses including, but not limited to, proliferation, activation, differentiation, etc. In the specific CARs of the present invention, the intracellular signaling domain in any one or more of the CARs of the present invention comprises an intracellular signaling sequence, e.g., a primary signaling sequence of CD3ζ.

The term "CD3ζ" is defined as the protein provided under GenBank accession No. BAG36664.1 or an equivalent thereof, and "CD3ζ stimulatory signaling domain" is defined as amino acid residues from a cytoplasmic domain of the CD3ζ chain, the amino acid residues being sufficient to functionally propagate an initial signal necessary for T cell activation. In one embodiment, the cytoplasmic domain of CD3ζ comprises residues 52 to 164 of GenBank accession No. BAG36664.1 or equivalent residues from a non-human species (e.g., mouse, rodent, monkey, ape, etc.) that serve as functional orthologs thereof. In one embodiment, the "CD3ζ stimulatory signaling domain" is the sequence provided in SEQ ID NO: 13 or a variant thereof.

The term "co-stimulatory molecule" refers to a corresponding binding partner on a cell that specifically binds to a co-stimulatory ligand, thereby mediating a co-stimulatory response (such as, but not limited to, proliferation) of the cell. Co-stimulatory molecules are cell surface molecules other than antigen receptors or ligands thereof that contribute to effective immune responses. Co-stimulatory molecules include, but are not limited to, MHC class I molecules, TNF receptor proteins, immunoglobulin-like proteins, cytokine receptors, integrins, signaling lymphocyte activation molecules (SLAM proteins), activating NK cell receptors, OX40, CD40, GITR, 4-1BB (i.e., CD137), CD27, and CD28. In some embodiments, the "co-stimulatory molecule" is CD28 or 4-1BB (i.e., CD137). The co-stimulatory signaling domain refers to the intracellular portion of the co-stimulatory molecule. The term "4-1BB" refers to a member of the TNFR superfamily, which has an amino acid sequence provided under GenBank accession No. AAA62478.2 or equivalent residues from a non-human species (e.g., mouse, rodent, monkey, ape, etc.); and "4-1BB co-stimulatory signaling domain" is defined as amino acid residues 214-255 under GenBank accession No. AAA62478.2 or equivalent residues from a non-human species (e.g., mouse, rodent, monkey, ape, etc.). In one embodiment, the "4-1BB co-stimulatory domain" is the sequence provided as SEQ ID NO: 11 or equivalent residues from a non-human species (e.g., mouse, rodent, monkey, ape, etc.).

The term "signaling pathway" refers to the biochemical relationships among a variety of signaling molecules that play a role in propagating signals from one part of a cell to another part of the cell.

The term "cytokine" is a generic term for proteins that are released by a cell population and act as intercellular mediators on another cell. Examples of such cytokines are lymphokines; monokines; interleukin (ILs), such as IL-1, IL-1α, IL-2, IL-3, IL-4, IL-5, IL-6, IL-7, IL-8, IL-9, IL-11, IL-12, and IL-15; tumor necrosis factors such as TNF-α or TNF-β; and other polypeptide factors, including γ-interferon.

"Isolated" antibody is an antibody that has been separated from components of its natural environment. In some embodiments, the antibody of the present invention is purified to a purity greater than 95% or 99% as determined by, for example, electrophoresis (e.g., SDS-PAGE, isoelectric focusing (IEF), and capillary electrophoresis) or chromatography (e.g., ion exchange or reverse-phase HPLC). For a review of methods for assessing antibody purity, see, e.g., Flatman et al., J. Chromatogr., B848: 79-87 (2007).

"Isolated" nucleic acid refers to a nucleic acid molecule that has been separated from components of its natural environment. The isolated nucleic acid includes a nucleic acid molecule contained in a cell that typically comprises the nucleic acid molecule, but the nucleic acid molecule exists extrachromosomally or at a chromosomal location that is different from its natural chromosomal location. "Isolated nucleic acid encoding the antibody of the present invention" refers to one or more nucleic acid molecules encoding chains of the antibody of the present invention or fragments thereof, including such nucleic acid molecules in a single vector or separate vectors, and such nucleic acid molecules present at one or more positions in a host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needleman and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

The terms "amino acid change" and "amino acid modification" are used interchangeably and refer to addition, deletion, substitution, and other modifications of amino acids. Any combination of additions, deletions, substitutions and other modifications of amino acids may be made, provided that the final polypeptide sequence possesses the desired properties. In some embodiments, the amino acid substitution in the antibody results in reduced binding of the antibody to the Fc receptor. For the purpose of changing, for example, the binding characteristics of the Fc region, non-conservative amino acid substitutions are particularly preferred, where one amino acid is substituted with another amino acid having different structural and/or chemical properties. Amino acid substitutions include substitutions with non-naturally occurring amino acids or naturally occurring amino acid derivatives of the twenty standard amino acids (e.g., 4-hydroxyproline, 3-methylhistidine, ornithine, homoserine, and 5-hydroxylysine). Amino acid changes can be made using genetic or chemical methods well known in the art. Genetic methods may include site-directed mutagenesis, PCR, gene synthesis, etc. Methods for altering side chain groups of amino acids by means other than genetic engineering, such as chemical modification, may be useful. Multiple names may be used herein to denote the same amino acid change. For example, a substitution from proline to glycine at position 329 of the Fc domain may be denoted as 329G, G329, G₃₂₉, P329G Pro329Gly, or simply "PG".

The terms "conservative sequence modification" and "conservative sequence change" refer to amino acid modifications or changes that do not significantly affect or change the binding characteristics of an antibody or an antibody fragment comprising the amino acid sequence. Such conservative modifications include amino acid substitutions, additions, and deletions. Modifications can be introduced into the antibody or the antibody fragment of the present invention by standard techniques known in the art, such as site-directed mutagenesis and PCR-mediated mutagenesis. Conservative substitutions are amino acid substitutions in which an amino acid residue is replaced with an amino acid residue having a similar side chain. Families of the amino acid residues having similar side chains have been defined in the art. These families include amino acids having basic side chains (e.g., lysine, arginine, and histidine), amino acids having acidic side chains (e.g., aspartic acid and glutamic acid), amino acids having uncharged polar side chains (e.g., glycine, asparagine, glutamine, serine, threonine, tyrosine, cysteine, and tryptophan), amino acids having non-polar side chains (e.g., alanine, valine, leucine, isoleucine, proline, phenylalanine, and methionine), amino acids having β-side chains (e.g., threonine, valine, and isoleucine), and amino acids having aromatic side chains (e.g., tyrosine, phenylalanine, tryptophan, and histidine). Thus, one or more amino acid residues within the CARs of the present invention may be replaced with other amino acid residues from the same side chain family, and the altered CARs may be tested using the functional assays described herein.

The term "autologous" refers to any substance that is derived from the same individual into whom the substance is later to be re-introduced.

The term "allogeneic" refers to any substance that is derived from a different animal of the same species as the individual into whom the substance is to be introduced. Two or more individuals are said to be allogeneic to one another when the genes at one or more loci are not identical. In some aspects, allogeneic substances from individuals of the same species may be genetically dissimilar enough to undergo antigenic interactions.

The term "xenogeneic" refers to a graft derived from an animal of a different species.

The term "apheresis" as used herein refers to the art-recognized extracorporeal method by which the blood of a donor or patient is removed from the donor or patient and passed through an apparatus that separates out selected particular components and returns the remainder to the circulation of the donor or patient, e.g., by retransfusion. Therefore, in the context of "an apheresis sample", it refers to a sample obtained using apheresis.

The term "immune effector cell" refers to a cell that is involved in an immune response, e.g., in the promotion of an immune effector response. Examples of immune effector cells include T cells, e.g., α/β T cells and γ/δ T cells, B cells, natural killer (NK) cells, natural killer T (NKT) cells, mast cells, and myeloid cell-derived phagocytes.

"Immune effector function" or "immune effector response" refers to, for example, a function or response of an immune effector cell that enhances or promotes an immune attack on a target cell. For example, the immune effector function or response refers to a property of a T cell or NK cell that promotes the killing of a target cell or inhibits the growth or proliferation of a target cell. In the case of T cells, primary stimulation and co-stimulation are examples of the immune effector function or response.

The term "effector function" refers to a specialized function of a cell. The effector function of a T cell may be, for example, cytolytic activity or helper activity, including secretion of cytokines.

The term "T cell activation" refers to one or more cellular responses of a T lymphocyte, in particular a cytotoxic T lymphocyte, selected from: proliferation, differentiation, cytokine secretion, release of cytotoxic effector molecules, cytotoxic activity, and expression of activation markers. The chimeric antigen receptor of the present invention is capable of inducing T cell activation. Suitable assays for measuring T cell activation are described in the examples and are known in the art.

The term "lentivirus" refers to a genus of the Retroviridae family. Lentiviruses are unique among retroviruses in their ability to infect non-dividing cells; they can deliver a significant amount of genetic information to a host cell, making them one of the most efficient methods of gene delivery vectors. HIV, SIV, and FIV are all examples of lentiviruses.

The term "lentiviral vector" refers to a vector derived from at least a portion of the lentiviral genome, in particular including a self-inactivating lentiviral vector as provided in Milone et al., Mol. Ther. 17(8): 1453-1464 (2009). Other examples of lentiviral vectors that can be used clinically include, for example, but are not limited to, the LENTIVECTOR^{®} gene delivery technology from Oxford BioMedica, the LENTIMAX^{™} vector system from Lentigen, etc. Non-clinical types of lentiviral vectors are also available and known to those skilled in the art.

The term "disease related to CLDN18.2" refers to any condition caused or exacerbated by or otherwise related to increased expression or activity of CLDN18.2 (e.g., human CLDN18.2).

The terms "individual" and "subject" are used interchangeably and include mammals. The mammals include, but are not limited to, domesticated animals (e.g., cows, sheep, cats, dogs, and horses), primates (e.g., humans and non-human primates such as monkeys), rabbits and rodents (e.g., mice and rats). In particular, the individual or subject is a human.

The terms "tumor" and "cancer" are used interchangeably herein and encompass solid and liquid tumors.

The terms "cancer" and "carcinoma" refer to a physiological disease in mammals in which cell growth is unregulated.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein.

"Tumor immune escape" refers to a process by which tumors evade immune recognition and clearance. As such, as a therapeutic concept, tumor immunity is "treated" when such evasion diminishes, and the tumor is recognized and attacked by the immune system. Examples of tumor recognition include tumor binding, tumor shrinkage, and tumor clearance.

The term "half maximal effective concentration (EC₅₀)" refers to the concentration of a drug, antibody or toxin that induces a response of 50% between the baseline and the maximum after a particular exposure time.

The term "fluorescence activated cell sorting" or "FACS" refers to a specialized type of flow cytometry. It provides a method for sorting a heterogeneous mixture of biological cells, one cell at a time, into two or more containers based on the specific light scattering and fluorescence characteristics of each cell (FlowMetric. "Sorting Out Fluorescence Activated Cell Sorting". 2017-11-09). Instruments for performing FACS are known to those skilled in the art and are commercially available to the public. Examples of such instruments include FACS Star Plus, FACScan and FACSort instruments from Becton Dickinson (Foster City, CA), Epics C from Coulter Epics Division (Hialeah, FL), and MoFlo from Cytomation (Colorado Springs, Colorado).

The term "pharmaceutically acceptable supplementary material" refers to diluents, adjuvants (e.g., Freund's adjuvants (complete and incomplete)), excipients, buffers, stabilizers, or the like, which are administered with the active substance.

As used herein, "treatment", "treat" or "treating" refers to slowing, interrupting, arresting, alleviating, stopping, lowering, or reversing the progression or severity of an existing symptom, condition, disorder, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

The term "effective amount" refers to an amount or dosage of the antibody or composition of the present invention which generates expected effects in a patient in need of treatment or prevention after administration to the patient in a single dose or multiple doses. The effective amount can be easily determined by an attending physician as a person skilled in the art by considering a variety of factors as follows: species such as mammals; weight, age, and general health condition; the specific disease involved; the extent or severity of the disease; response in an individual patient; specific antibody administered; mode of administration; bioavailability characteristics of the administered formulation; selected administration regimen; and use of any concomitant therapy.

"Therapeutically effective amount" refers to an amount effective to achieve a desired therapeutic result at a necessary dose for a necessary period of time. The therapeutically effective amount of an antibody or antibody fragment or a composition thereof may vary depending on a variety of factors such as disease state, age, sex, and weight of an individual, and the ability of the antibody or antibody portion to elicit a desired response in the individual. The therapeutically effective amount is also such an amount that any toxic or undesired effect of the antibody or antibody fragment or the composition thereof is inferior to the therapeutically beneficial effect. "Therapeutically effective amount" preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, relative to an untreated subject. The ability of a compound to inhibit a measurable parameter (e.g., cancer) can be evaluated in an animal model system that predicts efficacy in human tumors.

The term "pharmaceutical combination" refers to a non-fixed combination product or a fixed combination product, including but not limited to a kit and a pharmaceutical composition. The term "non-fixed combination" means that the active ingredients (e.g., (i) P329G CAR-T cells, and (ii) a P329G mutated antibody directed against CLDN18.2) are administered to a subject either simultaneously or sequentially (without particular time limitation, or at identical or different time intervals) as separate entities, wherein such administration provides effective treatment in the subject. The term "fixed combination" refers to a combination in which the P329G mutated antibody directed against CLDN18.2 and the P329G CAR-T cells of the present invention are administered to a patient simultaneously in the form of a specific single dose. The term "non-fixed combination" refers to a combination in which the P329G mutated antibody directed against CLDN18.2 and the P329G CAR-T cells of the present invention are simultaneously, concurrently, or sequentially administered to a patient as separate entities, without specific dosage and time limitation, wherein such administration provides a therapeutically effective level of the pharmaceutical combination of the present invention in the patient. In one preferred embodiment, the pharmaceutical combination is a non-fixed combination.

The term "combination therapy" or "combined therapy" means that two or more components are administered to treat a cancer as described herein. Such administration includes co-administration of these components in a substantially simultaneous manner. Alternatively, such administration includes co-administration or separate administration or sequential administration of the active ingredients in a variety of or separate containers (e.g., capsules, powder, and liquid). The powder and/or liquid can be reconstituted or diluted to a desired dose before administration. In some embodiments, the administration further includes using the P329G mutated antibody directed against CLDN18.2 and the P329G CAR-T cells of the present invention at approximately the same time or in a sequential manner at different times. In any case, the therapeutic regimen will provide the beneficial effect of the pharmaceutical combination in the treatment of conditions or symptoms described herein.

The term "vector" as used herein when referring to a nucleic acid refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of nucleic acids to which they are operatively linked. Such vectors are referred to as "expression vectors" herein.

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. Host cells are any type of cell system that can be used to produce the antibody molecule of the present invention, including eukaryotic cells, e.g., mammalian cells, insect cells, and yeast cells; and prokaryotic cells, e.g., *E*. *coli* cells. Host cells include cultured cells, as well as cells within a transgenic animal, a transgenic plant, or cultured plant tissue or animal tissue.

"Subject/patient sample" refers to a collection of cells, tissues, or body fluids obtained from a patient or a subject.

The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds that are naturally not mixed with tissues, such as preservatives, anticoagulants, buffers, fixatives, nutrients, and antibiotics. Examples of tumor samples include but are not limited to tumor biopsies, fine needle aspirates, bronchial lavage fluids, pleural fluids, sputa, urine, surgical specimens, circulating tumor cells, serum, plasma, circulating plasma proteins, ascites, primary cell cultures or cell lines derived from tumors or exhibiting tumor-like properties, and preserved tumor samples such as formalin-fixed, paraffin-embedded tumor samples or frozen tumors samples.

When referring to a disease, the term "treatment", "treat" or "treating" refers to alleviating the disease (i.e., slowing or arresting or reducing the development of the disease or at least one clinical symptom thereof), preventing or delaying the onset or development or progression of the disease.

### II. Molecular switch-regulated chimeric antigen receptor (CAR) of the present invention

The present invention relates to a chimeric antigen receptor polypeptide capable of specifically binding to a mutated Fc domain of an antibody directed against a CLDN18.2 molecule. Specifically, the chimeric antigen receptor of the present invention comprises a humanized anti-P329G mutation scFv sequence, and the scFv sequence is capable of specifically binding to an antibody Fc domain comprising a P329G mutation, but not capable of specifically binding to an unmutated parent antibody Fc domain. The binding of the antibody Fc domain comprising the P329G mutation to an Fc receptor (e.g., an Fcy receptor) is reduced compared to the binding of the unmutated parent antibody Fc domain to the Fc receptor.

The recombinant CAR construct of the present invention comprises a sequence encoding a CAR, wherein the CAR comprises a humanized anti-P329G mutation scFv sequence that specifically binds to a P329G mutated antibody Fc domain.

In one embodiment, the scFv sequence in the CAR construct of the present invention comprises the following sequences:
(i) a heavy chain variable region, which comprises, according to the Kabat numbering,
   (a) a heavy chain complementarity determining region (CDR H) 1 shown in the amino acid sequence RYWMN (SEQ ID NO: 54), or a variant of the CDR H1 with no more than 2 amino acid changes or no more than 1 amino acid change;
   (b) a CDR H2 shown in the amino acid sequence EITPDSSTINYAPSLKG (SEQ ID NO: 55), or a variant of the CDR H2 with no more than 2 amino acid changes or no more than 1 amino acid change; and
   (c) a CDR H3 shown in the amino acid sequence PYDYGAWFAS (SEQ ID NO: 56), or a variant of the CDR H3 with no more than 2 amino acid changes or no more than 1 amino acid change; and
(ii) a light chain variable region, which comprises, according to the Kabat numbering,
   (d) a light chain complementarity determining region (CDR L) 1 shown in the amino acid sequence RSSTGAVTTSNYAN (SEQ ID NO: 57), or a variant of the CDR L1 with no more than 2 amino acid changes or no more than 1 amino acid change;
   (e) a CDR L2 shown in the amino acid sequence GTNKRAP (SEQ ID NO: 58), or a variant of the CDR L2 with no more than 2 amino acid changes or no more than 1 amino acid change; and
   (f) a CDR L3 shown in the amino acid sequence ALWYSNHWV (SEQ ID NO: 59), or a variant of the CDR L3 with no more than 2 amino acid changes or no more than 1 amino acid change;
wherein the scFv may be linked at the N-terminus with a signal peptide sequence, such as the signal peptide sequence set forth in SEQ ID NO: 1, and the scFv may be linked at the C-terminus with an optional hinge region/spacer region sequence as provided in SEQ ID NO: 5 or SEQ ID NO: 6 or SEQ ID NO: 7, a transmembrane region as provided in SEQ ID NO: 8 or SEQ ID NO: 9, an optional membrane-proximal intracellular signaling domain as provided in SEQ ID NO: 14, a co-stimulatory signaling domain as provided in SEQ ID NO: 10 or SEQ ID NO: 11 or SEQ ID NO: 12, and an intracellular stimulatory signaling domain comprising SEQ ID NO: 13 or a variant thereof, wherein, for example, the individual domains are adjacent to each other and in the same reading frame to form a single fusion protein.

In some embodiments, the scFv domain comprises (i) a heavy chain variable region comprising the sequence of SEQ ID NO: 2 or a sequence having at least 90%, 91%, 92%, 93%, 94%, 95%, 96%, 97%, 98% or 99% identity thereto, and (ii) a light chain variable region comprising the sequence of SEQ ID NO: 3 or a sequence having at least 90%, 91%, 92%, 93%, 94%, 95%, 96%, 97%, 98% or 99% identity thereto;
in some embodiments, the scFv domain comprises (i) a heavy chain variable region set forth in SEQ ID NO: 2 and (ii) a light chain variable region set forth in SEQ ID NO: 3. In one embodiment, the scFv domain further comprises a (Gly4-Ser)n linker, wherein n is 1, 2, 3, 4, 5, or 6, preferably 3 or 4. The light chain variable region and the heavy chain variable region of the scFv may be, for example, in any of the following orientations: light chain variable region-linker-heavy chain variable region, and heavy chain variable region-linker-light chain variable region.

In some embodiments, the exemplary CAR construct of the present invention comprises a signal peptide sequence, a humanized anti-P329G mutation scFv sequence, a hinge region/spacer region, a transmembrane domain, an intracellular co-stimulatory signaling domain, and an intracellular stimulatory signaling domain.

In some embodiments, the exemplary CAR construct of the present invention comprises a signal peptide sequence, a humanized anti-P329G mutation scFv sequence, a hinge region/spacer region, a transmembrane domain, a membrane-proximal intracellular domain, an intracellular co-stimulatory signaling domain, and an intracellular stimulatory signaling domain.

In some embodiments, in the present invention, the amino acid sequence of the full-length CAR polypeptide is provided as SEQ ID NO: 21, 23 or 25, as shown in the sequence listing.

In some embodiments, in the present invention, the exemplary signal peptide sequence is provided as SEQ ID NO: 1; the exemplary hinge region/spacer region sequence is provided as SEQ ID NO: 5 or SEQ ID NO: 6 or SEQ ID NO: 7; the exemplary transmembrane domain sequence is provided as SEQ ID NO: 8 or SEQ ID NO: 9; the exemplary membrane-proximal intracellular signaling domain is provided as SEQ ID NO: 14; the exemplary intracellular co-stimulatory signaling domain is provided as SEQ ID NO: 10 or SEQ ID NO: 11 or SEQ ID NO: 12; and the exemplary intracellular stimulatory signaling domain is provided as SEQ ID NO: 13 or a variant thereof.

In some embodiments, the present invention provides a recombinant nucleic acid construct comprising a nucleic acid molecule encoding the CAR of the present invention. For example, the nucleic acid sequence of the CAR construct of the present invention is selected from SEQ ID NO: 22, 24 or 26. Recombination methods well known in the art may be used to obtain the nucleic acid molecule encoding the CAR construct of the present invention. Alternatively, the nucleic acid of interest may be produced synthetically, rather than by genetic recombination methods.

The present invention includes a retroviral vector construct and a lentiviral vector construct that express CARs that can be directly transduced into cells.

In some embodiments, the nucleic acid sequence of the CAR construct of the present invention is cloned into a lentiviral vector to produce a full-length CAR construct in a single coding frame and used for expression with an EF1α promoter.

One of ordinary skill in the art will appreciate that the CAR polypeptide of the present invention may also be modified so as to vary in the amino acid sequence, but not in the desired activity. For example, the CAR polypeptide may be subjected to an additional nucleotide replacement that results in an amino acid replacement at a "non-essential" amino acid residue. For example, a non-essential amino acid residue in the molecule may be replaced with another amino acid residue from the same side chain family. In another embodiment, an amino acid fragment may be replaced with a structurally similar fragment that differs in the order and composition of the side chain family members; for example, a conservative replacement may be made in which an amino acid residue is replaced with an amino acid residue having a similar side chain.

Families of amino acid residues having similar side chains have been defined in the art, including basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), non-polar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine tryptophan, and histidine).

In some embodiments, the present invention contemplates the creation of a functionally equivalent CAR polypeptide molecule; for example, the VH or VL of the humanized anti-P329G mutation scFv sequence contained in the CAR may be modified to obtain a VH having at least 90%, 91%, 92%, 93%, 94%, 95%, 96%, 97%, 98% or 99% identity to SEQ ID NO: 2 and a VL having at least 90%, 91%, 92%, 93%, 94%, 95%, 96%, 97%, 98% or 99% identity to SEQ ID NO: 3.

The transmembrane domain contained in the CAR of the present invention is an anchored transmembrane domain, which is a component of a polypeptide chain that can be integrated in the cell membrane. The transmembrane domain may be fused to other extracellular and/or intracellular polypeptide domains, wherein the extracellular and/or intracellular polypeptide domains will also be restricted to the cell membrane. In the chimeric antigen receptor (CAR) polypeptide of the present invention, the transmembrane domain confers membrane attachment to the CAR polypeptide of the present invention. The CAR polypeptide of the present invention comprises at least one transmembrane domain, which may be derived from a natural or recombinant source and comprises predominantly hydrophobic residues such as leucine and valine. Where the source is natural, the domain may be derived from a transmembrane domain of a membrane-bound protein or transmembrane protein such as CD28 and CD8 (e.g., CD8α and CD8β). In one embodiment, the transmembrane domain comprises the amino acid sequence of SEQ ID NO: 9. In one embodiment, the transmembrane domain comprises the amino acid sequence of SEQ ID NO: 8, wherein a triplet of phenylalanine, tryptophan and valine is present at both termini of the transmembrane domain.

In some embodiments, the transmembrane domain in the CAR of the present invention is linked to the extracellular region of the CAR (i.e., the humanized anti-P329G mutation scFv sequence) by means of the hinge region/spacer region. For example, in one embodiment, the hinge may be a human Ig (immunoglobulin) hinge region, such as an IgG4 hinge region, and may also be a CD8a hinge region or a CD28 hinge region. In some embodiments, the hinge region or spacer region sequence comprises the amino acid sequence of SEQ ID NO: 6 or 7.

Additionally, a glycine-serine doublet also provides a particularly suitable linker as a hinge region/spacer region. For example, in one embodiment, the linker comprises the amino acid sequence of GGGGS (SEQ ID NO: 5). The cytoplasmic domain contained in the CAR of the present invention comprises an intracellular signaling domain. The intracellular signaling domain is capable of activating at least one effector function of an immune cell into which the CAR of the present invention is introduced.

Examples of the intracellular signaling domain for use in the CAR of the present invention include cytoplasmic sequences of T cell receptors (TCRs) and co-receptors that act synergistically to initiate signal transduction following the binding of the extracellular domain to a P329G mutated antibody Fc domain, as well as any derivatives or variants of these sequences and any recombinant sequences with the same functional capabilities. Given that the signals generated by the TCR alone are not sufficient to fully activate T cells, the CAR of the present invention is also designed with a co-stimulatory signaling domain (CSD) capable of producing co-stimulatory signals. T cell activation is mediated by two distinct classes of cytoplasmic signaling sequences: those that initiate antigen-dependent primary activation through the TCR (primary intracellular signaling domains) and those that function in an antigen-independent manner to provide co-stimulatory signals (secondary cytoplasmic domains, e.g., co-stimulatory domains).

In one embodiment, the CAR of the present invention comprises a primary intracellular signaling domain, for example, a CD3ε chain membrane-proximal intracellular signaling domain, such as a CD3ε chain membrane-proximal intracellular signaling domain as set forth in SEQ ID NO: 14; and a CD3ζ primary signaling domain, such as a CD3ζ signaling domain as set forth in SEQ ID NO: 13.

The intracellular signaling domain in the CAR of the present invention further comprises a secondary signaling domain (i.e., a co-stimulatory signaling domain). The co-stimulatory signaling domain refers to a portion of the CAR that comprises the intracellular domain of the co-stimulatory molecule. Co-stimulatory molecules are cell surface molecules required by lymphocytes, in addition to antigen receptors or ligands thereof, for effective responses to antigens. In some embodiments, co-stimulatory molecules include, but are not limited to, CD28 and 4-1BB (CD137), which cause co-stimulatory effects that enhance the proliferation, effector function, and survival of human CART cells *in vitro* and improve the anti-tumor activity of human T cells *in vivo.*

The intracellular signaling sequences of the CAR in the present invention may be linked to each other in a random order or in a specified order. Optionally, short oligopeptide linkers or polypeptide linkers may form linkages between intracellular signaling sequences. In one embodiment, the glycine-serine doublet may be used as a suitable linker. In one embodiment, a single amino acid such as alanine or glycine may be used as a suitable linker.

In one embodiment, the intracellular signaling domain of the CAR of the present invention is designed to comprise a co-stimulatory signaling domain of CD28 and a stimulatory signaling domain of CD3ζ. In another embodiment, the intracellular signaling domain is designed to comprise a co-stimulatory signaling domain of 4-1BB and a stimulatory signaling domain of CD3ζ. In yet another embodiment, the intracellular signaling domain is designed to further comprise a CD3ε chain membrane-proximal intracellular signaling domain.

### III. Nucleic acid molecule encoding the CAR of the present invention, vector, and cell expressing the CAR of the present invention

The present invention provides a nucleic acid molecule encoding the CAR construct described herein. In one embodiment, the nucleic acid molecule is provided as a DNA construct.

In some embodiments, the CAR construct comprises the nucleotide sequence set forth in SEQ ID NO: 22, 24 or 26.

The present invention further provides a vector into which the CAR construct of the present invention is inserted. The expression of a natural or synthetic nucleic acid encoding a CAR is achieved by effectively linking the nucleic acid encoding the CAR polypeptide to a promoter and incorporating the construct into an expression vector. The vector may be suitable for replication and integration in a eukaryotic organism. Common cloning vectors contain transcriptional and translational terminators, initiation sequences, and promoters used for regulating the expression of the desired nucleic acid sequences.

Numerous virus-based systems have been developed for transferring genes into mammalian cells. For example, retroviruses provide a convenient platform for use in gene delivery systems. The selected gene can be inserted into a vector and packaged into a retroviral particle using techniques known in the art. Subsequently, the recombinant virus can be isolated and delivered to cells of a subject *in vivo* or *ex vivo.* Numerous retroviral systems are known in the art. In some embodiments, a lentiviral vector is used.

Vectors derived from retroviruses (e.g., lentiviruses) are suitable tools for achieving long-term gene transfer because they allow for long-term, stable integration of a transgene and its proliferation in progeny cells. Lentiviral vectors have the additional advantage over vectors derived from oncoretroviruses (e.g., murine leukemia virus) in that they can transduce non-proliferative cells, such as hepatocytes. They also have the additional advantage of low immunogenicity. The retroviral vector may also be, for example, a γ retroviral vector. The γ retroviral vector may, for example, comprise a promoter, a packaging signal (ψ), a primer binding site (PBS), one or more (e.g., two) long terminal repeats (LTRs), and a transgene of interest, such as a gene encoding a CAR. The γ retroviral vector may lack viral structural genes such as gag, pol, and env.

An example of a promoter capable of expressing the CAR transgene in a mammalian T cell is the EF1a promoter. The natural EF1a promoter drives the expression of an α subunit of an elongation factor-1 complex, the α subunit being responsible for the enzymatic delivery of aminoacyl tRNA to the ribosome. The EF1a promoter has been widely used in mammalian expression plasmids and has been shown to efficiently drive the CAR expression from a transgene cloned into a lentiviral vector. See, e.g., Milone et al., Mol. Ther. 17(8): 1453-1464 (2009).

Another example of a promoter is the immediate early cytomegalovirus (CMV) promoter sequence. This promoter sequence is a constitutive strong promoter sequence capable of driving high-level expression of any polynucleotide sequence operatively linked thereto. However, other constitutive promoter sequences may also be used, including, but not limited to, simian virus 40 (SV40) early promoter, mouse mammary tumor virus (MMTV) promoter, human immunodeficiency virus (HIV) long terminal repeat (LTR) promoter, MoMuLV promoter, avian leukemia virus promoter, Epstein-Barr virus immediate early promoter, Rous sarcoma virus promoter, and human gene promoters such as, but not limited to, actin promoter, myosin promoter, elongation factor-1α promoter, hemoglobin promoter, and creatine kinase promoter. Additionally, the present invention should not be limited to the use of constitutive promoters. Inducible promoters are also contemplated as part of the present invention.

In some embodiments, the present invention provides a method for expressing the CAR construct of the present invention in a mammalian immune effector cell (e.g., a mammalian T cell) and an immune effector cell produced thereby.

The cell source (e.g., an immune effector cell, such as a T cell) is obtained from a subject. The term "subject" is intended to include living organisms in which an immune response can be elicited (e.g., mammals). T cells may be obtained from many sources, including peripheral blood mononuclear cells, bone marrow, lymph node tissues, cord blood, thymus tissues, tissues from the sites of infection, ascites, pleural effusion, spleen tissues, and tumors. T cells may be obtained from blood components collected from a subject using any technique known to those skilled in the art, such as Ficoll^{™} separation. In a preferred aspect, cells from the circulating blood of an individual are obtained by apheresis. Apheresis products typically contain lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or culture medium for subsequent processing steps. In one aspect of the present invention, the cells are washed with phosphate-buffered saline (PBS).

Specific subpopulations of T cells, such as CD3+, CD28+, CD4+, CD8+, CD45RA+ and CD45RO+ T cells, may be further isolated by positive or negative selection techniques. For example, in one embodiment, T cells are isolated by incubation with anti-CD3/anti-CD28 conjugated beads (e.g., DYNABEADS^{®} M-450 CD3/CD28 T) for a time period sufficient for positive selection of the desired T cells. In some embodiments, the time period is approximately between 30 min and 36 h, or longer. Longer incubation times may be used to isolate T cells in any situation where there are small numbers of T cells, such as for isolating tumor infiltrating lymphocytes (TILs) from tumor tissues or from immunocompromised individuals. Additionally, using longer incubation times can increase the efficiency of capturing CD8+ T cells. Therefore, by simply shortening or extending the time, allowing T cells to bind to the CD3/CD28 beads, and/or by increasing or decreasing the bead-to-T cell ratio, the subpopulations of T cells can be preferentially selected at the beginning of the culture or at other time points during the culture process.

Enrichment of a population of T cells can be accomplished by a negative selection process using a combination of antibodies directed against surface markers unique to the negatively selected cells. One method is to sort and/or select cells by negative magnetic immunoadhesion or flow cytometry that uses a mixture of monoclonal antibodies directed against cell surface markers present on the negatively selected cells.

In some embodiments, the immune effector cell may be an allogeneic immune effector cell, such as a T cell. For example, the cell may be an allogeneic T cell, such as an allogeneic T cell lacking the expression of a functional T cell receptor (TCR) and/or a human leukocyte antigen (HLA) (e.g., HLA class I and/or HLA class II).

A T cell lacking a functional TCR may, for example, be engineered such that it does not express any functional TCR on its surface; engineered such that it does not express one or more subunits that form a functional TCR (e.g., engineered such that it does not express or exhibits reduced expression of TCRα, TCRβ, TCRγ, TCRδ, TCRε, and/or TCRζ); or engineered such that it produces very little functional TCR on its surface.

The T cell described herein may, for example, be engineered such that it does not express a functional HLA on its surface. For example, the T cell described herein may be engineered such that the expression of HLA (e.g., HLA class I and/or HLA class II) on the cell surface is down-regulated. In some aspects, the down-regulation of HLA may be achieved by reducing or eliminating the expression of β-2 microglobulin (B2M).

In some embodiments, the T cell may lack a functional TCR and a functional HLA, e.g., HLA class I and/or HLA class II.

In one embodiment, a cell transduced with the nucleic acid encoding the CAR described herein is proliferated, e.g., the cell is proliferated in culture for 2 h to about 14 days.

The CAR-expressing immune effector cell obtained after the *in-vitro* proliferation may be tested for effector function as described in the examples.

### IV. Antibody specifically binding to CLDN18.2 molecule and antibody comprising mutated Fc domain thereof

CLDN18 is a member of the Claudin protein family, which is an important molecule that forms the tight junction of epithelial cells, determines the permeability of epithelial cells, and also acts as a barrier to the diffusion of proteins and lipids on the cell membrane surface (Gunzel, D. and A. S. Yu (2013). "Claudins and the modulation of tight junction permeability." Physiol Rev 93(2): 525-569). The human CLDN18 gene has two different exons 1, which, after transcription, undergo alternative splicing to ultimately produce two protein isoforms, CLDN18.1 and CLDN18.2, that only differ in their N-terminal sequences. The two CLDN18 isoform proteins each consist of 261 amino acids and have four transmembrane domains, but they are distributed in different tissues; CLDN18.1 is primarily expressed in lung tissue, while CLDN18.2 is expressed only in differentiated gastric mucosal epithelial cells and not in gastric stem cells (Sahin, Ugur et al., "Claudin-18 splice variant 2 is a pan-cancer target suitable for therapeutic antibody development." Clinical Cancer Research 14.23 (2008): 7624-7634).

CLDN18.2 is highly expressed in a variety of tumor tissues, such as non-small cell lung cancer (25%), gastric cancer (70%), pancreatic cancer (50%) and esophageal cancer (30%), but is hardly expressed in normal tissues (Kumar, V. et al., (2018) "Emerging Therapies in the Management of Advanced-Stage Gastric Cancer." Front Pharmacol 9: 404); it has now become a very promising target for anti-cancer drug action due to its differential expression between tumor cells and normal tissues.

Among the antibody drugs targeting CLDN18.2 in the prior art, the antibody drug that has made the most progress is Zolbetuximab (also referred to herein simply as "Zmab") developed by the German company Ganymed. Zolbetuximab is a human-mouse chimeric IgG1 monoclonal antibody specifically targeting CLDN18.2, which binds to the first extracellular region of CLDN18.2 expressed on tumor cells and induces tumor cell death by antibody-dependent cell-mediated cytotoxicity (ADCC) and complement-dependent cytotoxicity (CDC). In a phase II trial for gastric cancer, Zolbetuximab significantly prolonged patient survival compared to standard chemotherapy (the survival with standard chemotherapy was 8.4 months, while the survival with Zmab treatment was 13.2 months), and the therapeutic effect of Zmab was more pronounced in patients with high expression of Claudin18.2.

Although there are currently clinical monoclonal antibody drugs targeting the CLDN18.2 target under research, the monoclonal antibodies differ in their affinity for antigens. The inventors have developed a class of anti-CLDN18.2 antibodies with suitable affinity for specifically recognizing CLDN18.2, comprising a heavy chain variable region and a light chain variable region, wherein
(a) the heavy chain variable region comprises, according to the Kabat numbering, a CDR H1 shown in the amino acid sequence SYVMS (SEQ ID NO: 60), or a variant of the CDR H1 with no more than 2 amino acid changes or no more than 1 amino acid change; a CDR H2 shown in the amino acid sequence TISHSGGSTYYADSVKG (SEQ ID NO: 61), or a variant of the CDR H2 with no more than 2 amino acid changes or no more than 1 amino acid change; and a CDR H3 shown in the amino acid sequence DAPYYDILTGYRY (SEQ ID NO: 62), or a variant of the CDR H3 with no more than 2 amino acid changes or no more than 1 amino acid change; and the light chain variable region comprises, according to the Kabat numbering, a CDR L1 shown in the amino acid sequence RASQSISSWLA (SEQ ID NO: 63), or a variant of the CDR L1 with no more than 2 amino acid changes or no more than 1 amino acid change; a CDR L2 shown in the amino acid sequence KASSLES (SEQ ID NO: 64), or a variant of the CDR L2 with no more than 2 amino acid changes or no more than 1 amino acid change; and a CDR L3 shown in the amino acid sequence QQYNSYSYT (SEQ ID NO: 65), or a variant of the CDR L3 with no more than 2 amino acid changes or no more than 1 amino acid change;
(b) the heavy chain variable region comprises, according to the Kabat numbering, a CDR H1 shown in the amino acid sequence SYNIH (SEQ ID NO: 66), or a variant of the CDR H1 with no more than 2 amino acid changes or no more than 1 amino acid change; a CDR H2 shown in the amino acid sequence YIAPFQGDSRYNQKFKG (SEQ ID NO: 67), or a variant of the CDR H2 with no more than 2 amino acid changes or no more than 1 amino acid change; and a CDR H3 shown in the amino acid sequence LNRGNSLDY (SEQ ID NO: 68), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 69), or a variant of the CDR L1 with no more than 2 amino acid changes or no more than 1 amino acid change; a CDR L2 shown in the amino acid sequence WASTRES (SEQ ID NO: 70), or a variant of the CDR L2 with no more than 2 amino acid changes or no more than 1 amino acid change; and a CDR L3 shown in the amino acid sequence QNNYIYPLT (SEQ ID NO: 71), or a variant of the CDR L3 with no more than 2 amino acid changes or no more than 1 amino acid change;
(c) the heavy chain variable region comprises, according to the Kabat numbering, a CDR H1 shown in the amino acid sequence SYNIH (SEQ ID NO: 72), or a variant of the CDR H1 with no more than 2 amino acid changes or no more than 1 amino acid change; a CDR H2 shown in the amino acid sequence YIAPFQGDARYNQKFKG (SEQ ID NO: 73), or a variant of the CDR H2 with no more than 2 amino acid changes or no more than 1 amino acid change; and a CDR H3 shown in the amino acid sequence LNRGQSLDY (SEQ ID NO: 74), or a variant of the CDR H3 with no more than 2 amino acid changes or no more than 1 amino acid change; and the light chain variable region comprises, according to the Kabat numbering, a CDR L1 shown in the amino acid sequence KSSQSLFNAGNQRNYLT (SEQ ID NO: 75), or a variant of the CDR L1 with no more than 2 amino acid changes or no more than 1 amino acid change; a CDR L2 shown in the amino acid sequence WASTRES (SEQ ID NO: 76), or a variant of the CDR L2 with no more than 2 amino acid changes or no more than 1 amino acid change; and a CDR L3 shown in the amino acid sequence QNNYIYPLT (SEQ ID NO: 77), or a variant of the CDR L3 with no more than 2 amino acid changes or no more than 1 amino acid change;
(d) the heavy chain variable region comprises, according to the Kabat numbering, a CDR H1 shown in the amino acid sequence SYNIH (SEQ ID NO: 78), or a variant of the CDR H1 with no more than 2 amino acid changes or no more than 1 amino acid change; a CDR H2 shown in the amino acid sequence YIAPFQGDARYNQKFKG (SEQ ID NO: 79), or a variant of the CDR H2 with no more than 2 amino acid changes or no more than 1 amino acid change; and a CDR H3 shown in the amino acid sequence LNRGNALDY (SEQ ID NO: 80), or a variant of the CDR H3 with no more than 2 amino acid changes or no more than 1 amino acid change; and the light chain variable region comprises, according to the Kabat numbering, a CDR L1 shown in the amino acid sequence KSSQSLFQSGNQRNYLT (SEQ ID NO: 81), or a variant of the CDR L1 with no more than 2 amino acid changes or no more than 1 amino acid change; a CDR L2 shown in the amino acid sequence WASTRES (SEQ ID NO: 82), or a variant of the CDR L2 with no more than 2 amino acid changes or no more than 1 amino acid change; and a CDR L3 shown in the amino acid sequence QNNYIYPLT (SEQ ID NO: 83), or a variant of the CDR L3 with no more than 2 amino acid changes or no more than 1 amino acid change;
(e) the heavy chain variable region comprises, according to the Kabat numbering, a CDR H1 shown in the amino acid sequence SYNIS (SEQ ID NO: 84), or a variant of the CDR H1 with no more than 2 amino acid changes or no more than 1 amino acid change; a CDR H2 shown in the amino acid sequence YIAPFQGDARYNQKFKG (SEQ ID NO: 85), or a variant of the CDR H2 with no more than 2 amino acid changes or no more than 1 amino acid change; and a CDR H3 shown in the amino acid sequence LNRGNSLDY (SEQ ID NO: 86), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 87), or a variant of the CDR L1 with no more than 2 amino acid changes or no more than 1 amino acid change; a CDR L2 shown in the amino acid sequence WASTRES (SEQ ID NO: 88), or a variant of the CDR L2 with no more than 2 amino acid changes or no more than 1 amino acid change; and a CDR L3 shown in the amino acid sequence QNNYIYPLT (SEQ ID NO: 89), or a variant of the CDR L3 with no more than 2 amino acid changes or no more than 1 amino acid change; or
(f) the heavy chain variable region comprises, according to the Kabat numbering, a CDR H1 shown in the amino acid sequence TYWMH (SEQ ID NO: 90), or a variant of the CDR H1 with no more than 2 amino acid changes or no more than 1 amino acid change; a CDR H2 shown in the amino acid sequence LIDPSDSETRLNQKFKD (SEQ ID NO: 91), or a variant of the CDR H2 with no more than 2 amino acid changes or no more than 1 amino acid change; and a CDR H3 shown in the amino acid sequence WGQGTLVTVSS (SEQ ID NO: 92), or a variant of the CDR H3 with no more than 2 amino acid changes or no more than 1 amino acid change; and the light chain variable region comprises, according to the Kabat numbering, a CDR L1 shown in the amino acid sequence KSSQSLLNSGNQKNYLT (SEQ ID NO: 93), or a variant of the CDR L1 with no more than 2 amino acid changes or no more than 1 amino acid change; a CDR L2 shown in the amino acid sequence WASTRES (SEQ ID NO: 94), or a variant of the CDR L2 with no more than 2 amino acid changes or no more than 1 amino acid change; and a CDR L3 shown in the amino acid sequence QNDYSYPLT (SEQ ID NO: 95), or a variant of the CDR L3 with no more than 2 amino acid changes or no more than 1 amino acid change;
wherein the amino acid change is an addition, deletion or conservative substitution of an amino acid.

In some embodiments, the antibody binding to the CLDN18.2 molecule disclosed herein binds to mammalian CLDN18.2, such as human CLDN18.2.

In some embodiments, the antibody binding to the CLDN18.2 molecule disclosed herein has one or more of the following properties:
(1) it specifically binds to CLDN18.2 and does not bind to CLDN18.1;
(2) it kills CLDN18.2-positive cancer cells through antibody-dependent cell-mediated cytotoxicity and/or complement-dependent cytotoxicity, in the absence of a mutated Fc domain, e.g., in the presence of an unmutated parent antibody Fc domain.

In some embodiments, the antibody binding to the CLDN18.2 molecule disclosed herein comprises a heavy chain variable region and a light chain variable region that specifically bind to CLDN18.2 but do not bind or substantially do not bind to CLDN18.1, wherein
(a) the heavy chain variable region comprises the sequence of SEQ ID NO: 34 or a sequence having at least 90%, 91%, 92%, 93%, 94%, 95%, 96%, 97%, 98% or 99% identity thereto, and the light chain variable region comprises the sequence of SEQ ID NO: 35 or a sequence having at least 90%, 91%, 92%, 93%, 94%, 95%, 96%, 97%, 98% or 99% identity thereto;
(b) the heavy chain variable region comprises the sequence of SEQ ID NO: 36 or a sequence having at least 90%, 91%, 92%, 93%, 94%, 95%, 96%, 97%, 98% or 99% identity thereto, and the light chain variable region comprises the sequence of SEQ ID NO: 37 or a sequence having at least 90%, 91%, 92%, 93%, 94%, 95%, 96%, 97%, 98% or 99% identity thereto;
(c) the heavy chain variable region comprises the sequence of SEQ ID NO: 44 or a sequence having at least 90%, 91%, 92%, 93%, 94%, 95%, 96%, 97%, 98% or 99% identity thereto, and the light chain variable region comprises the sequence of SEQ ID NO: 45 or a sequence having at least 90%, 91%, 92%, 93%, 94%, 95%, 96%, 97%, 98% or 99% identity thereto;
(d) the heavy chain variable region comprises the sequence of SEQ ID NO: 46 or a sequence having at least 90%, 91%, 92%, 93%, 94%, 95%, 96%, 97%, 98% or 99% identity thereto, and the light chain variable region comprises the sequence of SEQ ID NO: 47 or a sequence having at least 90%, 91%, 92%, 93%, 94%, 95%, 96%, 97%, 98% or 99% identity thereto;
(e) the heavy chain variable region comprises the sequence of SEQ ID NO: 48 or a sequence having at least 90%, 91%, 92%, 93%, 94%, 95%, 96%, 97%, 98% or 99% identity thereto, and the light chain variable region comprises the sequence of SEQ ID NO: 49 or a sequence having at least 90%, 91%, 92%, 93%, 94%, 95%, 96%, 97%, 98% or 99% identity thereto; or
(f) the heavy chain variable region comprises the sequence of SEQ ID NO: 42 or a sequence having at least 90%, 91%, 92%, 93%, 94%, 95%, 96%, 97%, 98% or 99% identity thereto, and the light chain variable region comprises the sequence of SEQ ID NO: 43 or a sequence having at least 90%, 91%, 92%, 93%, 94%, 95%, 96%, 97%, 98% or 99% identity thereto;
wherein the amino acid change in the sequence having at least 90%, 91%, 92%, 93%, 94%, 95%, 96%, 97%, 98% or 99% identity is preferably a substitution of an amino acid, more preferably a conservative substitution of an amino acid; preferably, the amino acid change does not occur in the CDR.

In some embodiments, the antibody binding to the CLDN18.2 molecule disclosed herein is an IgG1, IgG2, IgG3 or IgG4 antibody, preferably an IgG1 or IgG4 antibody, more preferably an IgG1 antibody such as a human IgG1 antibody.

In some embodiments, the antibody binding to the CLDN18.2 molecule provided herein comprises a mutated Fc domain, wherein the amino acid at position P329 according to the EU numbering is mutated to glycine (G), and the binding of the mutated Fc domain to an Fcy receptor is reduced compared to the binding of an unmutated parent antibody Fc domain to the Fcy receptor; for example, the mutated Fc domain is a mutated Fc domain of an IgG1, IgG2, IgG3 or IgG4 antibody, preferably a mutated Fc domain of an IgG1 or IgG4 antibody, more preferably a mutated Fc domain of an IgG1 antibody such as a mutated Fc domain of a human IgG1 antibody. The antibody binding to the CLDN18.2 molecule and comprising the P329G mutated Fc domain is unable to exert antibody-dependent cell-mediated cytotoxicity by binding to the Fcy receptor and is also unable to exert complement-dependent cytotoxicity, and therefore cannot kill CLDN18.2-positive cancer cells.

In some embodiments, the present invention provides a nucleic acid encoding any of the above antibodies or fragments thereof binding to the CLDN18.2 molecule, or any one of chains thereof. In one embodiment, provided is a vector comprising the nucleic acid. In one embodiment, the vector is an expression vector. In one embodiment, provided is a host cell comprising the nucleic acid or the vector. In one embodiment, the host cell is eukaryotic. In another embodiment, the host cell is selected from a yeast cell, a mammalian cell (e.g., a CHO cell or 293 cell), or other cells suitable for preparing an antibody or an antigen-binding fragment thereof. In another embodiment, the host cell is prokaryotic.

For example, the nucleic acid of the present invention comprises a nucleic acid encoding the antibody binding to the CLDN18.2 molecule disclosed herein. In some embodiments, provided are one or more vectors comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is a pcDNA3.4 expression vector.

Once the expression vector or DNA sequence has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, lipid-based transfection, or other conventional techniques. In the case of protoplast fusion, cells are cultured in a culture medium and screened for appropriate activity. Methods and conditions for culturing the resulting transfected cells and for recovering the resulting antibody molecules are known to those skilled in the art and may be changed or optimized according to the particular expression vector and the particular mammalian host cell used based on the present description and methods known in the prior art.

Additionally, cells having stably incorporated DNA in chromosomes thereof can be selected by introducing one or more markers permitting the selection of transfected host cells. The markers may, for example, provide prototrophy, biocidal (e.g., antibiotics) resistance, or heavy metal (e.g., copper) resistance, etc., for an auxotrophic host. Selectable marker genes may be linked directly to a DNA sequence to be expressed or introduced through co-transformation into the same cell. Additional elements may also be required for optimal synthesis of mRNA. The elements may include splicing signals, transcriptional promoters, enhancers, and termination signals.

In one embodiment, provided is a host cell comprising the polynucleotide of the present invention. In some embodiments, provided is a host cell comprising the expression vector of the present invention. In some embodiments, the host cell is selected from a yeast cell, a mammalian cell, and other cells suitable for preparing an antibody. Suitable host cells include prokaryotic microorganisms, such as *E*. *coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include monkey kidney CV1 line (COS-7) transformed by SV40; human embryonic kidney line (HEK293 or 293F cells), 293 cell, baby hamster kidney cell (BHK), monkey kidney cell (CV1), African green monkey kidney cell (VERO-76), human cervical cancer cell (HELA), canine kidney cell (MDCK), buffalo rat liver cell (BRL 3A), human lung cell (W138), human liver cell (HepG2), Chinese hamster ovary cell (CHO cell), CHO-S cell, NSO cell, and myeloma cell line such as Y0, NS0, P3X63, and Sp2/0. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO cell or an HEK293 cell.

In one embodiment, the present invention provides a method for preparing an antibody binding to a CLDN18.2 molecule (including a P329G mutated antibody), wherein the method comprises culturing a host cell comprising a nucleic acid encoding the antibody binding to the CLDN18.2 molecule (including the P329G mutated antibody) or an expression vector comprising the nucleic acid under conditions suitable for expression of the nucleic acid encoding the antibody binding to the CLDN18.2 molecule (including the P329G mutated antibody), and optionally isolating the antibody binding to the CLDN18.2 molecule (including the P329G mutated antibody). In a certain embodiment, the method further comprises recovering the antibody binding to the CLDN18.2 molecule (including the P329G mutated antibody) from the host cell (or host cell culture medium).

The antibody binding to the CLDN18.2 molecule (including the P329G mutated antibody) disclosed herein prepared as described herein can be purified by known prior art such as high performance liquid chromatography, ion exchange chromatography, gel electrophoresis, affinity chromatography, and size exclusion chromatography. The actual conditions used to purify a particular protein also depend on factors such as net charge, hydrophobicity, and hydrophilicity, and these will be apparent to those skilled in the art. The purity of the antibody binding to the CLDN18.2 molecule (including the P329G mutated antibody) disclosed herein can be determined by any one of a variety of well-known analytical methods including size exclusion chromatography, gel electrophoresis, high performance liquid chromatography, and the like.

The antibody binding to the CLDN18.2 molecule (including the P329G mutated antibody) provided herein can be identified, screened, or characterized for physical/chemical properties and/or biological activities through a variety of assays known in the art. In one aspect, the antibody binding to the CLDN18.2 molecule (including the P329G mutated antibody) disclosed herein is tested for the antigen-binding activity, for example, by known methods such as FACS, ELISA, or Western blotting. The binding to CLDN18.2 can be assayed by methods known in the art, and exemplary methods are disclosed herein. In some embodiments, FACS is used to assay the binding of the antibody binding to the CLDN18.2 molecule (including the P329G mutated antibody) disclosed herein to cell surface CLDN18.2 (e.g., human CLDN18.2).

The present invention further provides an assay for identifying antibodies binding to CLDN18.2 molecules (including P329G mutated antibodies) with biological activities. The biological activities may include, for example, ADCC effect, CDC effect, and the like.

Cells for use with any of the above *in-vitro* assays include cell lines that naturally express CLDN18.2 or are engineered to express CLDN18.2. The cell lines engineered to express CLDN18.2 are cell lines that do not normally express CLDN18.2, but express CLDN18.2 after transfection of DNA encoding CLDN18.2 into cells.

### V. Pharmaceutical combination of the present invention

For the optimization of the safety and efficacy of CAR therapies, the molecular switch-regulated chimeric antigen receptor of the present invention is a regulatable CAR with controllable CAR activity. The present invention uses an antibody with a Pro329Gly mutation (a mutation from proline to glycine at position 329 in an antibody Fc fragment according to the EU numbering, abbreviated as P329G) as a safety switch in the CAR treatment of the present invention. In the absence of the P329G mutated antibody, the CAR activity of the present invention is turned off; in the presence of the P329G mutated antibody, the CAR activity of the present invention is turned on; thus, the turning on and off of the activity of the CAR molecule of the present invention is regulated by the P329G mutated antibody.

In some embodiments, the present invention provides a pharmaceutical combination, comprising (i) an immune effector cell (e.g., a T cell) expressing the molecular switch-regulated CAR polypeptide of the present invention; and (ii) a P329G mutated antibody specifically binding to a CLDN18.2 molecule. For example, the immune effector cell is a T cell expressing the molecular switch-regulated CAR polypeptide of the present invention prepared from an autologous T cell or an allogeneic T cell, e.g., the immune effector cell is a T cell expressing the molecular switch-regulated CAR polypeptide of the present invention prepared from a T cell isolated from a human PBMC. In some embodiments, the P329G mutated antibody is an HB37A6 PG Ab, an Hz69H9 PG Ab, an Hz69H9-1.2 PG Ab, an Hz69H9-2.1 PG Ab, an Hz69H9-SA PG Ab, and/or an Hz3G3 PG Ab.

In some embodiments, the present invention provides a pharmaceutical combination, comprising (i) a nucleic acid molecule encoding the molecular switch-regulated CAR polypeptide of the present invention or a vector comprising the nucleic acid component; and (ii) a P329G mutated antibody specifically binding to a CLDN18.2 molecule.

In some embodiments, the pharmaceutical combination of the present invention optionally further comprises a pharmaceutically acceptable supplementary material for a suitable formulation. For example, (ii) in the pharmaceutical combination may be formulated according to conventional methods (e.g., Remington's Pharmaceutical Science, latest edition, Mark Publishing Company, Easton, U.S.A). Examples of the pharmaceutically acceptable supplementary material include surfactants, excipients, colorants, perfuming agents, preservatives, stabilizers, buffers, suspending agents, isotonic agents, binders, disintegrants, lubricants, flow promoters, flavoring agents, and the like. Further, other commonly used carriers such as light anhydrous silicic acid, lactose, crystalline cellulose, mannitol, starch, calcium carboxymethyl cellulose, sodium carboxymethyl cellulose, hydroxypropyl cellulose, hydroxypropyl methylcellulose, polyvinyl acetal diethylaminoacetate, polyvinylpyrrolidone, gelatin, medium-chain fatty acid triglyceride, polyoxyethylene hardened castor oil 60, white sugar, carboxymethyl cellulose, corn starch, inorganic salts, etc. may also be suitably used as carriers, but are not limited thereto.

In some embodiments, the pharmaceutical combination of the present invention is for use in the treatment of a disease related to CLDN18.2, for example, a cancer expressing or overexpressing CLDN 18.2, such as a CLDN18.2-positive solid tumor.

### VI. Use of the pharmaceutical combination of the present invention and method of treatment using the pharmaceutical combination of the present invention

The present invention provides the aforementioned pharmaceutical combination of the present invention for use in the treatment of a disease related to CLDN18.2 in a subject, wherein the disease related to CLDN18.2 is, for example, a cancer expressing or overexpressing CLDN 18.2, such as a CLDN18.2-positive solid tumor.

In one embodiment, the pharmaceutical combination of the present invention is for use in the treatment of a cancer expressing or overexpressing CLDN 18.2 (e.g., a CLDN18.2-positive solid tumor) in a subject and is capable of reducing the severity of at least one symptom or indication of the cancer or inhibiting the growth of cancer cells.

The present invention provides a method for treating a disease related to CLDN18.2 (e.g., a cancer expressing or overexpressing CLDN 18.2, such as a CLDN18.2-positive solid tumor) in a subject, comprising administering to an individual in need a therapeutically effective amount of the pharmaceutical combination of the present invention.

The present invention provides use of the aforementioned pharmaceutical combination of the present invention in the preparation of a medicament for treating a disease related to CLDN18.2 (e.g., a cancer expressing or overexpressing CLDN 18.2, such as a CLDN18.2-positive solid tumor).

The pharmaceutical combination of the present invention may also be administered to an individual who has been treated for a cancer with one or more prior therapies but subsequently relapsed or metastasized.

In some embodiments, the (i) immune effector cell (e.g., T cell) expressing the molecular switch-regulated CAR polypeptide of the present invention and the (ii) P329G mutated antibody specifically binding to the CLDN18.2 molecule in the pharmaceutical combination of the present invention are used for parenteral, transdermal, intracavitary, intraarterial, intravenous or intrathecal administration, or direct injection into a tissue or tumor. In some embodiments, the (ii) P329G mutated antibody specifically binding to the CLDN18.2 molecule in the pharmaceutical combination of the present invention is administered prior to, simultaneously with, or subsequent to (i) the immune effector cell (e.g., T cell) expressing the molecular switch-regulated CAR polypeptide of the present invention.

In some embodiments, the (i) immune effector cell expressing the molecular switch-regulated CAR polypeptide of the present invention in the pharmaceutical combination of the present invention is a T cell expressing the CAR polypeptide of the present invention prepared from an autologous T cell or an allogeneic T cell; the (ii) P329G mutated antibody specifically binding to the CLDN18.2 molecule in the pharmaceutical combination of the present invention is any antibody specifically binding to the CLDN18.2 molecule, which comprises a P329G mutation. Preferably, the P329G mutated antibody is an HB37A6 PG Ab, an Hz69H9 PG Ab, an Hz69H9-1.2 PG Ab, an Hz69H9-2.1 PG Ab, an Hz69H9-SA PG Ab, and/or an Hz3G3 PG Ab.

When component (i) in the pharmaceutical combination of the present invention is an immune effector cell (e.g., a T cell) expressing the molecular switch-regulated CAR polypeptide of the present invention, the present invention does not limit the order in which component (i) and component (ii) in the pharmaceutical combination of the present invention are administered to the subject, nor does it limit the timing arrangement between the administration of component (i) and component (ii) in the pharmaceutical combination of the present invention to the subject. Therefore, (i) and (ii) in the pharmaceutical combination of the present invention may be administered separately, simultaneously, or sequentially. When the two components are not administered simultaneously, the two components may be separated apart by 1 min, 5 min, 15 min, 30 min, 45 min, 1 h, 2 h, 4 h, 6 h, 12 h, 24 h, 48 h, or 72 h, or any suitable time readily determined by those skilled in the art. For example, (i) is administered intravenously on the first day, (ii) is administered on the second day, and then (ii) is administered multiple times at a certain frequency, while an *in-vivo* concentration of (i) and a desired therapeutic efficacy endpoint are monitored to determine whether to administer (i) multiple times; or (ii) is administered on the first day, (i) is administered intravenously on the second day, and then (ii) is administered multiple times at a certain frequency, while an *in-vivo* concentration of (i) and a desired therapeutic efficacy endpoint are monitored to determine whether to administer (i) multiple times.

In some embodiments, (i) and (ii) in the pharmaceutical combination of the present invention are each administered once, and then (ii) is administered multiple times at a frequency of once every 3-4 days, once a week, once every two weeks, once every three weeks, or once every four weeks, while the *in-vivo* concentration of (i) and the desired therapeutic efficacy endpoint are detected to determine whether to administer (i) multiple times.

In some embodiments, when component (i) in the pharmaceutical combination of the present invention is an immune effector cell (e.g., a T cell) expressing the molecular switch-regulated CAR polypeptide of the present invention, the case where component (i) and component (ii) are pre-incubated together before being administered to the subject is also included. Therefore, the two components may be pre-incubated for 1 min, 5 min, 10 min, 15 min, 30 min, 45 min, or 1 h, or any suitable time readily determined by those skilled in the art, before being administered.

The pharmaceutical combination of the present invention may be administered to the subject at an appropriate dose. The dose regimen will be determined by the attending physician and clinical factors. As is well known in the medical field, the dose for any one patient depends on many factors, including the patient's body weight, body surface area, age, the specific compound to be administered, sex, time and route of administration, general health condition, and other drugs to be administered concurrently.

In some embodiments, when component (i) of the pharmaceutical combination of the present invention is an immune effector cell (e.g., a T cell) expressing the molecular switch-regulated CAR polypeptide of the present invention, component (i) is administered intravenously at a dose of 1 × 10⁶-1 × 10¹² immune effector cells, preferably 5 × 10⁶-1 × 10¹¹ immune effector cells, more preferably 1 × 10⁷-1 × 10¹⁰ immune effector cells, such as 5 × 10⁷ immune effector cells, 2.5 × 10⁸ immune effector cells, 7.5 × 10⁸ immune effector cells, or 1.25 × 10⁹ immune effector cells, either in a single administration or in multiple administrations; and (ii) is administered, preferably parenterally, more preferably intravenously, in the form of a dose unit of 0.1-10 mg/kg, preferably 0.3 mg/kg, 0.5 mg/kg, 1 mg/kg, 3 mg/kg, 5 mg/kg, 7 mg/kg, or 9 mg/kg.

In some embodiments, the administration of the pharmaceutical combination of the present invention to the individual with the cancer results in the complete disappearance of the tumor. In some embodiments, the administration of the pharmaceutical combination of the present invention to the individual with the cancer results in a reduction in tumor cells or tumor size of at least 85% or more. The reduction in the tumor may be measured by any method known in the art, such as X-ray, positron emission tomography (PET), computed tomography (CT), magnetic resonance imaging (MRI), cytology, histology, or molecular genetic analysis.

In some embodiments, the pharmaceutical combination of the present invention may reduce the "on-target/off-tumor" toxicity present associated with CAR-T cells.

### VII. Kit of the present invention

The present invention provides a kit of parts, comprising the pharmaceutical combination of the present invention, wherein preferably, the kit is in the form of a pharmaceutical dose unit. Dose units may thus be provided according to the administration regimen or the interval between drug administrations.

In one embodiment, the kit of parts of the present invention comprises in the same package:
(i) an immune effector cell (e.g., a T cell) expressing the molecular switch-regulated CAR polypeptide of the present invention, a nucleic acid molecule encoding the molecular switch-regulated CAR polypeptide of the present invention, a vector comprising the nucleic acid, or any combination thereof; and
(ii) a P329G mutated antibody specifically binding to a CLDN18.2 molecule.

It should be understood that the various embodiments/technical solutions and the features in the embodiments/technical solutions described herein can be combined with each other arbitrarily, and each of the solutions resulting from these mutual combinations is included within the scope of the present invention, as if the solutions resulting from these mutual combinations are specifically and individually listed herein, unless the context clearly indicates otherwise.

The following examples are described to assist in understanding the present invention. The examples are not intended to be and should not be interpreted in any way as limiting the protection scope of the present invention.

### EXAMPLES

### Example 1. CAR Gene Synthesis and Viral Expression Vector Construction

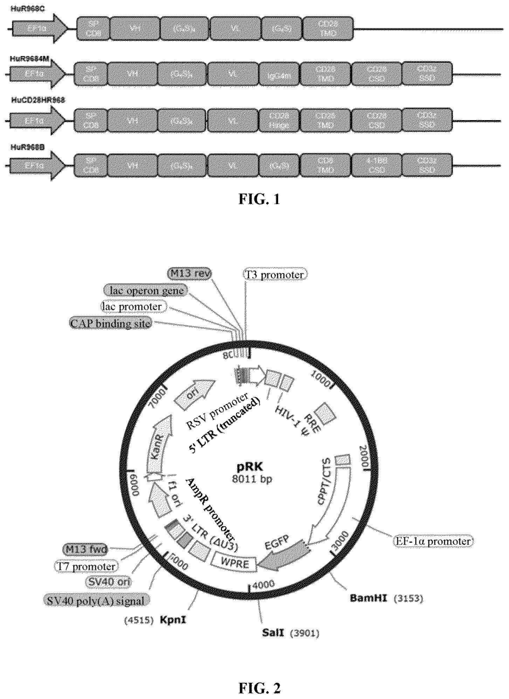

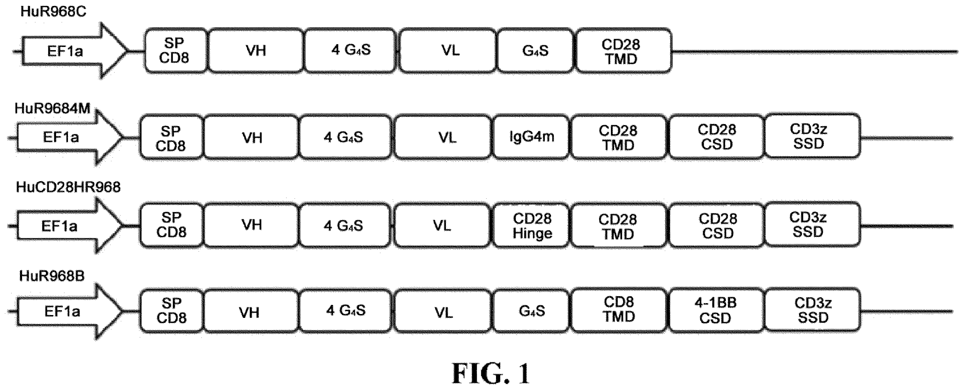

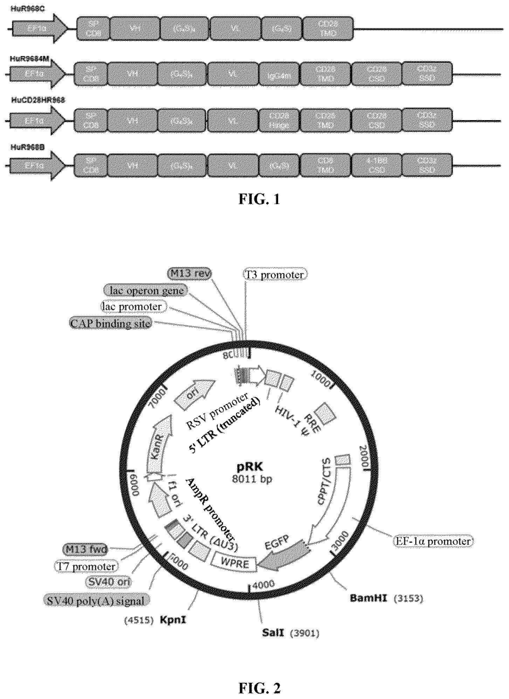

DNA fragments set forth in SEQ ID NO: 16, SEQ ID NO: 22, SEQ ID NO: 24, and SEQ ID NO: 26 were synthesized, which encode the amino acid sequences set forth in SEQ ID NO: 15, SEQ ID NO: 21, SEQ ID NO: 23, and SEQ ID NO: 25, respectively, corresponding to the 4 different CAR constructs HuR968C, HuR9684M, HuCD28HR968, and HuR968B shown in FIG. 1, respectively.

These CAR constructs (hereinafter sometimes also referred to as P329 CAR; P329G CAR; PG CAR) contained the same amino-terminal (N-terminal) signal peptide sequence (SEQ ID NO: 1), anti-P329G mutation antibody heavy chain variable region VH sequence (SEQ ID NO: 2), linker sequence (SEQ ID NO: 4), and anti-P329G mutation antibody light chain variable region VL (SEQ ID NO: 3).

At the C-terminus of the anti-P329G mutation antibody light chain variable region VL, the CAR constructs HuR968C and HuR968B each contained a G4S hinge region sequence (SEQ ID NO: 5), the construct HuR9684M contained a mutated hinge region sequence derived from IgG4 (SEQ ID NO: 6), HuCD28HR968 contained a hinge region sequence derived from a CD28 molecule (SEQ ID NO: 7); HuR968C, HuR9684M, and HuCD28HR968 each contained a transmembrane region sequence derived from the CD28 molecule (SEQ ID NO: 8), HuR968B contained a transmembrane region sequence derived from the CD8 molecule (SEQ ID NO: 9); HuR968C lacked an intracellular signaling sequence, HuR9684M and HuCD28HR968 each contained a co-stimulatory signaling sequence derived from the CD28 molecule (SEQ ID NO: 10), and HuR968B contained a co-stimulatory signaling sequence derived from the 4-1BB molecule (SEQ ID NO: 11); with the exception of HuR968C, all other CAR constructs each contained an intracellular stimulatory signaling domain derived from the CD3ζ chain (CD247) (SEQ ID NO: 13).

Additionally, an 8E5 CAR (SEQ ID NO: 31), artificially synthesized to directly target a CLDN18.2 molecule, was used as a positive control for the direct killing of tumor cells expressing the CLDN18.2 molecule by CAR-T cells. The 8E5 CAR contained, from N-terminus to C-terminus, a signal peptide, an anti-CLDN18.2 scFv, a hinge region of a CD8α molecule (SEQ ID NO: 18), the transmembrane region of the CD28 molecule (SEQ ID NO: 8), the CD28 co-stimulatory signaling domain (SEQ ID NO: 10), and a CD3ζ chain intracellular activation domain (SEQ ID NO: 17).

A CD16-158V CAR targeting and binding to an antibody Fc fragment (SEQ ID NO: 32) was also constructed. The CD16-158V CAR contained, from N-terminus to C-terminus, a signal peptide, an extracellular segment of a CD16 molecule (158V), the transmembrane region of the CD28 molecule (SEQ ID NO: 8), the CD28 co-stimulatory signaling domain (SEQ ID NO: 10), and the CD3ζ chain intracellular activation domain (SEQ ID NO: 13). The high-affinity CD16 158V variant binds to both wild-type and Fc-engineered antibodies, being capable of strongly promoting the activity of CD16-158V CAR T cells. The CD16-158V CAR was used as a positive control that worked in combination with an antibody.

Additionally, a Blue21 CAR targeting BCMA (SEQ ID NO: 33) was constructed for use as a negative control. The Blue21 CAR contained, from N-terminus to C-terminus, a signal peptide, an anti-BCMA single-chain antibody (from clone 11D53), the hinge region of the CD8α molecule (SEQ ID NO: 18), the transmembrane region of the CD8α molecule (SEQ ID NO: 9), the 4-1BB co-stimulatory signaling domain (SEQ ID NO: 11), and the CD3ζ chain intracellular activation domain (SEQ ID NO: 13).

The elements of a pRK lentiviral expression vector are shown in FIG. 2. The above DNA fragments encoding the CAR polypeptides were separately inserted downstream of an EF1α promoter of the pRK lentiviral expression vector (restructured from a pRRLSIN.cPPT.PGK-GFP.WPRE vector (Addgene, 12252, purchased from Biofeng) by replacing the promoter and resistance gene) to replace the EGFR sequence in the vector, such that CAR expression plasmids (pRK-HuR968C, pRK-HuR9684M, pRK-HuCD28HR968, pRK-HuR968B, pRK-8E5, pRK-CD16, and pRK-Blue21) targeting the P329G mutation or for various controls were obtained.

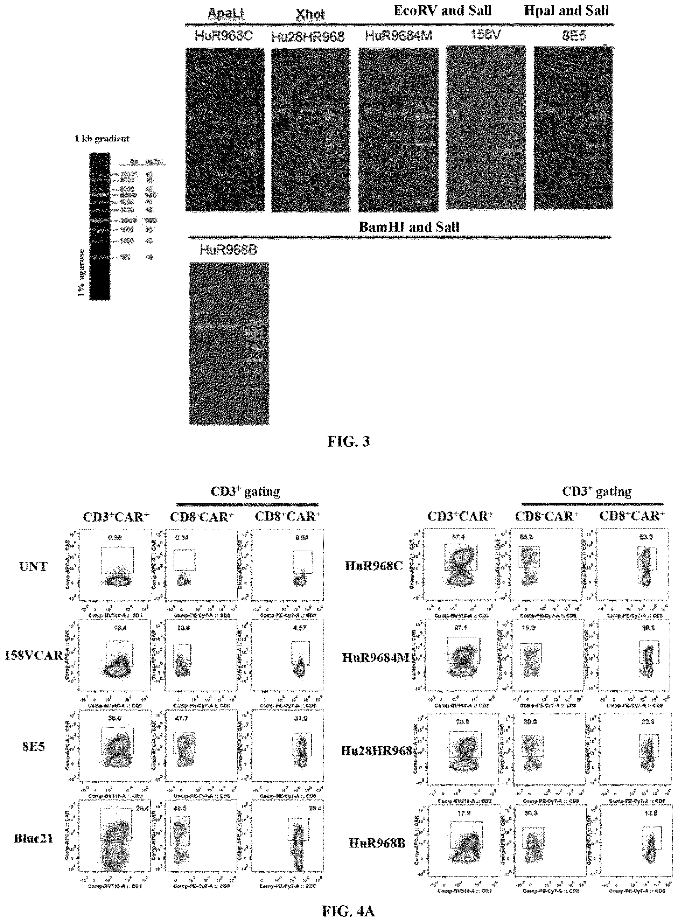

The lentivirus expression vectors for expressing the CARs were each digested with a restriction endonuclease and identified by electrophoresis. The results are shown in FIG. 3, indicating that the lentivirus expression vectors for expressing the CARs were correctly constructed.

### Example 2. CAR-T Cell Preparation, CAR Expression Detection, and Phenotype Detection

### (2-1) Preparation of lentivirus concentrate

Each CAR expression plasmid prepared in Example 1 was co-transfected with a structural plasmid pMDLg/pRRE (Addgene, 12251, purchased from Biofeng), a regulatory plasmid pRSV-rev (Addgene, 12253, purchased from Biofeng), and an envelope plasmid pMD2G (Addgene, 12259, purchased from Biofeng) at a mass ratio of 3:3:2:2 into Lenti-X-293T cells (Takara) using the PEI transfection method. 16 h after transfection, the medium was changed to a fresh DEME medium containing 2% fetal bovine serum (FBS). The cells were cultured for another 48 h, and then the cell supernatant was collected and centrifuged to remove cell debris. PEG8000 was then added, and the mixture was incubated at 4 °C for 16-64 h for lentivirus concentration. After another centrifugation, the supernatant was discarded, and the lentivirus pellet was resuspended in a T cell culture medium to obtain a lentivirus concentrate, which was then aliquoted and subjected to cryopreservation at -80 °C.

### (2-2) Lentiviral titer detection

Lenti-X-293T cells (Takara) were digested and then resuspended in a DMEM medium containing 8 µg/mL Polybrene (Sigma, H9268-5G). The cell suspension was then added to a 24-well plate, and different volumes of the lentivirus concentrate obtained from Example 2-1 were added. The mixture was incubated for 72 h, and then transduction of 293T cells was performed.

The 293T cells transduced with the lentivirus concentrate were digested and then stained with Biotin-SP-conjugated anti-Human IgG, F(ab')₂-specific (Jackson ImmunoResearch, 109-066-006) and APC-Streptavidin (BioLegend, 405207). The proportion of APC-positive cells was detected using flow cytometry. The viral titer (TU/mL) was calculated based on the initial cell count, the virus volume, and the proportion of positive cells.

### (2-3) T cell sorting, infection, and CAR-T expansion culture

Recombinant human interleukin-2 for injection (NMPA Approval No. S20040020) was added to TexMACS GMP Medium (Miltenyi Biotec, 170-076-309) to prepare a T cell culture medium with an IL-2 concentration of 200 IU/mL.

On Day 0, thawed PBMCs were sorted using the Pan T Cell Isolation Kit (human) (Miltenyi, 130-096-535) to obtain T cells, which were then resuspended in the T cell culture medium to a certain density and activated by adding TransAct (Miltenyi, 130-111-160).

On Day 1, a certain amount of T cells was separated out and then cultured without the addition of lentivirus; this portion of T cells served as un-transduced T cells (UNT). Different types of lentivirus concentrates obtained from Example 2-1 were added to the remaining cells at an MOI of 1-5, and the T cells were pipetted uniformly. On Day 2, the cells were centrifuged to remove the viral supernatant and then resuspended in a fresh T cell culture medium. The UNT cells were not subjected to any manipulation. On day 3, all cells were transferred to G-Rex (WILSONWOLF, Catalog No. 80040S), and an appropriate amount of fresh T cell culture medium was added. The cells were then placed in a CO₂ incubator at 37 °C for static culture. Every 2-3 days, half of the culture medium was replaced with a fresh culture medium, or IL-2 was added directly until the IL-2 concentration in the cell culture medium was 200 IU/mL. When the number of cells was expanded to about 20-80 times, meeting the requirement (generally reaching (2-8) × 10⁸ cells), the cells were harvested. After centrifugation to remove the culture medium, the CAR-T cells were resuspended in CryoStor^{®} CS10 (Stemcell, 07930), then aliquoted, and subjected to programmed cooling to -80 °C for cryopreservation.

### (2-4) CAR expression detection

An appropriate amount of the CAR-T cells obtained from the above Example 2-3 were taken, washed once with an FACS buffer (PBS + 2% FBS), resuspended, and then stained at room temperature for 10-15 min with an FACS buffer containing LIVE/DEAD Fixable Dead Cell Stain (Thermo, L34963). After two washes, an antibody combination of PerCP-Cy5.5-CD3 (BD, 560835), BUV805-CD8 (BD, 749366), and Biotin-F(ab')₂ Fragment Goat Anti-Human IgG (Jackson ImmunoResearch, 109-066-006) was added. For the detection of the Blue21 CAR, Biotin-F(ab')₂ Fragment Goat Anti-Mouse IgG antibody (Jackson ImmunoResearch, 115-066-006) was used instead; after two washes, APC-streptavidin was added, and the cells were stained at 4 °C for 30-45 min. For the detection of the CD16 (158V) CAR, an antibody combination of PerCP-Cy5.5-CD3 (BD, 560835), BUV805-CD8 (BD, 749366), and Allophycocyanin (APC)-Fragment Goat Anti-Human IgG (Jackson ImmunoResearch, 109-136-098) was added directly. The cells were washed twice, then resuspended in an FACS buffer, and detected using a flow cytometer.

### (2-5) CAR-T cell phenotype detection:

An appropriate amount of CAR-T cells were taken, washed once with an FACS buffer, resuspended, and then stained at room temperature for 10-15 min with an FACS buffer containing LIVE/DEAD Fixable Dead Cell Stain. After two washes, an antibody combination of PerCP-Cy5.5-CD3 (BD, 560835), BUV805-CD8 (BD, 749366), APC-Cy7-CD45RA (Biolegend, 304127) and FITC-CCR7 (Biolegend, 353206) or an antibody combination of PerCP-Cy5.5-CD3 (BD, 560835), BUV805-CD8 (BD, 749366), BUV737-CD28 (BD, 564438) and PE-CD27 (BD, 557330) was added. The cells were stained at 4 °C for 30-45 min, washed twice, then resuspended in an FACS buffer, and detected using a flow cytometer.

FIG. 4A shows the expression of CAR in subpopulations of CD3⁺ T cells, CD8⁻ (CD4⁺) T cells and CD8⁺ T cells after transduction of T cells with each of the 7 types of CAR constructed in Example 1. The results indicate that the positive rate of CAR expression in these transduced T cells was about 15% to 60%.

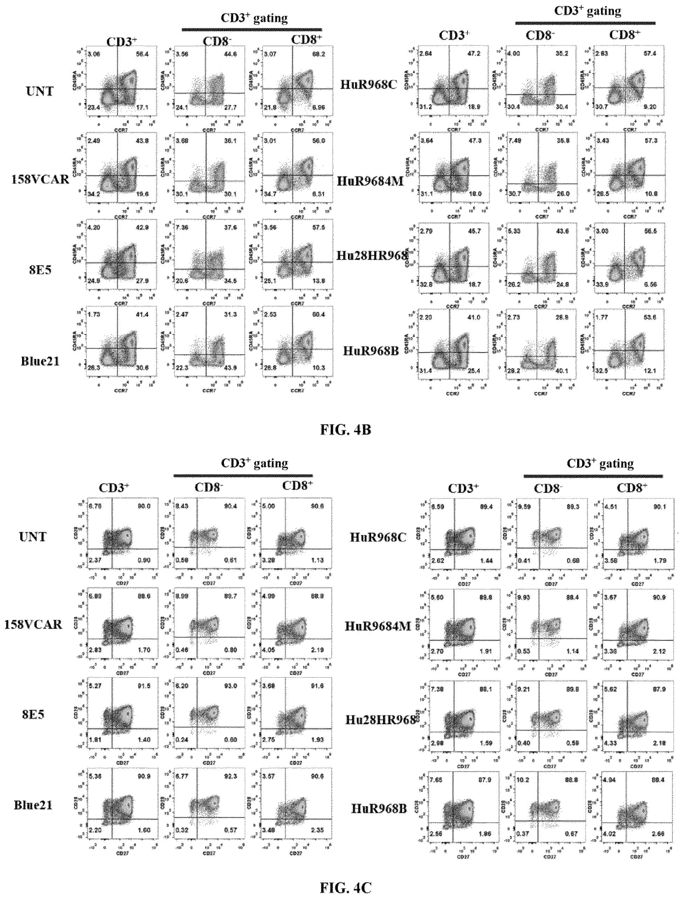

FIG. 4B shows the expression of CD45RA and CCR7 on CAR-T cells after transduction of T cells with each of the 7 types of CAR constructed in Example 1. The results indicate that the CAR-T cells were composed of subpopulations of CD45RA⁺CCR7⁺ naive or stem cell-like memory T cells (T_{N}/T_{SCM}), CD45RA⁻CCR7⁺ central memory T cells (T_{CM}), CD45RA-CCR7- effector memory T cells (T_{EM}) and CD45RA+CCR7- effector T cells (T_{E}), and the majority of the CAR-T cells were T_{N}/T_{SCM} and T_{CM} cells.

FIG. 4C shows the expression of CD27 and CD28 on CAR-T cells after transduction of T cells with each of the 7 types of CAR constructed in Example 1. The results indicate that the majority of the CAR-T cells were CD27⁺CD28⁺ cells.

### Example 3. Detection of CLDN18.2 Expression in Tumor Cells

An appropriate amount of tumor cells that were in the logarithmic growth phase and harvested after digestion were taken, including gastric cancer cell lines AGS, SNU601, SNU620 and NUGC4, and a human pancreatic cancer cell line DAN-G18.2 stably transfected to express CLDN18.2 (constructed by the New Drug Department of Innovent Bio, with the specific construction method being the same as that recorded in Patent Application No. PCT/CN2021/100870 for the construction of the DAN-G-CLDN18.2 tumor cell line). The cells were washed twice with an FACS buffer, and then an HB37A6 antibody (the antibody is an anti-CLDN18.2 antibody, the amino acid sequence of which is set forth in SEQ ID NO: 41, also see Patent Application No. PCT/CN2021/100870) was added. The CLDN18.2 antibody was not added to the cells used as staining controls. The cells were stained at 4 °C for 30-45 min and washed twice, and then the APC-F(ab')₂ Fragment Goat Anti-Human IgG antibody was added. The cells were then stained at 4 °C for another 30-45 min, washed twice, then resuspended in an FACS buffer, and detected using a flow cytometer.

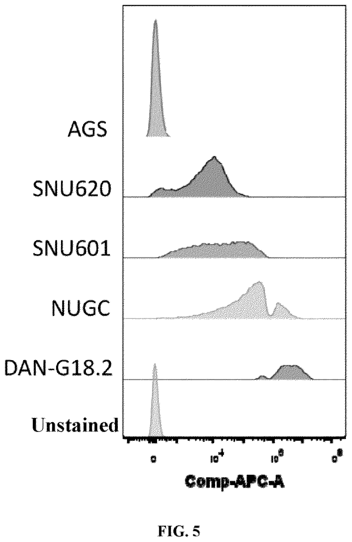

FIG. 5 is a representative flow cytogram showing the expression of the CLDN18.2 molecule in the cells from the gastric cancer cell lines AGS, SNU601, SNU620 and NUGC4 and in the cells from the human pancreatic cancer cell line DAN-G18.2 stably transfected to express CLDN18.2. As can be seen from FIG. 5, DAN-G18.2 cells had the highest level of CLDN18.2 expression, NUGC4 expressed CLDN18.2 at a moderate level, SNU601 and SNU620 expressed CLDN18.2 at a low level, and AGS cells did not express CLDN18.2. Table 1 shows the mean fluorescence intensity (MFI) values for each cell control and CLDN18.2 antibody staining, as well as the MFI ratio of positive staining to control.

**Table 1**

| **Mean fluorescence intensity (MFI)** | | | **MFI ratio of experimental group/control group** |
|---|---|---|---|
| Cell | Control group: without addition of CLDN18.2 antibody | Experimental group: with addition of CLDN18.2 antibody (1:200) | Fold change |
| AGS | 182 | 128 | 0.703297 |
| SNU-620 | 127 | 6013 | 47.34646 |
| SNU-601 | 293 | 16821 | 57.40956 |
| NUGC4 | 306 | 125979 | 411.6961 |
| DAN-G18.2 | 96 | 2770000 | 28854.17 |

### Example 4. Synthesis and Functional Activity Assay of CLDN18.2-Specific WT Antibody and P329G Mutated Antibody

### (4-1) Synthesis of CLDN18.2-specific antibody

The light and heavy chain variable regions (SEQ ID NO: 34, SEQ ID NO: 35) of the CLDN18.2 antibody clone HB37A6 (also referred to herein simply as A6 antibody) and the light and heavy chain variable region sequences (SEQ ID NO: 36, SEQ ID NO: 37) of the clone Hz69H9 (also referred to herein simply as H9) were obtained from a patent (Patent Application No. PCT/CN2021/100870). The light and heavy chain variable region sequences of the clone 175D10 were obtained from Patent No. US10813996B2, serving as a positive control antibody (Zolbetuximab, Zmab). The nucleotide sequences of the antibody light and heavy chain variable regions were subjected to whole-gene synthesis and loaded into pcDNA3.4 expression vectors (purchased from Shanghai Bio-Innovation) containing a WT human IgG1 heavy chain constant region sequence (SEQ ID NO: 38) or a P329G point-mutated human IgG1 heavy chain constant region sequence (SEQ ID NO: 39) and a κ light chain constant region sequence (SEQ ID NO: 40), respectively. The light and heavy chain expression vectors were co-transfected into HEK293 cells by PEI at a molar ratio of 2:3. The cells were cultured for 5-7 days, and then the supernatant of the culture medium was collected. The antibody-containing supernatant medium was purified in one step through a Protein A column, followed by dialysis with PBS. The concentration was detected using a NanoDrop instrument by reading the absorbance at 280 nm, and the purity of the sample was detected using SDS-PAGE and SEC-HPLC methods.

### (4-2) Assay for specificity binding activity of P329G A6 antibody to CLDN18.2 as antigen:

The P329G A6 antibody, P329G H9 antibody and P329G Zmab antibody were prepared into 5-fold gradient diluted antibody solutions at different concentrations with an FACS buffer, which were then separately incubated with 1E5 target cells expressing the CLDN18.2 molecule as the antigen at 4 °C for 30 min, washed with an FACS buffer, and further incubated with APC-goat anti-human IgG, Fcy fragment specific (Jackson ImmunoResearch, 109-136-098) at 4 °C for another 30 min. The P329G antibodies bound to the cells were detected by flow cytometry, and the APC channel MFI was analyzed. Plotting was performed with the antibody concentration as the X-axis and the APC channel MFI as the Y-axis, and the EC₅₀ for binding was calculated.

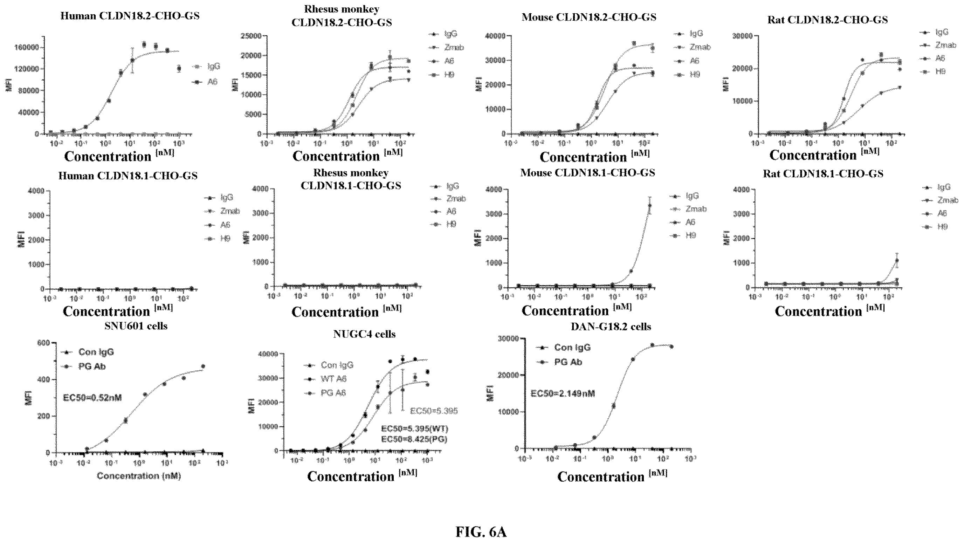

FIG. 6A shows the binding abilities of the A6, H9 and Zmab antibodies at different concentrations to CHO-GS cells stably expressing human, rhesus monkey, mouse and rat CLDN18.1 and CLDN18.2 (constructed by the New Drug Department of Innovent Bio, with the specific construction methods being the same as those recorded in Patent Application No. PCT/CN2021/100870 for the construction of human CLDN18.1- and CLDN18.2-overexpressing CHO-S cell strains: CHO-hCLDN18.1 and CHO-hCLDN18.2). The results indicate that the A6, H9 and Zmab antibodies were all capable of binding to CLDN18.2 from different species; the H9 and Zmab antibodies exhibited high specificity for binding to CLDN18.2 and did not recognize human, monkey and murine CLDN18.1, whereas the A6 antibody showed weak binding to mouse and rat CLDN18.1 only at a high concentration (200 nM). FIG. 6A also shows the binding of the WT and P329G mutated A6 antibodies at different concentrations to CLDN18.2 expression-positive tumor cells SUN601, NUGC4, and DAN-G18.2. The results indicate that the P329G A6 antibody was capable of binding to CLDN18.2 expression-positive tumor cells in a concentration-dependent manner.

Table 2 below summarizes the EC₅₀ and EC₉₀ values for the binding of the A6, H9 and Zmab antibodies as 3 different clonal antibodies to CLDN18.2 and CLDN18.1 from different species.

**Table 2**

| Isoform | Source species | A6 | | Zmab | | H9 | |
|---|---|---|---|---|---|---|---|
| | | EC₅₀ | EC₉₀ | EC₅₀ | EC₉₀ | EC₅₀ | EC₉₀ |
| CLDN18.2 | Human | 1.755 | 9.615 | 14.37 | 112.6 | 6.854 | 30.46 |
| | Rhesus monkey | 1.191 | 5.337 | 2.663 | 15.77 | 2.264 | 10.81 |
| | Mouse | 1.751 | 5.821 | 4.114 | 25.39 | 3.239 | 17 |
| | Rat | 1.635 | 4.511 | 5.928 | 65.91 | 2.970 | 14.16 |
| CLDN18.1 | Human | N/A | N/A | N/A | N/A | N/A | N/A |
| | Rhesus monkey | N/A | N/A | N/A | N/A | N/A | N/A |
| | Mouse | ++ | ++ | N/A | N/A | N/A | N/A |
| | Rat | + | + | N/A | N/A | N/A | N/A |

### (4-3) Assay for binding activity of P329G A6 antibody to P329G CAR:

The P329G CAR-T cells prepared in Example 2 were thawed, resuspended in an RPMI 1640 medium containing 10% FBS, and stabilized in culture at 37 °C for 24 h. The P329G A6 antibody and the wild-type antibody were each prepared into 5-fold gradient diluted antibody solutions at different concentrations with an FACS buffer, which were then separately incubated with 1E5 P329G CAR positive cells at 4 °C for 30 min, washed with an FACS buffer, and then incubated with APC-goat anti-human IgG, Fcy fragment specific (Jackson ImmunoResearch, 109-136-098) at 4 °C for another 30 min. The antibodies bound to the P329G CAR-T cells were detected by flow cytometry, and the APC channel MFI was analyzed. Plotting was performed with the antibody concentration as the X-axis and the APC channel MFI as the Y-axis, and the EC₅₀ for binding was calculated.

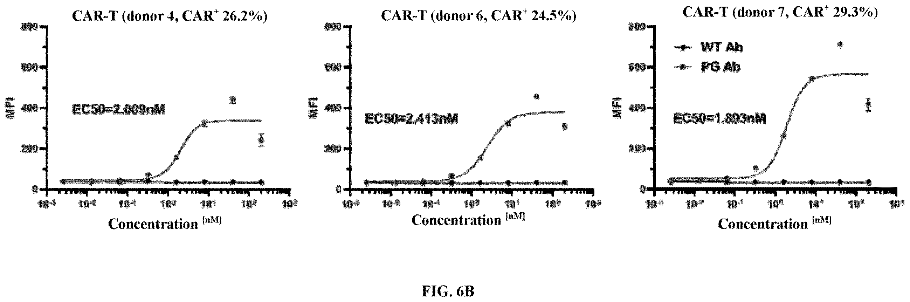

FIG. 6B shows the binding of the WT and P329G mutated A6 antibodies to P329G CAR-T cells. The results indicate that only the P329G mutated antibody (also sometimes referred to herein simply as "PG antibody" or "PG Ab") showed binding to P329G CAR, while the WT antibody (WT Ab) did not bind.

### (4-4) Functional assay for ADCC effect of PBMCs mediated by wild-type/PG A6 antibody

Peripheral blood mononuclear cells (PBMCs) from different donors (see Table 3 for donor PBMC information) were thawed, resuspended in an RPMI 1640 medium, and stabilized at 37 °C for 1-2 h. The PBMCs and target cells (DAN-G18.2 tumor cells) were mixed at an effector cell-to-target cell ratio of 20:1 and then mixed with WT and P329G mutated A6 antibodies at different concentrations. The mixture was further incubated at 37 °C for 24 h. The antibody-mediated killing effect of the PBMCs on the target cells was detected using an LDH assay kit (Promega, G1780). Plotting and analysis were performed with the antibody concentration as the X-axis and the proportion of lysed cells as the Y-axis.

**Table 3**

| **Donor ID** | **LOT No.** | **Cat. No.** | **Age** | **Sex** | **Blood type** | **Virus test** | **CD3+** | **CD4+** | **CD8+** | **CD14+** | **CD19+** | **CD56+** |
|---|---|---|---|---|---|---|---|---|---|---|---|---|
| Donor 3 | PCH2020110004 | PB004F-C | 39 | Female | A | Negative | 63% | 35% | 25% | 15% | 10% | 7% |
| Donor 4 | PCH20201200002 | FPB005F-C | 20 | Male | A | Negative | 58 | 34 | 17 | 24 | 5 | 8 |
| Donor 6 | PCH20201200043 | FPB004F-C | 26 | Male | A | Negative | 58 | 35 | 19 | 17 | 13 | 7 |
| Donor 7 | PCH20210100012 | FPB004F-C | 25 | Male | AB | Negative | 55 | 27 | 21 | 24 | 7 | 10 |

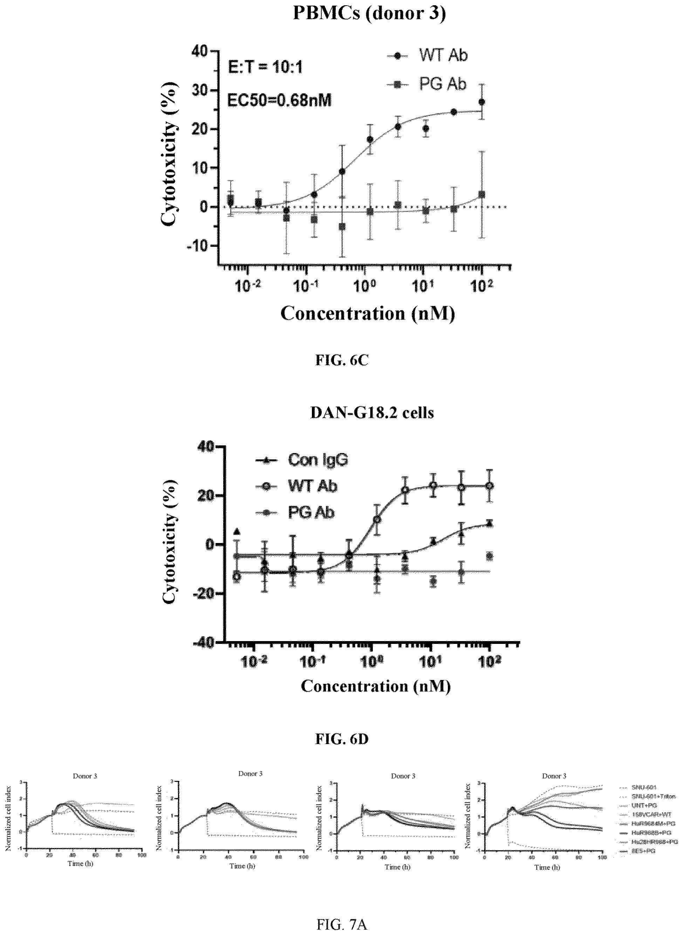

FIG. 6C shows whether the WT and P329G mutated A6 antibodies mediated the PBMCs to exert a cell killing function through ADCC. Donor 3-derived PBMCs were tested. The results indicate that only the WT antibody mediated the ADCC killing effect of the PBMCs on the CLDN18.2 expression-positive DAN-G18.2 tumor cells, whereas the P329G mutated antibody lacked the ability to induce the PBMCs to exert the ADCC effect on the CLDN18.2 expression-positive DAN-G18.2 tumor cells.

### (4-5) Functional assay for CDC effect mediated by P329G A6 antibody

P329G A6 and wild-type A6 antibodies at different concentrations were separately incubated with target cells (DAN-G18.2 tumor cells) at 37 °C for 30 min. 10 µL of a human serum complement (Sigma, S1764-1ML) was then added, and the mixture was further incubated for 3 h. The proportion of viable cells was detected using CellTiter-Glo (Promega, G9242). Plotting was performed with the antibody concentration as the X-axis and the proportion of viable cells as the Y-axis, and the CDC effect mediated by the antibodies was analyzed.

FIG. 6D shows whether the WT and P329G mutated A6 antibodies mediated the complement to exert a cell killing function through CDC. The results indicate that only the WT antibody mediated the CDC killing effect on the CLDN18.2 expression-positive DAN-G18.2 tumor cells, whereas the P329G mutated antibody lacked the ability to induce the CDC effect.

### Example 5. Screening of P329G CAR Candidate Molecules

### (5-1) In-vitro repeated dynamic killing experiments on CLDN18.2-expressing tumor target cells:

The continuous killing of CLDN18.2-expressing target cells by the CAR-T cells prepared in Example 2 was dynamically detected in real time using an xCELLigence RTCA MP instrument (Agilent). In a first 96-well E-Plate (Agilent, 3006000910), 50 µL of culture medium was added. After the instrument read the baseline value, 50 µL of tumor target cells (30000 cells) were added, and the plate was placed in the instrument for dynamic detection. At the same time, UNT cells and the CAR-T cells prepared in Example 2 were thawed and incubated in an incubator at 37 °C overnight. After about 24 h, the positive rates of all CAR-T cells were adjusted to be consistent using the UNT cells. Different CAR-T cells prepared from PBMCs from donor 3 were added at an E:T ratio of 1:2, and a WT or P329G mutated A6 antibody was added to the corresponding wells. The killing of the target cells by the P329G CAR-T cells was dynamically monitored by the xCELLigence RTCA MP instrument system for 72-96 h. After the first round of killing experiment in the first E-Plate was completed, all the suspended cells (mainly containing CAR-T cells) were transferred to a 96-well V-bottom plate, centrifuged to remove the supernatant, resuspended in a fresh culture medium, and then added to a second E-Plate containing target tumor cells. The antibody was then added to the corresponding wells again. The system was continuously monitored for 72-96 h, and the killing experiment was repeated for 2-4 rounds.

FIG. 7A shows the experimental results of repeated killing of SNU601 cells by HuR968B, HuR9684M and Hu28HR968 CAR-T cells prepared from donor 3 (PCH20201100004:allcells, cat: PB004F-C, 39-year-old female) at an E:T ratio of 1:5. All CAR-T cells maintained their killing activity for 3 rounds. In the 4^{th} round of killing experiment, only the HuR968B CAR-T cells maintained their killing activity, which was superior to that of the conventional 8E5 CAR-T cells, and the CD16 CAR-T cells as a positive control also lost their killing activity in the 4^{th} round of killing experiment.

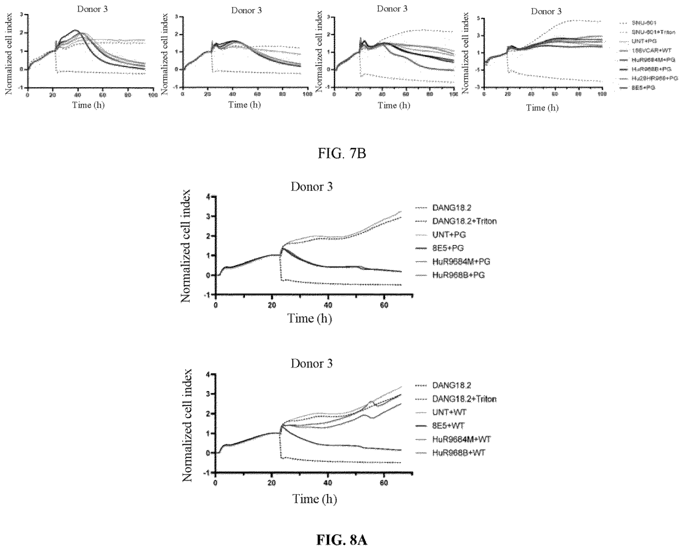

FIG. 7B shows the experimental results of repeated killing of SNU601 cells by HuR968B, HuR9684M and Hu28HR968 CAR-T cells prepared from donor 3 (PCH20201100004:allcells, cat: PB004F-C, 39-year-old female) at an E:T ratio of 1:10. 3 different CAR-T cells maintained their killing activity for 2 rounds. In the 3^{rd} round of killing experiment, only the HuR9684M and HuR968B CAR-T cells maintained their killing activity; in the 4^{th} round of killing experiment, all the CAR-T cells substantially lost their killing activity. The conventional 8E5 CAR-T cells as the positive control maintained their killing activity for 3 rounds, but lost their activity in the 4^{th} round of killing experiment, whereas the CD16 CAR-T cells only maintained their killing activity for 2 rounds.

### Example 6. In-Vitro Functional Study of HuR968B and HuR9684M CAR-T Cells

### (6-1) Dynamic killing experiment on CLDN18.2-expressing tumor target cells:

The killing of target cells by CAR-T cells was dynamically detected in real time using an xCELLigence RTCA MP instrument. In an E-Plate, 50 µL of culture medium was added. After the instrument read the baseline value, 50 µL of tumor target cells (30000 cells) were added, and the plate was placed in the instrument for dynamic detection. At the same time, UNT and CAR-T cells were thawed and incubated in an incubator at 37 °C overnight. After about 24 h, the positive rates of all CAR-T cells were adjusted to be consistent using the UNT cells. Different CAR-T cells prepared from PBMCs from the same donor were added at an E:T ratio of 1:2, and a WT or P329G mutated A6 antibody was added to the corresponding wells. The killing of the target cells by the P329G CAR-T cells was dynamically monitored by the xCELLigence RTCA MP instrument system for 72-96 h.

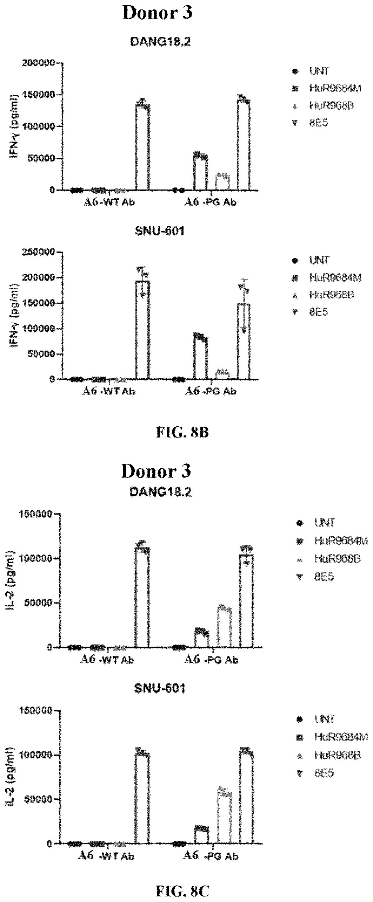

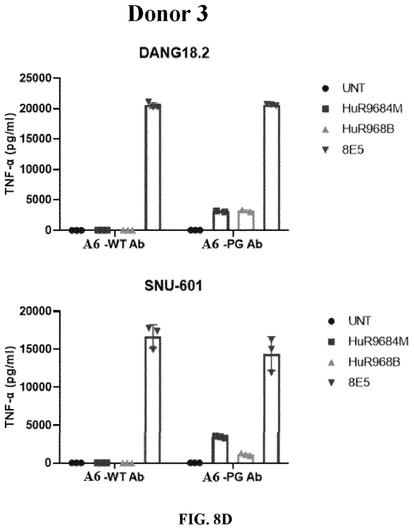

FIG. 8A shows the killing effects of HuR968B and HuR9684M CAR-T cells prepared from donor 3 (PCH20201100004:allcells, cat: PB004F-C, 39-year-old female) on DAN-G18.2 tumor cells. At an E:T ratio of 1:2, the addition of 100 ng/mL P329G A6 antibody induced the complete killing effect of the HuR968B and HuR9684M CAR-T cells on the DAN-G18.2 cells, while the addition of WT A6 antibody failed to induce the killing activity of the aforementioned CAR-T cells; the conventional 8E5 CAR-T cells as the positive control were capable of producing a killing effect upon addition of either P329G or WT A6 antibody, whereas the untransduced UNT cells as the negative control did not produce a killing effect.

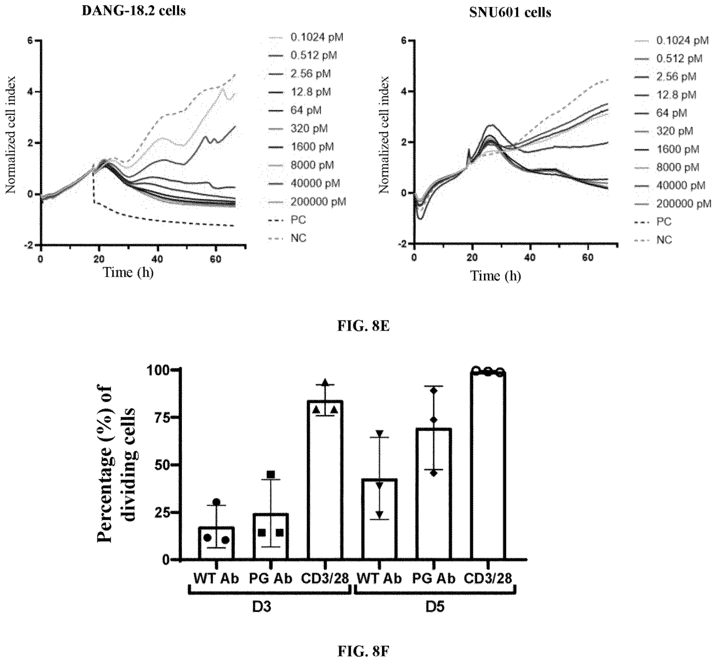

FIG. 8E shows the killing effects of HuR968B CAR-T cells on DAN-G18.2 tumor cells at different concentrations of P329G A6 antibody. In the DAN-G18.2 tumor cells with high CLDN18.2 expression, as low as 12.8 pM of P329G A6 antibody was capable of inducing a complete killing effect of the HuR968B CAR-T cells on the target cells, whereas in the SNU601 tumor cells with low CLDN18.2 expression, 64 pM of P329G A6 antibody was capable of inducing a complete killing effect of the HuR968B CAR-T cells on the target cells.

### (6-2) Cytokine detection:

Cytokines were detected using the BD^{™} Cytometric Bead Array (CBA) Human Th1/Th2 Cytokine Kit II. Equal volumes of Capture Beads in the kit were mixed well and then plated at 25 µL/well. An equal volume of the supernatant from the killing experiment or a supernatant dilution or a standard was added. The mixture was mixed well, and then an equal volume (25 µL) of Human Th1/Th2 PE Detection Reagent was added, followed by incubation at room temperature in the dark for 3 h. The cells were washed twice with a Wash buffer, then resuspended, and detected using a flow cytometer, and the cytokine concentrations were calculated based on PE channel MFI values.

FIGs. 8B-8D show the results of the release of effector cytokines from HuR968B and HuR9684M CAR-T cells co-cultured with tumor cells from donor 3 (PCH20201100004:allcells, cat: PB004F-C, 39-year-old female), respectively. With the addition of WT, the HuR968B and HuR9684M CAR-T cells were not activated and did not secrete effector cytokines such as IL-2, IFN-γ, and TNF-α. Only with the addition of the P329G A6 antibody, the P329G CAR-T cells were activated and secreted the effector cytokines. HuR9684M secreted higher levels of cytokines compared to HuR968. The conventional 8E5 cells as a positive control secreted high levels of cytokines with the addition of either WT or P329G A6 antibody, with the levels slightly higher than those of HuR9684M CAR-T cells.

### (6-3) CAR-T cell proliferation experiment:

CFSE staining method: CAR-T cells were thawed, stabilized in culture at 37 °C overnight, and then labeled with the CellTrace CFSE Cell Proliferation Kit (Invitrogen, C34554). A WT or P329G A6 antibody was diluted with PBS, and a 96-well flat-bottom plate was coated with the diluted antibody and then incubated at 4 °C overnight. The labeled cells were separately added to the well plate coated with the antibody; as a positive control, CD3/CD28-coupled magnetic beads (Gibco, 11132D) were added directly to the CAR-T cells (at a magnetic bead-to-cell ratio of 1:1); the cells were cultured at 37 °C for 72-120 h, collected, washed, and resuspended, and then an FACS buffer containing LIVE/DEAD Fixable Dead Cell Stain was added; the cells were then stained at room temperature for 10-15 min and washed twice, and the dilution of CFSE fluorescence in the CAR-T cells was analyzed by flow cytometry. CellTiter method: The CAR-T cells were thawed and stabilized in culture at 37 °C overnight. A WT or P329G A6 antibody was diluted with PBS, and a well plate was coated with the diluted antibody and then incubated at 4 °C overnight. The overnight-thawed CAR-T cells were added to the well plate coated with the antibody, and as a positive control, CD3/CD28-coupled magnetic beads were directly added to the CAR-T cells (at a magnetic bead-to-cell ratio of 3:1); the cells were cultured at 37 °C for 48-72 h, and the cell luminescence values were detected using CellTiter-Glo^{®} Luminescent Cell Viability Assay (Promega, G7572).

FIG. 8F shows the proliferation of HuR9684M CAR-T cells from donor 3 (PCH20201100004:allcells, cat: PB004F-C, 39-year-old female) after stimulation by the plate-bound WT or P329G A6 antibody. The CFSE-labeled HuR9684M CAR-T cells proliferated under stimulation by the plate-bound P329G A6 antibody, with the proliferation being particularly noticeable after 5 days of stimulation (an average of 69.5% of cells underwent proliferation). Although proliferation also occurred under stimulation of the WT A6 antibody (an average of 42.8% of cells underwent proliferation), it was significantly lower than the proliferation effect induced by the P329G antibody stimulation. CD3/CD28 antibody-coupled magnetic beads as a positive control significantly stimulated the proliferation of the CAR-T cells.

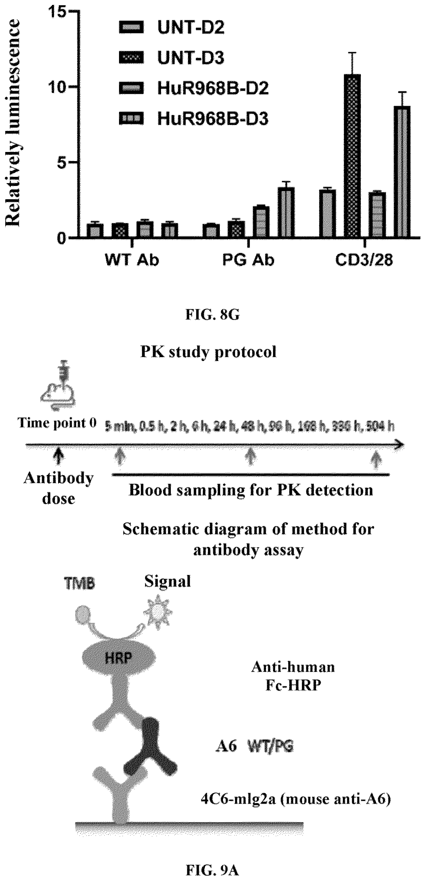

FIG. 8G shows the proliferation of HuR968B CAR-T cells from donor 3 (PCH20201100004:allcells, cat: PB004F-C, 39-year-old female) after stimulation by the plate-bound WT or P329G A6 antibody. The HuR968B CAR-T cells proliferated under stimulation by the plate-bound P329G A6 antibody. Compared to stimulation with the WT antibody, stimulation with the P329G A6 antibody for 2 or 3 days resulted in approximately 2-fold or 3.3-fold proliferation of the HuR968B CAR-T cells, respectively; however, the UNT cells did not show significant proliferation under stimulation with the P329G antibody, compared to stimulation with the WT antibody; both HuR968B CAR-T cells and UNT cells showed significant proliferation under stimulation with the CD3/CD28 antibody-coupled magnetic beads.

### Example 7. In-Vivo Pharmacokinetic Study of P329G A6 Antibody

### (7-1) Antibody injection and sampling

BALB/c mice (aged 4-6 weeks, weighing 15-17 g, female) were divided into 2 groups of 9, namely a P329G A6 antibody (PG Ab) group and a WT A6 antibody (WT Ab) group. Each antibody was diluted with 1× PBS to 0.1 mg/mL and administered in a volume of 10 mL/kg per mouse, i.e., the antibody was administered at a dose of 1 mg/kg; the mode of administration was intravenous injection, and the frequency of administration was a single dose. Blood samples of 100 µL were collected from the retroorbital venous plexus of the mice at 5 min, 30 min, 2 h, 6 h, 24 h, 48 h, 96 h, 168 h, 336 h, and 504 h after the antibody administration and then centrifuged at 3000 g. The supernatant was collected for the determination of plasma concentration.

### (7-2) A6 antibody assay

A 96-well microplate was coated with the antibody one day in advance. 4C6-mIgG2a (synthesized by Biointron) was diluted to 4 µg/mL with the coating solution and added to the plate at 100 µL/well. The plate was then sealed with a sealing film and left at room temperature overnight. The coating solution was poured out, and the plate was patted dry on absorbent paper. 300 µL of a washing solution was added to each well, and the mixture was mixed well by shaking for 10 s. The plate was patted dry to remove the washing solution and then washed 3 times. A blocking solution was added at 200 µL/well using a multichannel pipettor, and the plate was sealed with a sealing film, incubated at room temperature for 2 h, and then washed once. The diluted standard curve sample (prepared by gradient dilution of A6-PG antibody with known concentration to create the standard curve), quality control samples (A6-PG and WT-PG, synthesized by Biointron), and test samples were each added at 100 µL/well, and the plate was incubated at room temperature for 2 h. The pre-coating solution was poured out, and the plate was patted dry on absorbent paper. 300 µL of a washing solution was added to each well, and the mixture was mixed well by shaking for 10 s. The plate was patted dry to remove the washing solution and then washed 3 times. The procedure was repeated once. Goat anti-human IgG-Fc-HRP (BETHYL) was diluted to 20 ng/mL and added at 100 µL/well, and the plate was incubated at room temperature for 1 h and then washed once. A TMB substrate was added to the 96-well microplate at 100 µL/well, and a chromogenic reaction was conducted at room temperature in the dark for 5-15 min. An ELISA stop solution was added at 100 µL/well, and the plate was shaken for 10 s. OD values at 450 nm and 620 nm were then read.

FIG. 9A shows sampling time points for the A6 antibody pharmacokinetic experiment in mice and a schematic diagram of the A6 antibody ELISA in mice.

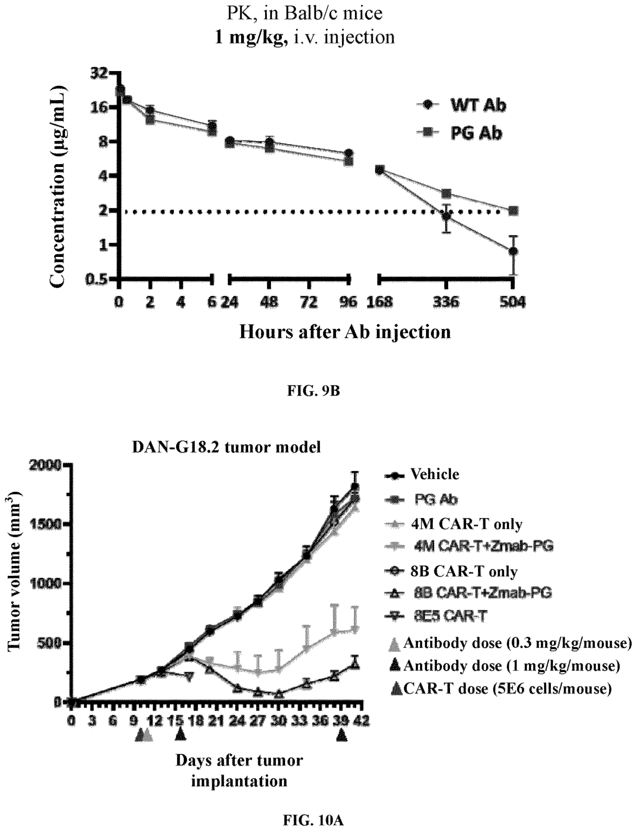

FIG. 9B shows the results of the A6 antibody pharmacokinetic experiment in mice. After intravenous injection of 1 mg/kg P329G or WT A6 antibody, the exposure (Cₘₐₓ and AUCₗₐₛₜ) of the P329G and WT antibodies in serum was similar, with no difference in the main pharmacokinetic parameters. As shown in Table 4, the AUC_{0-inf}, Cₘₐₓ, CL, and T_{1/2} of the P329G A6 antibody were 2875 µg·h/mL, 21.5 µg/mL, 0.35 mL/kg/h, and 279 h, respectively; the AUC_{0-inf}, Cₘₐₓ, CL, and T_{1/2} of the WT A6 antibody were 2090 µg·h/mL, 23.1 µg/mL, 0.48 mL/kg/h, and 141 h, respectively; the half-life of the P329G A6 antibody was slightly longer than that of the WT antibody.

**Table 4**

| **Group** | **Cmax (µg/mL)** | **AUC₀₋ₜ (µg*h/mL)** | **AUC_{0-∞} (µg*h/mL)** | **MRT_{0-∞} (h)** | **T_{1/2} (h)** | **CL (ml/kg/h)** | **Vss (ml/kg)** |
|---|---|---|---|---|---|---|---|
| **WT Ab** | 23.1 | 1914 | 2090 | 193 | **141** | 0.48 | 92.45 |
| **PG Ab** | 21.5 | 2080 | 2875 | 383 | **279** | 0.35 | 133.31 |

### Example 8. Study on In-Vivo Anti-Tumor Effects of HuR968B and HuR9684M CAR-T Cells in Combination with Zmab Antibody

### (8-1) Mouse tumor inoculation and treatment, and anti-tumor effect

DAN-G18.2 cells were resuspended in 1× PBS to prepare a cell suspension at a concentration of 5 × 10⁶ cells/mL. NOG mice (aged 4-6 weeks, weighing 15-17 g, female) were subjected to shaving at the right back and injected subcutaneously with the DAN-G18.2 cell suspension at 5 × 10⁶ cells/mL in a volume of 0.2 mL/mouse, i.e., at an inoculum size of 1 × 10⁶ cells/mouse. 10 days after the tumor cell inoculation, mice with tumor volumes of 135.98-243.23 mm³ were divided into 7 groups of 5, namely a vehicle group (i.e., a group given PBS), a PG Ab group (i.e., a group given a P329G Zmab antibody), a 4M CAR-T-only group (i.e., a group given only HuR9684M CAR-T, with HuR9684M hereinafter also abbreviated as "4M"), a 4M CAR-T + Zmab-PG group, an 8B CAR-T-only group (i.e., a group given only HuR968B CAR-T, with HuR968B hereinafter also abbreviated as "8B"), an 8B CAR-T + Zmab-PG group, and an 8E5 CAR-T group. The CAR-T cells were resuspended in 1× PBS to prepare a CAR⁺ cell suspension at 2.5 × 10⁷ cells/mL. The cell suspension was injected into the mice via the tail vein at 0.2 mL/mouse, corresponding to the reinfusion of 5 × 10⁶ CAR⁺ cells/mouse. On day 1 after the reinfusion of CAR-T cells, the 1^{st} antibody administration (0.3 mg/kg) was performed; after 1 week, the 2^{nd} antibody administration (1 mg/kg) was performed; 3 weeks after the 2^{nd} antibody administration, the 3^{rd} antibody administration (1 mg/kg/mouse) was performed; all the administrations were performed by intraperitoneal injection. The mice were monitored twice a week for the body weight and the maximum length of major axis (L) and maximum length of minor axis (W) of tumor tissues.

FIG. 10A shows the therapeutic effects of the HuR968B and HuR9684M CAR-T cells in the immunodeficient tumor-bearing mice inoculated with the human DAN-G18.2 tumor cells. The results show that treatment with HuR968B or HuR9684M CAR-T cells alone or P329G Zmab antibody alone did not produce an anti-tumor effect, and only mice treated with both CAR-T cells and P329G Zmab antibody produced a significant anti-tumor effect; tumor growth began to be inhibited approximately 1 week after the administration of the P329G Zmab antibody, and after approximately 2 more weeks, the tumor inhibition reached its highest point; at this time, the tumor growth inhibition rates (TGI) for the groups treated with the antibody along with the HuR968B and HuR9684M CAR-T cells were 93% and 74%, respectively, while the mice in the groups treated with CAR-T cells alone and antibody alone showed substantially no anti-tumor effect, with TGI values of 1.3%, 7.0%, and 4.9%, respectively. In addition, the conventional 8E5 CAR-T cells as a positive control caused severe toxicity at the same dose, resulting in the death of the mice within a week after the CAR-T cell administration.

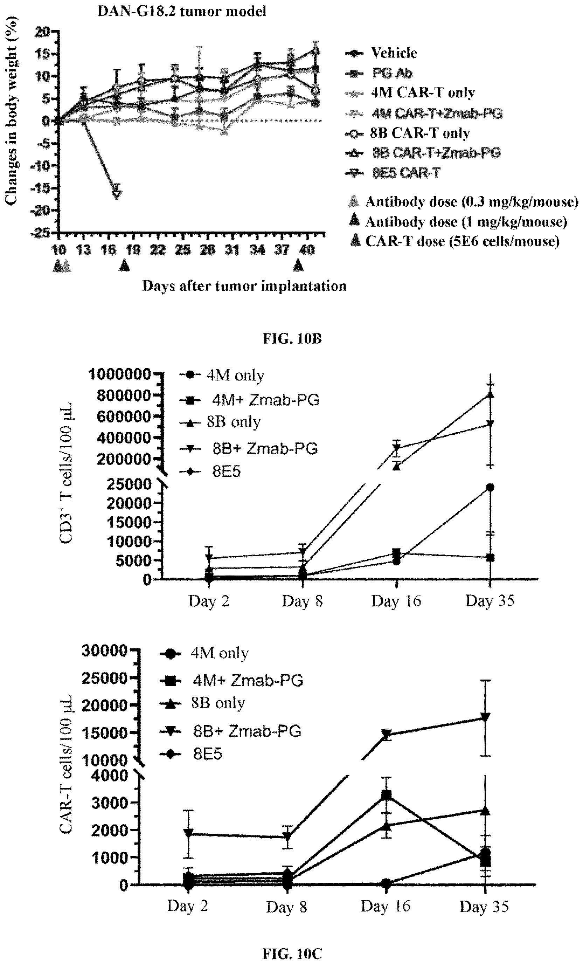

FIG. 10B shows the changes in body weight of mice during this experiment. The results show that the changes in body weight of mice treated with the HuR968B or HuR9684M CAR-T cells in combination with the P329G Zmab antibody were similar to those of untreated mice (i.e., the vehicle control treatment group), indicating that the combination therapy did not induce significant toxicity. In contrast, mice treated with the conventional 8E5 CAR-T cells experienced a rapid decrease in body weight within 1 week after CAR-T cell infusion, with an average decrease of 16.6%, and all mice reached the endpoint of sacrifice, indicating that the treatment with conventional 8E5 CAR-T cells induced severe toxicity in the mice.

### (8-2) In-vivo CAR-T cell assay

A mouse blood sample was taken and added to a 96-well V-bottom plate at 30 µL/well, labeled as sample detection wells; a mouse blood sample was taken and added to a 96-well V-bottom plate at 10 µL/well, labeled as control wells. 100 µL of an FACS buffer containing LIVE/DEAD Fixable Dead Cell Stain and TruStain FcX^{™} (anti-mouse CD16/32) (Biolegend) was added to each well, and the mixture was gently mixed well and then incubated at 4 °C in the dark for 15 min. Subsequently, a Biotin-F(ab')₂ Fragment Goat Anti-Human IgG antibody was added to the sample detection wells, and the mixture was incubated at 4 °C in the dark for 30 min. Then, 100 µL of FACS buffer was added to each sample detection well, and the mixture was centrifuged at 400 g, followed by removal of the supernatant. 100 µL of an FACS buffer containing APC-Cy7 anti-human CD45 (Biolegend), PerCP-Cy5.5-CD3 (BD Biosciences), and APC-streptavidin (Biolegend) was added to each well, and the mixture was gently mixed well and then incubated at 4 °C in the dark for 30 min. Then, FACS buffer was added to all wells at 200 µL/well, and the mixture was centrifuged at 400 g, followed by removal of the supernatant. Afterward, 1× RBC Lysis/Fixation solution (Biolegend) was added at 250 µL/well, and the mixture was mixed well, then incubated at room temperature in the dark for 20 min, and centrifuged at 400 g, followed by removal of the supernatant. The cells were resuspended in 100 µL of FACS buffer, and then 10 µL of 123count eBeads was added to each well, followed by assay on a flow cytometer.

FIG. 10C shows the *in-vivo* expansion of the HuR968B and HuR9684M CAR-T cells in mice in the experiment of Example 8-1. The results show that the HuR968B CAR-T cells began to expand in the mice 1 week after reinfusion, reached a high level of expansion after 2 weeks (2166 cells/100 µL of peripheral blood), and remained at a high level after 3 weeks (2726 cells/100 µL of peripheral blood); the administration of the HuR968B CAR-T cells along with the P329G antibody greatly promoted the *in-vivo* expansion of the cells, with CAR-T cell counts at 2 weeks and 3 weeks being 14507 and 17617 cells/100 µL of peripheral blood, respectively, which was 6-7 times higher than those without the administration of the antibody. The HuR9684M CAR-T cells also began to expand in the mice 1 week after reinfusion, and reached a peak level of expansion after 2 weeks (51 cells/100 µL of peripheral blood), which was much lower than that of HuR968B cells. The administration of the HuR9684M CAR-T cells along with the P329G antibody also greatly promoted the *in-vivo* expansion of the cells, with the peak level of expansion reaching 3260 cells/100 µL of peripheral blood after 2 weeks, and the CAR-T cell counts began to decline after 3 weeks. The overall T cell expansion kinetics of the two types of CAR-T cells were similar in the presence of antibody administration, reaching and maintaining at a high level after 2 weeks, which was much higher than the total T cell expansion in the mice not treated with the antibody. In the absence of antibody administration, the total T cells in the mice treated with HuR9684M alone were expanded significantly and maintained sustained expansion after 2 weeks, whereas the total T cells in the mice treated with HuR968B alone reached a peak level of expansion after 2 weeks and began to decline after 3 weeks.

### Example 9. Study on In-Vivo Anti-Tumor Effect of Single Administration of HuR968B CAR-T Cells in Combination with A6 Antibody

### (9-1) Mouse tumor inoculation and treatment, and anti-tumor effect

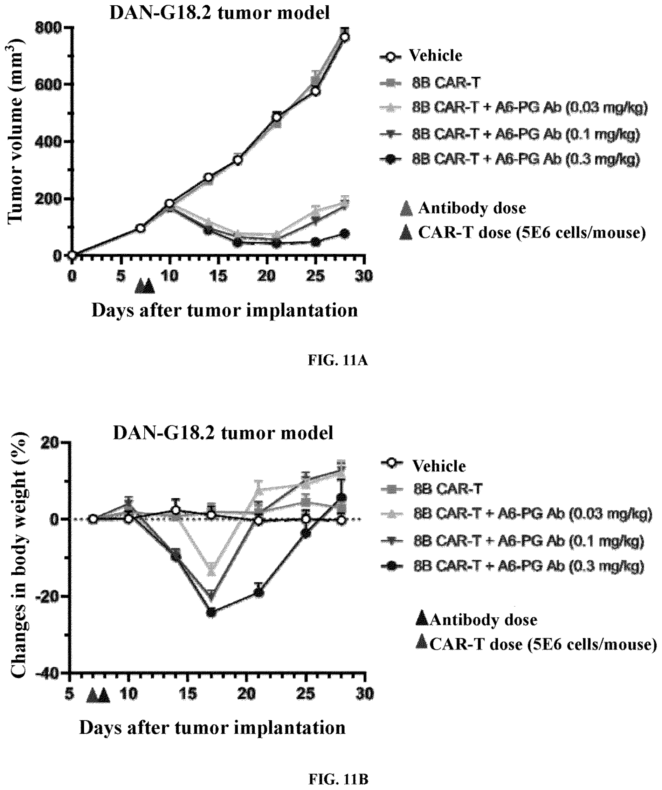

DAN-G18.2 cells were resuspended in 1× PBS to prepare a cell suspension at a concentration of 5 × 10⁶ cells/mL. NOG mice were subjected to shaving at the right back and injected subcutaneously with the DAN-G18.2 cell suspension at 5 × 10⁶ cells/mL in a volume of 0.2 mL/mouse, i.e., at an inoculum size of 1 × 10⁶ cells/mouse. 7 days after the tumor cell inoculation, mice with tumor volumes of 72.13-113.94 mm³ were divided into 5 groups of 7-14, namely a vehicle group, an 8B CAR-T-only group (with HuR968B CAR-T abbreviated as "8B CAR-T"), an 8B CAR-T + A6-PG (0.03 mg/kg) group, an 8B CAR-T + A6-PG (0.1 mg/kg) group, and an 8B CAR-T + A6-PG (0.3 mg/kg) group. The 8B CAR-T cells were resuspended in 1× PBS to prepare an 8B CAR⁺ cell suspension at 2.5 × 10⁷ cells/mL. The cell suspension was injected into the mice via the tail vein at 0.2 mL/mouse, corresponding to the reinfusion of 5 × 10⁶ CAR⁺ cells/mouse. On day 1 after the CAR-T cell reinfusion, i.e., on day 8 after the tumor cell inoculation, the antibody was administered; the frequency of administration was a single dose, and the mode of administration was intraperitoneal injection. The mice were monitored twice a week for the body weight and the maximum length of major axis (L) and maximum length of minor axis (W) of tumor tissues. FIG. 11A shows the therapeutic effects of HuR968B CAR-T cells in combination with different doses of P329G A6 antibody in immunodeficient tumor-bearing mice inoculated with human DAN-G18.2 tumor cells. The results show that in the presence of the concomitant administration of the P329G A6 antibody at a dose as low as 0.03 mg/kg, the HuR968B CAR-T cells induced a significant anti-tumor effect; increasing the dose of the P329G A6 antibody to 0.1 and 0.3 mg/kg did not significantly enhance the maximum anti-tumor effect induced by the HuR968B CAR-T cells, but the anti-tumor effect was maintained for a longer duration. For the 0.03, 0.1 and 0.3 mg/kg treatment groups, the tumor growth inhibition rates (TGI) were 84.6%, 88.7%, and 91.3%, respectively, 2 weeks after treatment, and were 75.9%, 77.6%, and 90.0%, respectively, 3 weeks after treatment. The CAR-T cell-only group showed no anti-tumor effect.

FIG. 11B shows the changes in body weight of mice during this experiment. The results show that the body weight of the mice treated with the HuR968B CAR-T cells and P329G A6 antibody began to decrease after the treatment, and decreased to the lowest point 9 days after the concomitant administration of the P329G A6 antibody, with an average decrease in body weight of 13.5%, 20.6%, and 24.4% in the 0.03, 0.1 and 0.3 mg/kg treatment groups, respectively. Subsequently, the mice began to regain weight. The body weight of the mice in the 0.03 and 0.1 mg/kg treatment groups returned to normal within 4 days, and the body weight of the mice in the 0.3 mg/kg treatment group also returned to normal levels after 1 week. The results indicate that the treatment with the HuR968B CAR-T cells in combination with a low dose of P329G A6 antibody induced significant anti-tumor effects, but also produced transient tolerable toxic side effects.

### (9-2) In-vivo CAR-T cell assay:

The assay method was the same as that in Example 8-2.

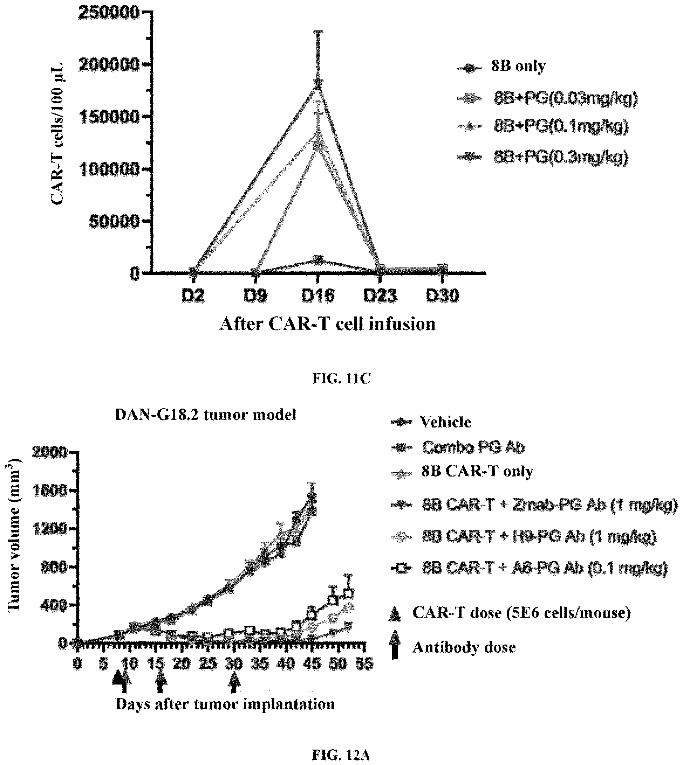

FIG. 11C shows the *in-vivo* expansion of the HuR968B CAR-T cells in mice in the experiment of Example 9-1. The results show that the HuR968B CAR-T cells began to expand in the mice 1 week after reinfusion, reached a peak level of expansion after 2 weeks, then rapidly declined, and returned to the baseline level after 3 weeks. The *in-vivo* CAR-T cell expansion was dependent on the P329G A6 antibody, and the mice in the group without the concomitant antibody administration did not exhibit significant cell expansion; furthermore, the CAR-T cell expansion showed a certain P329G A6 antibody dose-dependence, and the mice in the higher dose group showed higher levels of CAR-T cell expansion. The peak levels of expansion in the 0.03, 0.1 and 0.3 mg/kg dose groups were 121783, 135765 and 180927 cells/100 µL of peripheral blood, respectively, which were much higher than that in the group without the concomitant antibody administration (12160 cells/100 µL of peripheral blood).

### Example 10. Study on In-Vivo Anti-Tumor Effect of Multiple Administrations of HuR968B CAR-T Cells in Combination with A6 Antibody

Mouse tumor inoculation and treatment were performed. DAN-G18.2 cells were resuspended in 1× PBS to prepare a cell suspension at a concentration of 5 × 10⁶ cells/mL. NOG mice were subjected to shaving at the right back and injected subcutaneously with the DAN-G18.2 cell suspension at 5 × 10⁶ cells/mL in a volume of 0.2 mL/mouse, i.e., at an inoculum size of 1 × 10⁶ cells/mouse. 8 days after the tumor cell inoculation, mice with tumor volumes of 65.00-102.85 mm³ were divided into 6 groups of 7, namely a vehicle group, a Comb PG Ab group (combination of Zmab-PG, H9-PG, and A6-PG antibodies), an 8B CAR-T-only group, an 8B CAR-T + Zmab-PG (1 mg/kg) group, an 8B CAR-T + H9-PG (1 mg/kg) group, and an 8B CAR-T + A6-PG (0.1 mg/kg) group. The 8B CAR-T cells were resuspended in 1× PBS to prepare an 8B CAR⁺ cell suspension at 2.5 × 10⁷ cells/mL. The cell suspension was injected into the mice via the tail vein at 0.2 mL/mouse, corresponding to the reinfusion of 5 × 10⁶ CAR⁺ cells/mouse. On day 1 after the CAR-T cell reinfusion, i.e., on day 9 after the tumor cell inoculation, the antibody was administered; the administration was performed at an interval of 1 week initially, followed by an interval of 2 weeks, and the mode of administration was intraperitoneal injection. The mice were monitored twice a week for the body weight and the maximum length of major axis (L) and maximum length of minor axis (W) of tumor tissues.

FIG. 12A shows the therapeutic effects of HuR968B CAR-T cells in combination with multiple doses of P329G antibody in immunodeficient tumor-bearing mice inoculated with human DAN-G18.2 tumor cells. The results indicate that administering multiple doses of the P329G (Zmab, H9, or A6) antibody could induce a more persistent anti-tumor effect. The results also show that after cessation of the P329G antibody administration, the concentration of the P329G antibody in the mice decreased gradually over time, and the effect of the CAR-T cells also began to diminish.

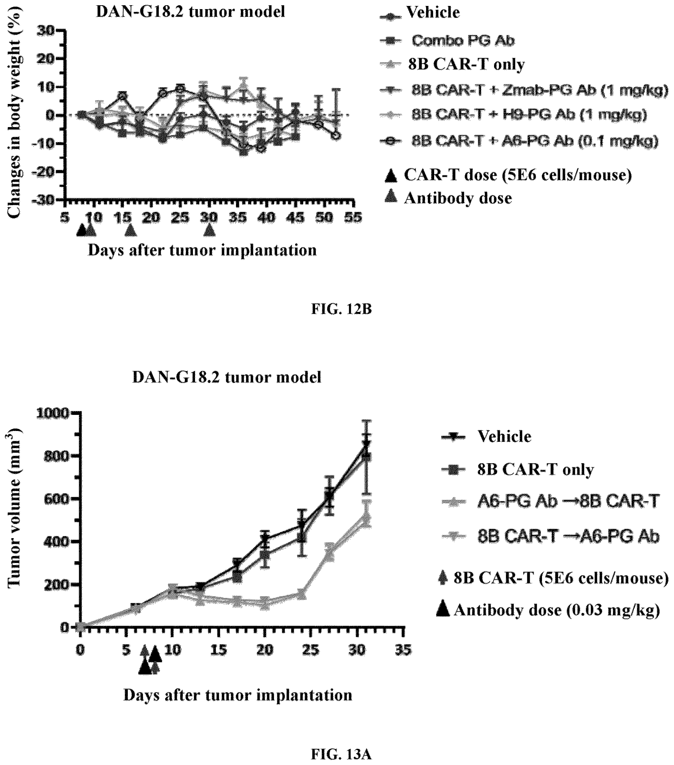

FIG. 12B shows the changes in body weight of mice during this experiment. The results show that although there were fluctuations in the body weight of mice in each treatment group during the treatment period, there was no significant change compared to mice in the control treatment group, suggesting that the treatment with HuR968B CAR-T cells in combination with multiple doses of P329G antibody did not induce significant toxicity.

### Example 11. Study on In-Vivo Anti-Tumor Effects of HuR968B CAR-T Cells in Combination with A6 Antibody with Different Administration Sequences

Mouse tumor inoculation and treatment were performed. DAN-G18.2 cells were resuspended in 1× PBS to prepare a cell suspension at a concentration of 5 × 10⁶ cells/mL. NOG mice were subjected to shaving at the right back and injected subcutaneously with the DAN-G18.2 cell suspension at 5 × 10⁶ cells/mL in a volume of 0.2 mL/mouse, i.e., at an inoculum size of 1 × 10⁶ cells/mouse. 6 days after the tumor cell inoculation, mice with tumor volumes of 62.05-122.04 mm³ were divided into 6 groups of 7, namely a vehicle group, an 8B CAR-T-only group, an 8B CAR-T + A6-PG (0.03 mg/kg) group, an 8B CAR-T + A6-PG (0.1 mg/kg) group, an A6-PG (0.03 mg/kg) + 8B CAR-T group, and an A6-PG (0.1 mg/kg) + 8B CAR-T group. On day 7 after the tumor cell inoculation, the 8B CAR-T cells were resuspended in 1× PBS to prepare an 8B CAR⁺ cell suspension at 2.5 × 10⁷ cells/mL. The cell suspension was injected into the mice in the 8B CAR-T + A6-PG (0.03 mg/kg) group and the 8B CAR-T + A6-PG (0.1 mg/kg) group via the tail vein at 0.2 mL/mouse. The antibody was administered to the mice in the A6-PG (0.03 mg/kg) + 8B CAR-T group and the A6-PG (0.1 mg/kg) + 8B CAR-T group at doses of 0.03 mg/kg and 0.1 mg/kg, respectively, and the mode of administration was intraperitoneal injection. On day 8 after the tumor cell inoculation, the antibody was administered to the mice in the 8B CAR-T + A6-PG (0.03 mg/kg) group and the 8B CAR-T + A6-PG (0.1 mg/kg) group, and the mode of administration was intraperitoneal injection; the cell suspension was injected into the mice in the A6-PG (0.03 mg/kg) + 8B CAR-T group and the A6-PG (0.1 mg/kg) + 8B CAR-T group via the tail vein at 0.2 mL/mouse. The mice were monitored twice a week for the body weight and the maximum length of major axis (L) and maximum length of minor axis (W) of tumor tissues.

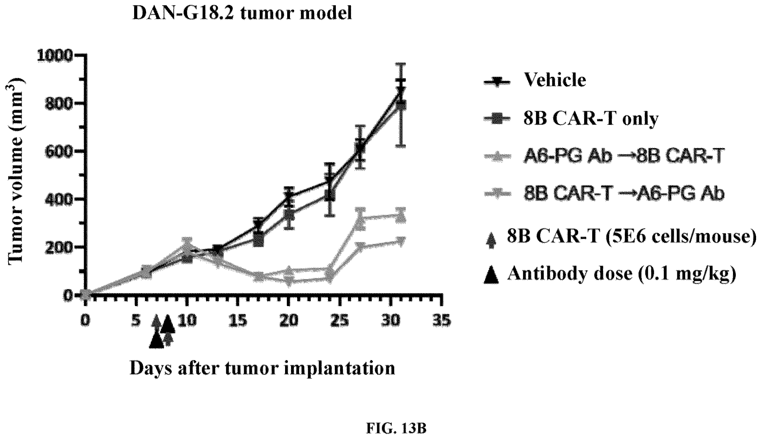

FIGs. 13A and 13B show the anti-tumor effects produced by different administration sequences of the HuR968B CAR-T cells and the P329G A6 antibody in immunodeficient tumor-bearing mice inoculated with human DAN-G18.2 tumor cells. The results indicate that whether the CAR-T cells or the P329G antibody was administered first, it ultimately did not affect the anti-tumor effect, and that the 2 different administration regimens induced similar anti-tumor effects: in the 0.03 mk/kg dose groups, the maximum TGI was 70.4% for the group given the CAR-T cells first and 75.4% for the group given the antibody first; in the 0.1 mk/kg dose groups, the maximum TGI was 86.4% for the group given the CAR-T cells first and 76.7% for the group given the antibody first.

### Example 12. Expression, Purification, and In-Vitro Binding Ability Study of Low-Affinity Antibodies

### (12-1) Expression and purification of antibodies

The light and heavy chain variable region sequences (SEQ ID NO: 42, SEQ ID NO: 43) of an Hz3G3 antibody (this antibody is an anti-CLDN18.2 antibody, the sequence of which is disclosed in Patent Application No. PCT/CN2021/100870 and which is also referred to herein simply as G3 antibody), the light and heavy chain variable region sequences (SEQ ID NO: 44, SEQ ID NO: 45) of an Hz69H9-1.2 antibody (also referred to herein simply as H9-1.2 antibody), the light and heavy chain variable region sequences (SEQ ID NO: 46, SEQ ID NO: 47) of an Hz69H9-2.1 antibody (also referred to herein simply as H9-2.1 antibody), and the light and heavy chain variable region sequences (SEQ ID NO: 48, SEQ ID NO: 49) of an Hz69H9-SA antibody (also referred to herein simply as H9-SA antibody) were loaded into pcDNA3.4 expression vectors (Shanghai Bio-Innovation) containing a P329G point-mutated human IgG1 heavy chain constant region sequence (SEQ ID NO: 39) and a κ light chain constant region sequence (SEQ ID NO: 40), respectively, wherein these antibodies are directed against CLDN18.2; the Hz69H9-1.2, Hz69H9-2.1 and Hz69H9-SA antibodies are mutants of the parent Hz69H9 antibody, with point mutations introduced into some amino acids of VH or VL. The light and heavy chain expression vectors were co-transfected into HEK293 cells by PEI at a molar ratio of 2:3. The cells were cultured for 5-7 days, and then the supernatant of the culture medium was collected. The antibody-containing supernatant medium was purified in one step through a Protein A column, followed by dialysis with PBS to obtain an Hz69H9-1.2 PG antibody, an Hz69H9-2.1 PG antibody, an Hz69H9-SA PG antibody, and an Hz3G3 PG antibody. The concentration of the antibodies was detected using a NanoDrop instrument by reading the absorbance at 280 nm, and the purity of the samples was detected using SDS-PAGE and SEC-HPLC methods.

### (12-2) Study on in-vitro binding abilities of antibodies

The antibodies were prepared into 5-fold gradient diluted antibody solutions at different concentrations with an FACS buffer, which were then separately incubated with 1E5 target cells (CHO-GS, DAN-G18.2, NUGC-4) at 4 °C for 30 min, washed with an FACS buffer, and further incubated with APC-goat anti-human IgG, Fcy fragment specific (Jackson ImmunoResearch, 109-136-098) at 4 °C for another 30 min. The antibodies bound to the cells were detected by flow cytometry, and the APC channel MFI was analyzed. Plotting was performed with the antibody concentration as the X-axis and the APC channel MFI as the Y-axis, and the EC₅₀ for binding was calculated.

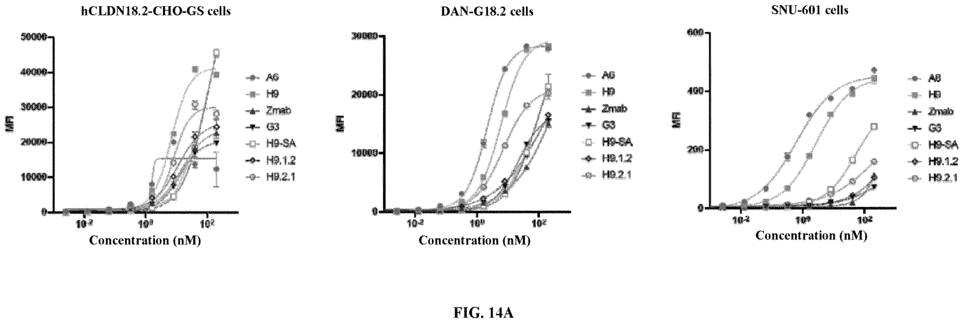

FIG. 14A shows the binding abilities of the 7 different anti-CLDN18.2 antibodies to CLDN18.2 obtained based on cytological experiments. Overall, the A6 (HB37A6 antibody) and H9 (Hz69H9 antibody) antibodies had high binding abilities, while the binding abilities of the H9-SA, H9-2.1, H9-1.2, Zmab and G3 antibodies were relatively lower.

Table 5 summarizes the EC₅₀ values for the binding of the 7 different anti-CLDN18.2 antibodies to the 3 different CLDN18.2-expressing cells in the experiments of FIG. 14A. The results indicate that for the CLDN18.2-CHO-GS cells with high CLDN18.2 expression, the binding abilities are ranked as follows: A6 > H9 > H9-2.1 > H9-1.2 > G3 > Zmab > H9-SA; for the DNA-G18.2 cells with high CLDN18.2 expression, the binding abilities are ranked as follows: A6 > H9 > H9-2.1 > G3 > H9-SA > H9-1.2 > Zmab; for the SNU601 cells with low CLDN18.2 expression, the binding abilities are ranked as follows: A6 > H9 > H9-SA > H9-2.1 > G3 > H9-1.2 > Zmab.

**Table 5**

| EC50 (nM) | **A6** | **H9** | **H9.2.1** | **H9-SA** | **G3** | **H9.1.2** | **Zmab** |
|---|---|---|---|---|---|---|---|
| CHO-GS-hCLDN18.2 | ∼ 1.605 | 6.854 | 8.21 | 113.6 | 12.57 | 11.06 | 14.37 |
| DAN-G18.2 | 2.11 | 5.977 | 6.95 | 92.14 | 20.83 | 174.4 | 562.9 |
| SUN601 | 0.6034 | 2.754 | 261.0 | 49.25 | 794.1 | ∼ 8234 | ∼ 97876 |

### (12-3) Antibody affinity (avidity) assay

According to the manufacturer's instructions, human Claudin18.2-His (Aero, CL2-H5546) was coupled onto a CM5 chip (GE Healthcare, 29-1496-03) using an amino coupling kit (GE Healthcare, BR-1000-50), and the remaining activation sites were blocked by addition of 1 M ethanolamine after coupling. The affinity parameters were obtained by detecting the binding and dissociation of the surface antigen to each antibody in the mobile phase using Biacore (GE Healthcare, T200). The diluted antibody flowed over the chip in order from low to high concentration, and the chip was finally regenerated using 10 mM glycine pH 1.5 (GE Healthcare, BR-1003-54). Finally, the data were analyzed by the Biacore T200 analysis software.

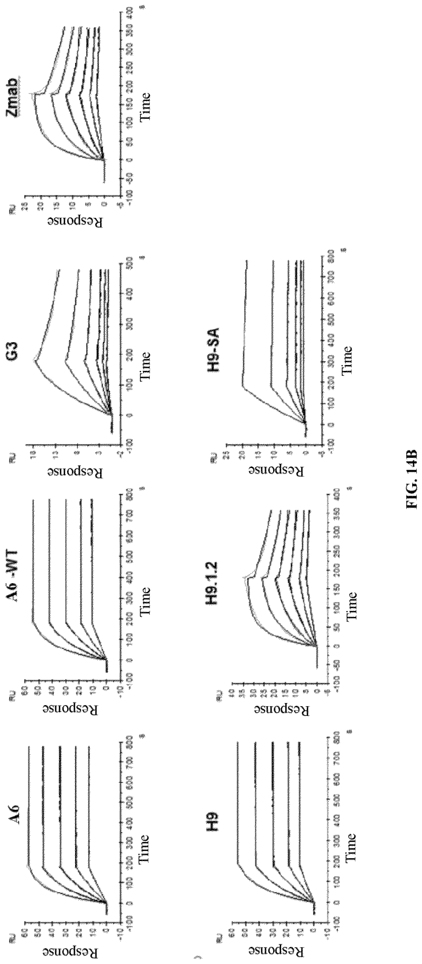

FIG. 14B shows representative affinity profiles of the above 6 antibodies for the recombinant human CLDN18.2 protein as determined using surface plasmon resonance (SPR). The results show that the A6 and H9 antibodies had the highest affinity (with KD values of <5.043E-12 and <5.269E-12, respectively), followed by the H9-SA and H9-1.2 (with KD values of 3.254E-10 and 9.804E-10, respectively), and then the Zmab and G3 antibodies (with KD values of 1.378E-9 and 2.502E-9, respectively).

Table 6 shows the specific assay results. These results are consistent with the results of the cytological binding experiments, indicating that A6 and H9 are high-affinity antibodies, while H9-SA, H9-1.2, Zmab, and G3 are low-affinity antibodies as compared to the A6 and H9 antibodies.

**Table 6**

| Antibody | | KD (M) | | ka (1/Ms) | | kd (1/s) | |
|---|---|---|---|---|---|---|---|
| | A6 PG | | <4.085E-12 | | 2.448E+6 | | <1.000E-5 |
| | A6 WT | | <5.043E-12 | | 1.983E+6 | | <1.000E-5 |
| | H9 | | <5.269E-12 | | 1.898E+6 | | <1.000E-5 |
| | H9-SA | | 3.254E-10 | | 4.709E+5 | | 1.532E-4 |
| | H9.1.2 | | 9.804E-10 | | 1.886E+6 | | 1.849E-3 |
| | Zmab | | 1.378E-9 | | 1.648E+6 | | 2.270E-3 |
| | G3 | | 2.502E-9 | | 7.090E+5 | | 1.774E-3 |

### Example 13. In-Vitro Functional Study of Low-Affinity Antibodies

### (13-1) Cytokine release experiment

CAR-T cells were thawed and stabilized in culture at 37 °C overnight. Tumor target cells and the CAR-T cells were mixed at an E:T ratio of 1:1, and 5-fold gradient diluted antibody solutions at different concentrations were separately added. The mixture was incubated at 37 °C for about 24 h and then centrifuged to collect the cell supernatant. The factors in the cell supernatant were detected using the BD^{™} Cytometric Bead Array (CBA) Human Th1/Th2 Cytokine Kit II (BD, 551809). The specific procedure was as follows: Equal volumes of Capture Beads in the kit were mixed well and then plated on a 96-well V-bottom plate at 25 µL/well. Equal volumes of the supernatant or a supernatant dilution and a standard were separately added. The mixture was mixed well, and then an equal volume of Human Th1/Th2 PE Detection Reagent was added, followed by incubation at room temperature in the dark for 3 h. The cells were washed twice with a Wash buffer, then resuspended, and detected using a flow cytometer, and the cytokine concentrations were calculated based on PE channel MFI values.

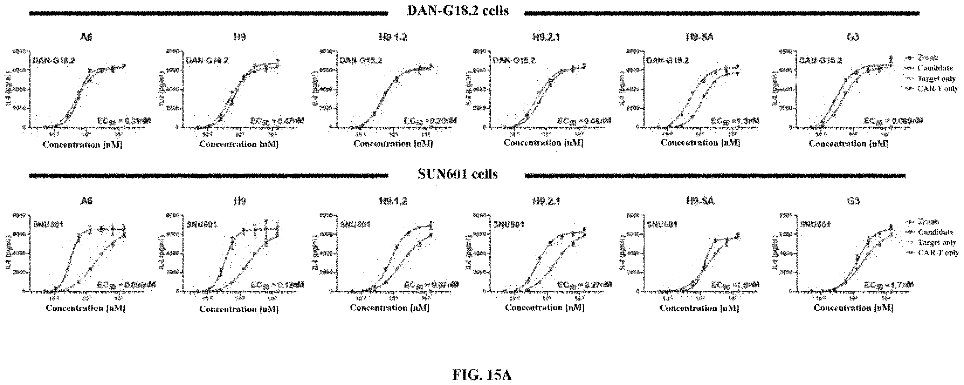

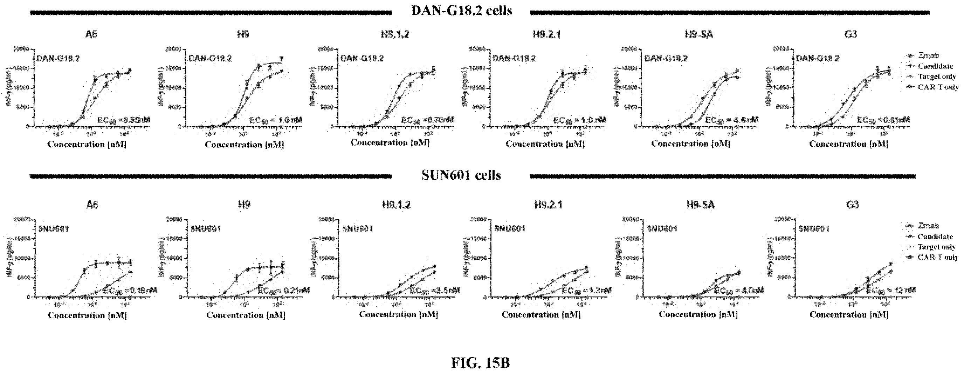

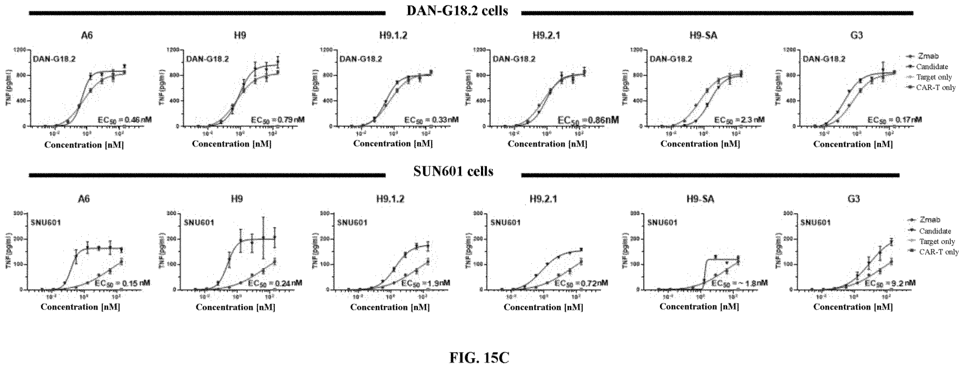

FIGs. 15A-15C show the results of 7 different antibodies inducing HuR968B CAR-T cells to release effector cytokines IL-2, IFN-γ and TNF-α in co-culture experiments with DAN-G18.2 or SNU601 tumor cells, respectively. Under the condition of adding different concentrations of P329G mutated antibody, the HuR968B CAR-T cells were activated and secreted effector cytokines such as IL-2, IFN-γ, and TNF-α, and the release levels of the cytokines exhibited an antibody dose-dependence; using the Zmab antibody as a control, under the stimulation by the DAN-G18.2 cells with high CLDN18.2 expression, the high-affinity antibodies A6 (A6 PG) and H9 (H9 PG) induced slightly higher levels of IFN-γ and TNF-α secretion, and the effector cytokine secretion induced by the other 4 low-affinity antibodies showed no significant difference; however, under the stimulation by the SNU601 cells with low CLDN18.2 expression, the levels of effector cytokines induced by the high-affinity antibodies A6 (A6 PG) and H9 (H9 PG) were significantly higher than those induced by the Zmab (Zmab PG) and other low-affinity antibodies, and the levels of cytokine secretion induced by the antibodies H9-2.1 (H9-2.1 PG) and H9-2.1 (H9-2.1 PG) were also slightly higher than those induced by the Zmab (Zmab PG) antibody, especially at high concentrations. These results are consistent with the above antibody affinity results.

### (13-2) End-Point killing experiment

The CAR-T cells prepared in Example 2 were thawed and stabilized in culture at 37 °C overnight. Tumor target cells and the CAR-T cells were mixed at an E:T ratio of 1:1, and 5-fold gradient diluted antibody solutions at different concentrations were separately added to achieve a total volume of 200 µL. The mixture was incubated at 37 °C for about 24 h and then centrifuged. The cell supernatant was transferred to a 96-well white-bottom microplate. The LDH value in the supernatant was measured using the CytoTox 96 Non-Radioactive Cytotoxicity Assay (Promega, G1780) and a microplate reader (Molecular Devices, SpectraMax i3x) to calculate the killing efficiency.

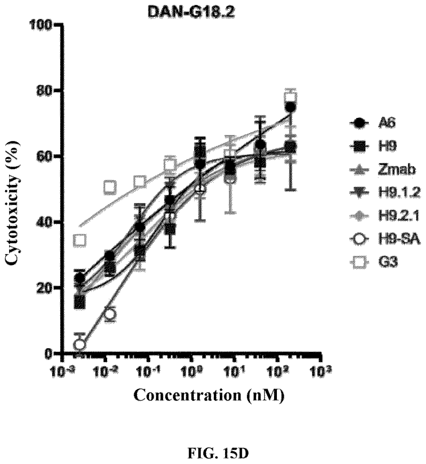

FIG. 15D shows the killing effects of HuR968B CAR-T cells on DAN-G18.2 tumor cells induced by the 7 different antibodies in Example 12. Under the condition of using DAN-G18.2 cells with high CLDN18.2 expression as target cells, there was no significant difference in the killing effects of the HuR968B CAR-T cells induced by high- or low-affinity antibodies, consistent with the effector cytokine results of Example 13-1.

### (13-3) Dynamic killing experiments comparing killing effects of CAR-T cells on tumor cells induced by high- or low-affinity antibodies

The killing of target cells by CAR-T cells was dynamically detected in real time using an xCELLigence RTCA MP instrument. In an E-Plate, 50 µL of culture medium was added. After the instrument read the baseline value, 50 µL of tumor target cells were added, and the plate was placed in the instrument for dynamic detection. At the same time, CAR-T cells were thawed and incubated in an incubator at 37 °C overnight. After about 24 h, the CAR-T cells were added at an E:T ratio of 1:1, and gradient diluted antibody solutions at different concentrations were separately added to the corresponding wells. The plate was placed in an xCELLigence RTCA MP instrument system to dynamically monitor the killing of the target cells by the P329G CAR-T cells for 72-96 h.

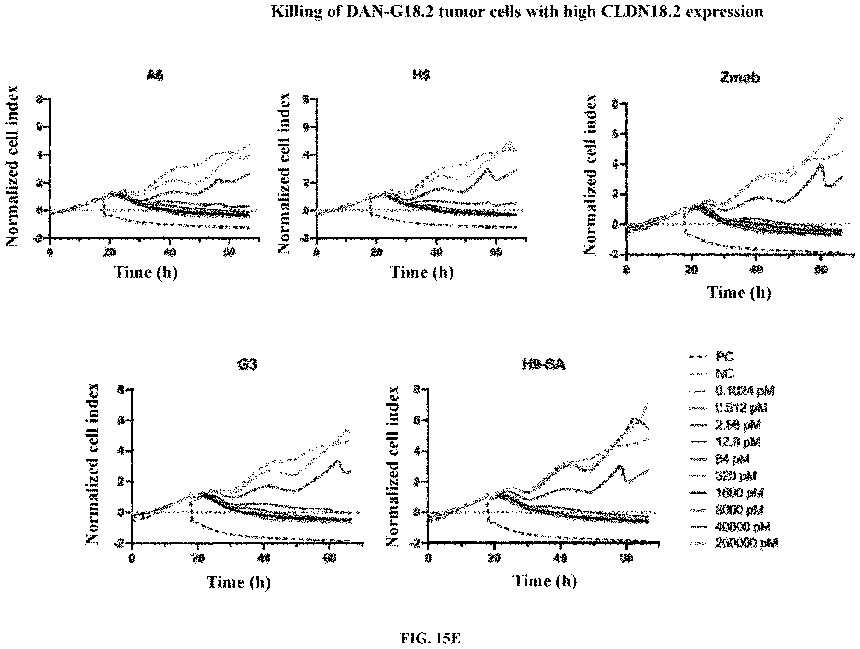

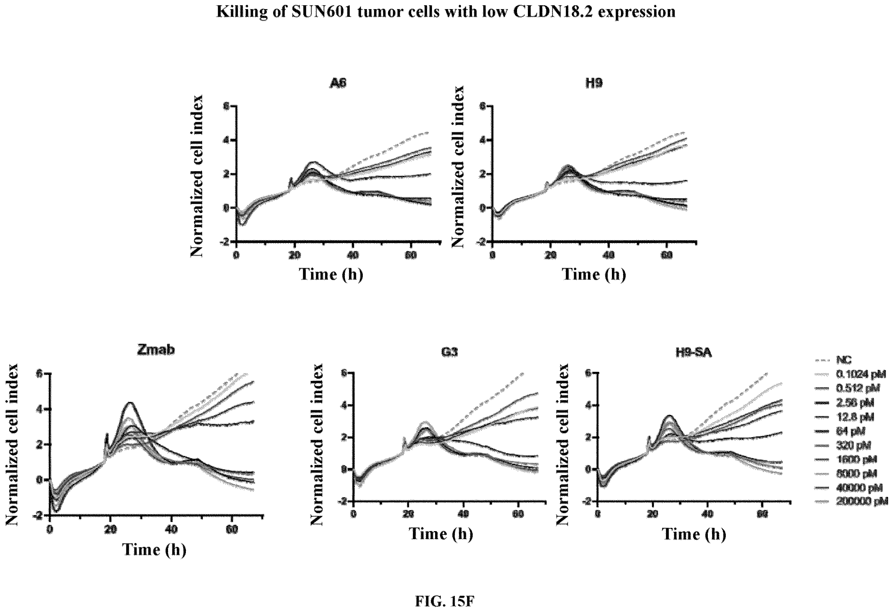

FIGs. 15E and 15F show the results of dynamic killing experiments comparing the killing effects of HuR968B CAR-T cells on tumor cells with high and low CLDN18.2 expression induced by high- or low-affinity antibodies, respectively. The results in FIG. 15E show that the high-affinity antibodies (A6, H9) and low-affinity antibodies (Zmab, G3, H9-SA) induced an equivalent degree of killing of the DAN-G18.2 tumor cells with high CLDN18.2 expression by the HuR968B CAR-T cells, and were capable of inducing a complete killing effect of the HuR968B CAR-T cells on the tumor cells in a short time (8 h) at concentrations as low as 12.8-64 pM. FIG. 15F results show that the high-affinity antibodies (A6, H9) were still capable of inducing complete killing of the SNU601 tumor cells with low CLDN18.2 expression at concentrations of 12.8-64 pM, but the low-affinity antibodies (Zmab, G3, H9-SA) required concentrations above 64 pM to induce a complete killing effect; furthermore, compared to the killing of the DAN-G18.2 tumor cells with high CLDN18.2 expression, the complete killing of the SNU601 tumor cells with low CLDN18.2 expression took longer (36 h or longer).

### Example 14. Study on In-Vivo Anti-Tumor Effects of HuR968B CAR-T Cells in Combination with Low-Affinity Antibodies

Mouse tumor inoculation and treatment were performed. DAN-G18.2 cells were resuspended in 1× PBS to prepare a cell suspension at a concentration of 5 × 10⁶ cells/mL. NOG mice were subjected to shaving at the right back and injected subcutaneously with the DAN-G18.2 cell suspension at 5 × 10⁶ cells/mL in a volume of 0.2 mL/mouse, i.e., at an inoculum size of 1 × 10⁶ cells/mouse. 7 days after the tumor cell inoculation, mice with mouse tumor volumes of 76.25-125.62 mm³ were divided into 6 groups of 7, namely a vehicle group, an 8B CAR-T-only group, an 8B CAR-T + Zmab-PG group, an 8B CAR-T + H9-PG group, an 8B CAR-T + H9.1.2-PG group, an 8B CAR-T + H9.SA-PG group, and an 8B CAR-T + G3-PG group. After the grouping was completed, antibody administration was performed at a dose of 1 mg/kg, and the 2^{nd} antibody administration was performed 3 weeks later. On day 1 after the antibody administration, i.e., on day 8 after the tumor cell inoculation, the 8B CAR-T cells were resuspended in 1× PBS to prepare an 8B CAR⁺ cell suspension at 2.5 × 10⁷ cells/mL. The cell suspension was injected into the mice via the tail vein at 0.2 mL/mouse, corresponding to the reinfusion of 5 × 10⁶ CAR⁺ cells/mouse. The mice were monitored twice a week for the body weight and the maximum length of major axis (L) and maximum length of minor axis (W) of tumor tissues.

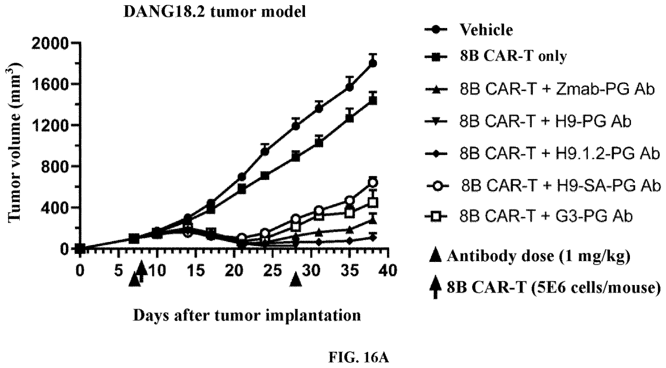

FIG. 16A shows the therapeutic effects of HuR968B CAR-T cells in combination with different CLDN18.2 antibodies in immunodeficient tumor-bearing mice inoculated with human DAN-G18.2 tumor cells. The results show that tumor growth was significantly inhibited after the infusion of HuR968B CAR-T cells in combination with 2 doses of different P329G antibodies (administered 3 weeks apart), with the maximum anti-tumor effect achieved approximately 2 weeks after treatment (D24 in FIG. 16A); the TGI values for the mice in the combination treatment groups administered the Zmab PG antibody, H9 PG antibody, H9-1.2 PG antibody, H9-SA PG antibody, and G3 PG antibody were 93.8%, 97.0%, 94.6%, 85.0%, and 89.5%, respectively; among them, the mice in the combination treatment group given the H9-1.2 PG antibody maintained a sustained anti-tumor effect, with TGI still at 94.0% 30 days after treatment (D38 in FIG. 16A); in contrast, the tumor growth inhibition (TGI) values of the mice in the combination treatment groups administered the Zmab PG, H9-SA PG and G3 PG antibodies were 84.4%, 64.3%, and 75.1%, respectively. Although the high-affinity antibody H9 PG induced a good anti-tumor effect, it also caused toxicity, necessitating the early sacrifice of this group of mice.

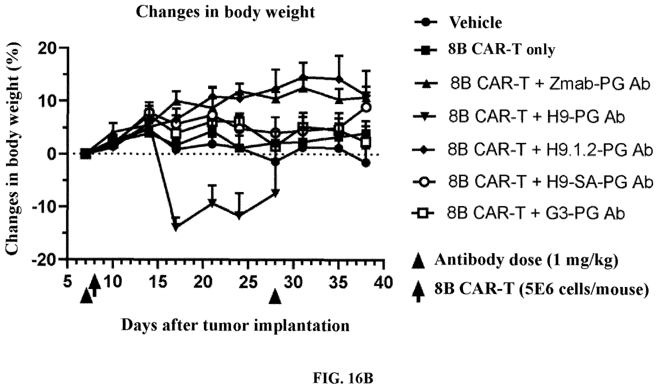

FIG. 16B shows the changes in body weight of mice during this experiment. The results show that mice treated with the HuR968B CAR-T cells in combination with the Zmab PG, H9-1.2 PG, H9-SA PG and G3 PG antibodies did not show significant changes in body weight compared to the mice in the control group. However, the body weight of the mice in the combination treatment group given the H9 PG antibody rapidly declined within 1 week after receiving the 1^{st} dose of antibody, with an average weight loss of 8.9%; the body weight then returned to normal levels approximately in 1 week, but after administration of the 2^{nd} dose of antibody, the body weight of the mice rapidly declined again, necessitating sacrifice of the mice. The results indicate that at a higher antibody dose (1 mg/kg), the combination treatment with the high-affinity H9 PG antibody induced a significant anti-tumor effect but also produced intolerable toxicity, whereas the combination treatment with the low-affinity Zmab PG, H9-1.2 PG, H9-SA PG and G3 PG antibodies induced similar anti-tumor effects but did not induce significant toxic effects.

### Example 15. PG CAR Off-Target Binding Site Screening and Functional Assay

### (15-1) Membrane proteome array (MPA) study protocol

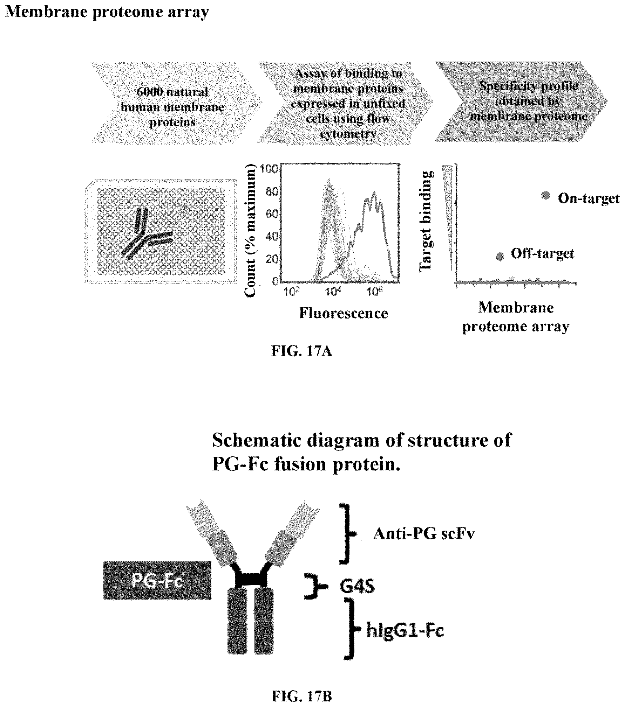

This study was done at Intergral Molecular. MPA is a high-throughput method for analyzing antibody-target specificity (FIG. 17A). This method uses flow cytometry to directly detect the binding of a test antibody to membrane proteins expressed in unfixed cells. The antibody of interest was subjected to reactivity testing against a library of approximately 6000 natural human membrane proteins, including 94% of all single-transmembrane, multi-transmembrane and GPI-anchored proteins. All of the screened target proteins had natural conformation and appropriate post-translational modifications. After high-throughput identification of binding targets, subsequent experiments were performed to further confirm the binding reactivity of the antibody of interest and the membrane proteins. To optimize the detection conditions for the test antibody, HEK-293T cells (ATCC, CRL-3216; 18,000 cells/well) and QT6 cells (ATCC, CRL-1708; 18,000 cells/well) were plated in a 384-well cell culture plate (Corning, 3764) using a DMEM (Corning, 10-017-CM) complete medium (containing 10% FBS (Tissue Culture Biologicals, 101), 2 mM L-alanyl-L-glutamine (Corning, 25-015-CI), penicillin/streptomycin (Corning, 30-002-CI), MEM NEAA (Corning, 25-025-CI), and HEPES 10 mM (Corning, 25-060-CI)), transfected with a plasmid encoding protein A (which bound to an antibody Fc fragment; positive control) or an empty vector (pUC; negative control), and incubated at 37 °C with 5% CO₂ for 36 h. The test antibody (PG-Fc, FIG. 17B; SEQ ID NO: 52) was four-fold diluted starting at 20 µg/mL (using PBS containing 10% normal sheep serum (Sigma, G6767) as the diluent) to give four dilutions. Four replicates of each dilution were added to the transfected cells, and then a secondary antibody (AlexaFluor 647-AffiniPure Goat Fab Anti-Human IgG, Jackson ImmunoResearch, 109-606-008) at a fixed concentration was added. Detection was performed using high-throughput immunofluorescence flow cytometry. The mean fluorescence intensity (MFI) value for each antibody dilution was determined using the ForeCyt software (Intellicyt) and plotted using Excel (Microsoft). The MFI value detected for each antibody concentration was converted into a signal ratio consisting of target binding (positive signal) and negative control (background signal), based on which the optimal screening concentration for the test antibody was determined. On this basis, plasmids containing approximately 6000 membrane protein cDNA clones were transfected into HEK-293T cells, with each well containing one unique cDNA plasmid, and a GFP-encoding plasmid or membrane-bound protein A construct was used as a control for transfection efficiency and positive binding. After 36 h of incubation, the cells were stripped using CellStripper (Corning, 25-056-CI) and re-plated in a new 384-well plate using a JANUS automated workstation (Perkin-Elmer, AJL8M01). The test antibody was added to the membrane proteome array at the optimized concentration described above, and then a secondary antibody (AlexaFluor 647-AffiniPure Goat Fab Anti-Human IgG, Jackson ImmunoResearch, 109-606-008) at a fixed concentration was added. Detection was performed using high-throughput immunofluorescence flow cytometry, and flow cytometry data were analyzed using the ForeCyt software (Intellicyt). The binding targets identified in the first round of screening were verified through a second round of screening using the aforementioned method.

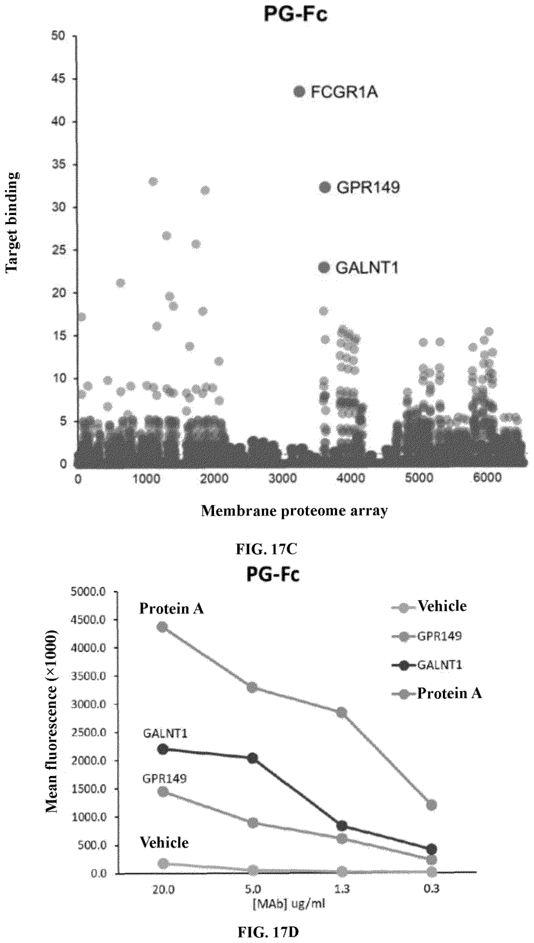

FIG. 17C shows the results of the first round of high-throughput screening for the binding of the test PG-Fc fusion protein to membrane proteins, indicating that the PG-Fc fusion protein could bind not only to the FCGR1A protein as a positive control, but also to the GALNT1 and GPR149 proteins named according to the HUGO Gene Nomenclature Committee (HGNC).

FIG. 17D shows the results of the second round of experimental validation for the binding of the test PG-Fc fusion protein to membrane proteins, indicating that the PG-Fc fusion protein bound to the GALNT1 and GPR149 proteins.

This indicates that the GALNT1 and GPR149 proteins bound to PG-Fc fusion proteins are off-target proteins for the binding of the PG-Fc fusion protein, relative to the binding of the PG-Fc fusion protein to the target P329G antibody.

### (15-2) Construction of cell lines overexpressing off-target genes

The gene coding sequences for GPR149 (G protein-coupled receptor 149) and GALNT1 (polypeptide N-acetylgalactosaminyltransferase 1) were downloaded from the Uniprot database, wherein the Uniprot accession number for GPR149 is **Q86SP6,** and the Uniprot accession number for GALNT1 is **Q10472.** These sequences were then subjected to whole-gene synthesis and cloned into a pRK lentiviral vector by Genewiz, and a lentiviral vector plasmid expressing both the off-target proteins and the EGFR fluorescence protein was constructed. A lentiviral vector expressing CH2-PG was constructed as a positive control (the amino acid sequence of CH2-PG is set forth in SEQ ID NO: 53, the CH2-PG in the text is equivalent to the expression of a CAR-recognized PG antibody fragment on the cell surface). Lentiviruses were prepared in a manner similar to Example 2-1, and used to infect CHO GS (ATCC, CRL-3216) suspension and 293T (ATCC, CRL-3216) adherent cells at an MOI of 5. After 3 days, cells positive for high-purity EGFR expression were obtained using EGFR fluorescence flow cytometry sorting, which were then expanded for use in subsequent experiments.

### (15-3) Off-target gene expression detection

The above overexpression cell line was detected for off-target gene expression using flow cytometry and immunohistochemistry (IHC). For flow cytometry detection, a single-cell suspension of the above overexpression cells was prepared, and a gradient dilution of the PG-Fc fusion protein **(0.004-400 nM)** was added; the mixture was incubated at 4 °C for 30 min and washed twice with an FACS buffer, and then an APC-goat anti-human IgG, Fcy fragment specific (Jackson ImmunoResearch, 109-136-098) secondary antibody was added; the mixture was further incubated at 4 °C for another 30 min and washed twice with an FACS buffer; after resuspension, the cells were detected using a flow cytometer. For IHC detection, the overexpression cells were collected, fixed in 10% neutral buffered formalin for 8 h, and centrifuged to prepare cell clusters, which were placed in a full-automatic dehydration machine for dehydration and paraffin immersion; after the dehydration and paraffin immersion, the cell clusters were placed in a paraffin embedding machine for paraffin embedding; the paraffin cell blocks were sectioned on a sectioning machine to a thickness of 3 µm; the sections of the paraffin cell blocks were stained in a Ventana full-automatic immunohistochemistry staining instrument (Ventana Discovery ULTRA) using the following antibodies: an anti-GALNT1 polyclonal antibody (HPA012628, Sigma) and an anti-GPR149 polyclonal antibody (HPA018020, Sigma); the 3 types of stained cell block sections were mounted with neutral gum, observed under a microscope, and scanned using a digital pathology scanner to capture images.

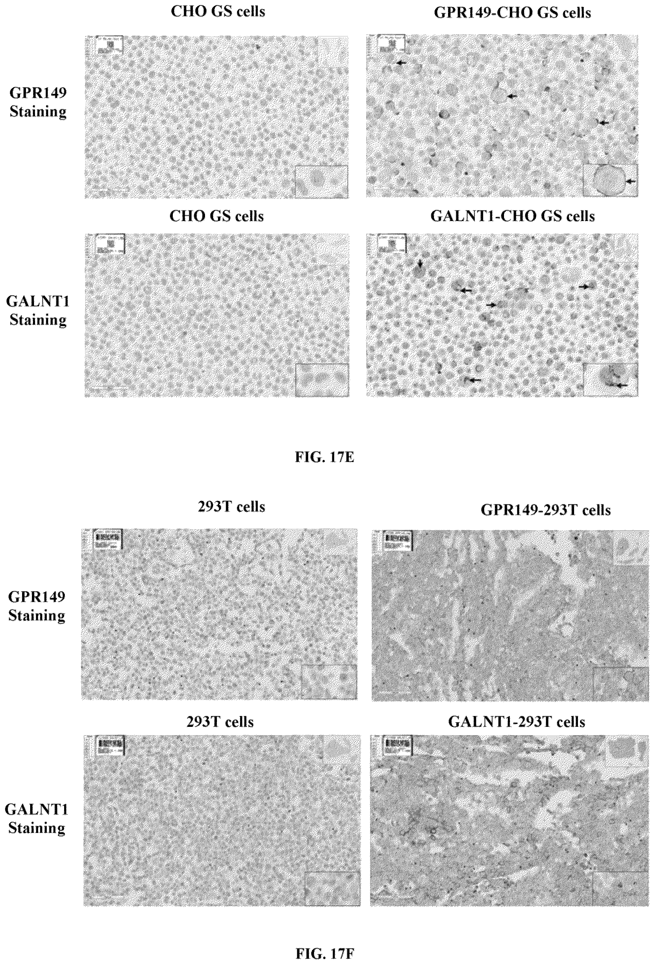

FIG. 17E shows the expression of GALNT1 and GPR149 proteins in CHO-GS cells overexpressing off-target genes as detected by IHC, with GALNT1 primarily expressed in the cytoplasm and GPR149 primarily expressed on the cell membrane.

FIG. 17F shows the expression of GALNT1 and GPR149 proteins in 293T cells overexpressing off-target genes as detected by IHC, with GALNT1 also primarily expressed in the cytoplasm and GPR149 also primarily expressed on the cell membrane.

### (15-4) Activating effects of off-target genes on P329G CAR-T cells:

P329G CAR-T cells were thawed and stabilized in culture at 37 °C overnight. The cells overexpressing off-target genes and CAR-T cells were mixed at E:T ratios of 3:1, 10:1, and 20:1, and an appropriate amount of complete medium was added to each well to achieve a total volume of 200 µL. After incubation at 37 °C for about 24 h, the mixture was centrifuged, and the cells were collected, washed twice with an FACS buffer, then resuspended, and stained with an FACS buffer containing LIVE/DEAD Fixable Dead Cell Stain and Fragment Goat Anti-Human IgG (Jackson ImmunoResearch, 109-066-006) at 4 °C for 30 min. The stained cells were then washed twice, and an antibody combination of CD4, CD8, CD25, CD69, and APC-streptavidin was added. The above cell-antibody mixed solution was stained at 4 °C for 30 min, washed twice, resuspended in an FACS buffer, and then detected using a flow cytometer.

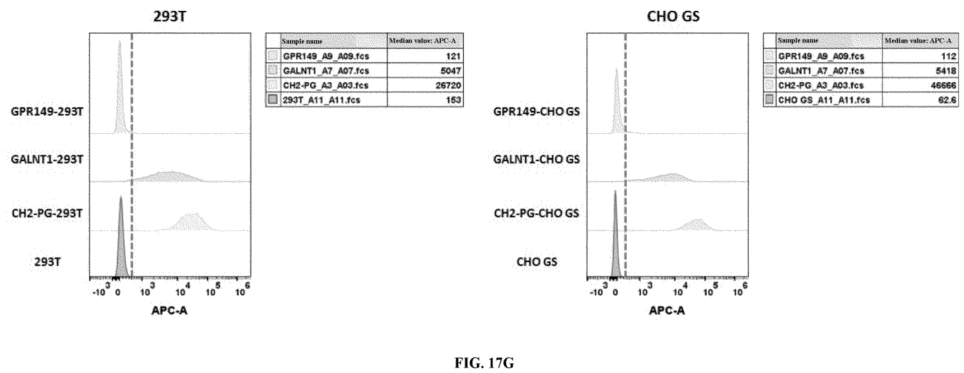

FIG. 17G shows the expression of off-target genes as detected by flow cytometry, with CH2-PG-expressing cells as a positive control.

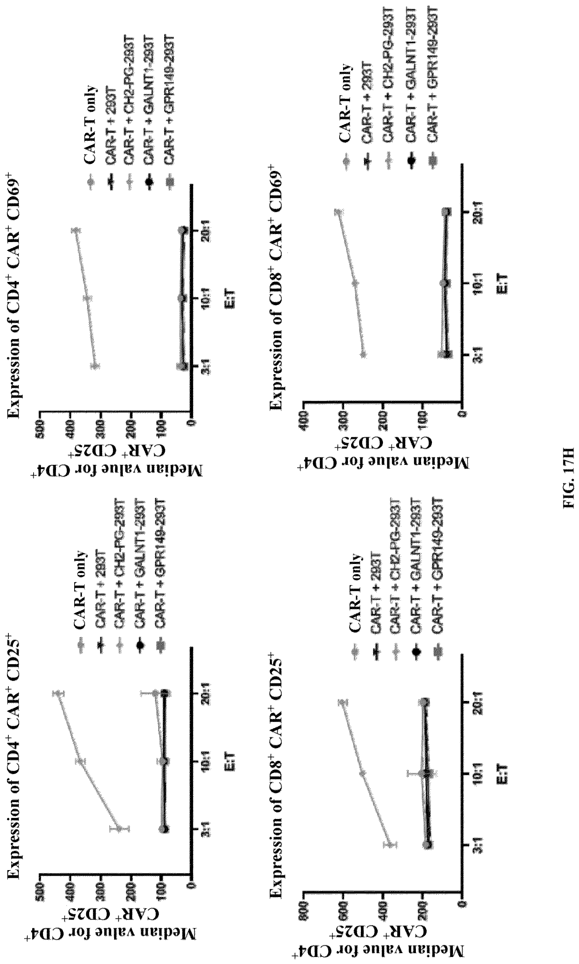

FIG. 17H shows the activating effects of HuR968B CAR-T cells on different target cells. Neither wild-type 293T cells nor 293T cells expressing the off-target gene GALNT1 or GPR149 were capable of mediating the activating effect on P329G CAR-T cells. Only the CH2-PG-293T cells expressing the P329G mutation were capable of specifically mediating the CAR⁺-T activation, up-regulating the expression levels of CD25 and CD69, with no differential activation between CD4-CAR⁺ and CD8-CAR⁺ cells, and exhibiting a gradient dependence on the E:T ratio.

**(15-5) Cytokine detection:** The cell supernatant in the above experiment was collected after centrifugation, and cytokines were detected using the BD^{™} Cytometric Bead Array (CBA) Human Th1/Th2 Cytokine Kit II. Equal volumes of Capture Beads in the kit were mixed well and then plated at 25 µL/well. An equal volume of the supernatant or a supernatant dilution or a standard was added. The mixture was mixed well, and then an equal volume (25 µL) of Human Th1/Th2 PE Detection Reagent was added, followed by incubation at room temperature in the dark for 3 h. The cells were washed twice with a Wash buffer, then resuspended, and detected using a flow cytometer, and the cytokine concentrations were calculated based on PE channel MFI values.

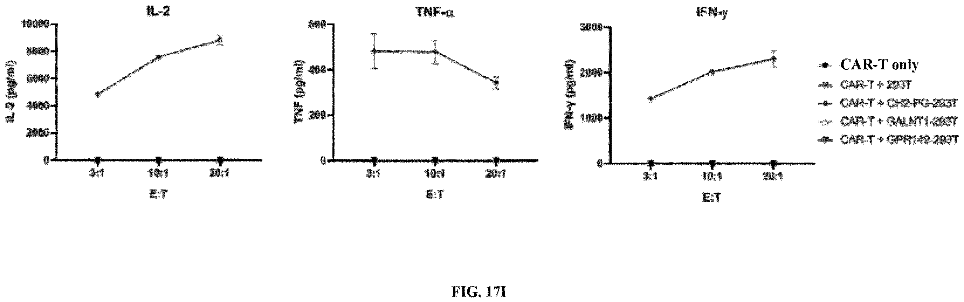

FIG. 17I shows the results of the release of effector cytokines from the P329G CAR-T cells co-cultured with different target cells. Neither wild-type 293T cells nor 293T cells expressing off-target proteins GALNT1 and GPR149 were capable of activating the P329G CAR-T CAR-T cells to secrete effector cytokines such as IL-2, IFN-γ, and TNF. Only with the addition of the CH2-PG-293T cells expressing the P329G mutation, the P329G CAR-T cells were activated to secret effector cytokines, exhibiting a gradient dependence on the E:T ratio.

**(15-6) Killing efficiency detection:** The killing of target cells expressing off-target genes by the CAR-T cells described above was dynamically detected in real time using an xCELLigence RTCA MP instrument. In an E-Plate, 50 µL of culture medium was added. After the instrument read the baseline value, 50 µL of off-target gene-overexpressing cells (10000 cells) were added, and the plate was placed in the instrument for dynamic detection. At the same time, UNT and CAR-T cells were thawed and incubated in an incubator at 37 °C overnight. After about 24 h, the positive rates of all CAR-T cells were adjusted to be consistent using the UNT cells. Different CAR-T cells prepared from PBMCs from the same donor were added at E:T ratios of 6.7:1, 13:1, and 20:1, respectively, and an appropriate amount of complete medium was added to each corresponding well to achieve a total volume of 200 µL. The killing of the target cells by the P329G CAR-T cells was dynamically monitored by the xCELLigence RTCA MP instrument system for 110 h.

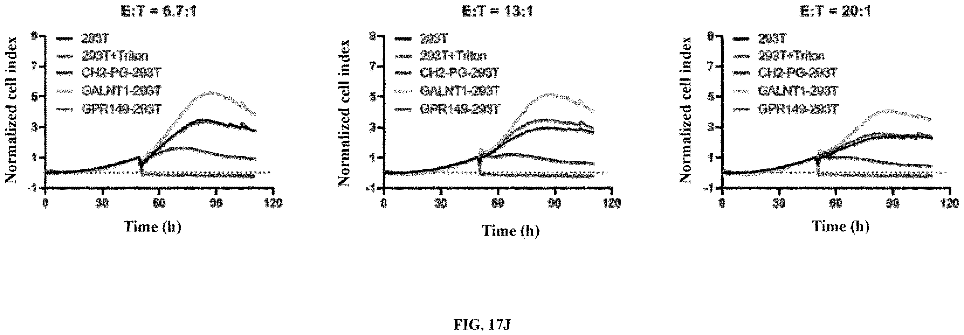

FIG. 17J shows the results of sustained killing of different target cells by P329G CAR-T cells at E:T ratios of 6.7:1, 13:1 and 20:1, respectively. Under the conditions of 3 different E:T ratios, only the CH2-PG-293T cells expressing the P329G mutation were capable of activating P329G CAR-T, thereby generating a sustained killing effect on the target cells. The 293T cells expressing off-target proteins GALNT1 and GPR149 yielded results consistent with those in the wild-type 293T negative control group, as both failed to induce the killing of the target cells by the P329G CAR-T cells and exhibited a gradient dependence on the E:T ratio. These results were consistent with the above results showing that the CAR-T cell activation and effector cytokines exhibited a gradient dependence on the E:T ratio.

The exemplary embodiments of the present invention have been described above. It should be understood by those skilled in the art that these contents are merely exemplary, and various other replacements, adaptations, and modifications can be made within the scope of the present invention. Therefore, the present invention is not limited to the specific embodiments listed herein.

### SEQUENCE LISTING

| **Sequence No.** | **Name** | **Amino acid sequence/nucleotide sequence** |
|---|---|---|
| SEQ ID NO: 1 | SP | MALPVTALLLPLALLLHAARP |
| SEQ ID NO: 2 | Anti-PG antibody VH | EVQLVESGGGLVQPGGSLRLSCAASGFTFSRYWMNWVRQAPGKGLE WVGEITPDSSTINYAPSLKGRFTISRDNAKNSLYLQMNSLRAEDTAVYY CARPYDYGAWFASWGQGTLVTVSS |
| SEQ ID NO: 3 | Anti-PG antibody VL | QAVVTQEPSLTVSPGGTVTLTCRSSTGAVTTSNYANWVQEKPDHLFTG LIGGTNKRAPGTPARFSGSLLGGKAALTLSGAQPEDEAEYYCALWYSN HWVFGGGTKLTVL |
| SEQ ID NO: 4 | (G4S)4 | GGGGSGGGGSGGGGSGGGGS |
| SEQ ID NO: 5 | (G4S) | GGGGS |
| SEQ ID NO: 6 | IgG4m (IgG4 spacer region) | ESKYGPPCPPCP |
| SEQ ID NO: 7 | CD28 hinge region | IEVMYPPPYLDNEKSNGTIIHVKGKHLCPSPLFPGPSKP |
| SEQ ID NO: 8 | CD28 TMD | FWVLUWGGVLACYSLLVTVAFIIFWV |
| SEQ ID NO: 9 | CD8 TMD | IYIWAPLAGTCGVLLLSLVITLYC |
| SEQ ID NO: 10 | CD28 CSD | RSKRSRLLHSDYMNMTPRRPGPTRKHYQPYAPPRDFAAYRS |
| SEQ ID NO: 11 | 4-1BB CSD | KRGRKKLLYIFKQPFMRPVQTTQEEDGCSCRFPEEEEGGCEL |
| SEQ ID NO: 12 | CD28F CSD | RSKRSRLLHSDYMFMTPRRPGPTRKHYQPYAPPRDFAAYRS |
| SEQ ID NO: 13 | CD3ζ SSD | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR |
| SEQ ID NO: 14 | CD3ε | KNRKAKAKPVTRGAGAGGRQRGQNKERPPPVPNPDYEPIRKGQRDLY SGLNQRRI |
| SEQ ID NO: 15 | HuR968C Amino acid sequence | MALPVTALLLPLALLLHAARPEVQLVESGGGLVQPGGSLRLSCAASGF TFSRYWMNWVRQAPGKGLEWVGEITPDSSTINYAPSLKGRFTISRDNA KNSLYLQMNSLRAEDTAVYYCARPYDYGAWFASWGQGTLVTVSSGG GGSGGGGSGGGGSGGGGSQAVVTQEPSLTVSPGGTVTLTCRSSTGAVT TSNYANWVQEKPDHLFTGLIGGTNKRAPGTPARFSGSLLGGKAALTLS GAQPEDEAEYYCALWYSNHWVFGGGTKLTVLGGGGSFWVLVVVGG VLACYSLLVTVAFIIFWVRS |
| SEQ ID NO: 16 | HuR968C Nucleotide sequence | ATGGCCCTGCCTGTGACAGCACTGCTACTGCCTCTGGCTCTTCTGTT GCATGCTGCTAGACCTGAGGTTCAGTTGGTGGAGTCTGGGGGTGGA TTAGTGCAGCCTGGGGGTAGTCTGAGACTGAGCTGTGCTGCCTCTG GCTTCACCTTCAGCAGATATTGGATGAACTGGGTGAGACAAGCCCCT GGCAAGGGCCTGGAGTGGGTGGGGGAGATCACCCCTGACAGCAGC ACCATCAACTATGCCCCTAGCCTGAAGGGCAGATTCACCATCAGCAG AGACAATGCCAAGAACAGCCTGTACCTGCAGATGAACAGCCTGAGA GCTGAGGACACAGCTGTGTACTATTGTGCTAGACCCTATGACTATGG GGCCTGGTTTGCTAGCTGGGGCCAAGGCACCCTGGTAACAGTAAGC TCTGGGGGAGGGGGCTCTGGGGGGGGGGGTCTGGGGGGGGGGC TCTGGGGGGGGAGGCTCTCAAGCTGTGGTGACCCAAGAGCCTAGCC TCACTGTGAGCCCTGGGGGCACAGTGACCCTGACCTGCAGAAGCAG CACTGGGGCTGTGACCACAAGCAACTATGCCAACTGGGTGCAAGAG AAGCCTGACCACCTGTTTACTGGCCTGATTGGGGGCACCAACAAGA GAGCCCCTGGCACCCCTGCTAGATTTCTGGCAGCCTCCTGGGGGC AAGGCTGCCCTGACCCTGTCTGGGGCACAACCTGAGGATGAGGCTG AGTACTACTGTGCTCTCTGGTACAGCAACCACTGGGTGTTTGGGGG GGCACCAAATTGACAGTGTTGGGTGGGGGGGGCTCCTTTTGGGTGC TGGTGGTGGTGGGGGGGGTGCTGGCCTGCTACAGCCTGTTAGTAAC AGTGGCCTTTATCATCTTCTGGGTGAGAAGCTGA |
| SEQ ID NO: 17 | CD3ζ chain intracellular activation domain | RVKFSRSADAPAYQQGQNQLYNELNLGRREEYDVLDKRRGRDPEMGG KPQRRKNPQEGLYNELQKDKMAEAYSEIGMKGERRRGKGHDGLYQG LSTATKDTYDALHMQALPPR |
| SEQ ID NO: 18 | Hinge region of CD8α molecule | TTTPAPRPPTPAPTIASQPLSLRPEACRPAAGGAVHTRGLDFACD |
| SEQ ID NO: 21 | HuR9684M Amino acid sequence | MALPVTALLLPLALLLHAARPEVQLVESGGGLVQPGGSLRLSCAASGF TFSRYWMNWVRQAPGKGLEWVGEITPDSSTINYAPSLKGRFTISRDNA KNSLYLQMNSLRAEDTAVYYCARPYDYGAWFASWGQGTLVTVSSGG GGSGGGGSGGGGSGGGGSQAVVTQEPSLTVSPGGTVTLTCRSSTGAVT TSNYANWVQEKPDHLFTGLIGGTNKRAPGTPARFSGSLLGGKAALTLS GAQPEDEAEYYCALWYSNHWVFGGGTKLTVLESKYGPPCPPCPFWVL VVVGGVLACYSLLVTVAFIIFWVRSKRSRLLHSDYMNMTPRRPGPTRK HYQPYAPPRDFAAYRSRVKFSRSADAPAYQQGQNQLYNELNLGRREEY DVLDKRRGRDPEMGGKPRRKNPQEGLYNELQKDKMAEAYSEIGMKG ERRRGKGHDGLYQGLSTATKDTYDALHMQALPPR |
| SEQ ID NO: 22 | HuR9684M Nucleotide sequence | ATGGCCCTGCCTGTGACAGCCCTATTACTGCCCCTGGCCCTTCTGTTA CATGCTGCTAGACCTGAGGTTCAACTGGTGGAGTCTGGGGGGGCC TAGTGCAGCCTGGGGGCAGCCTGAGACTGAGCTGTGCTGCCTCTGG CTTCACCTTCAGCAGATACTGGATGAACTGGGTGAGACAAGCCCCT GGCAAGGGCCTGGAGTGGGTGGGGGAGATCACCCCTGACAGCAGC ACCATCAACTATGCCCCTAGCCTGAAGGGCAGATTCACCATCAGCAG AGACAATGCCAAGAACAGCCTGTACCTGCAGATGAACAGCCTGAGA GCTGAGGACACAGCTGTGTATTATTGTGCTAGACCATATGACTATGGG GCCTGGTTTGCCTCTTGGGGCCAAGGCACACTGGTTACAGTGAGTT CTGGGGGGGGGGTTCTGGAGGGGGGGATCTGGGGGTGGAGGTT CTGGGGGGGGGGCAGTCAAGCTGTGGTGACCCAAGAGCCTAGCC TGACAGTGTCCCCTGGGGGCACAGTCACCCTGACCTGCAGAAGCAG CACTGGGGCTGTGACCACAAGCAACTATGCCAACTGGGTGCAAGAG AAGCCTGACCACCTGTTCACTGGCCTGATTGGGGGCACCAACAAAA GAGCCCCTGGCACCCCTGCTAGATTCTCTGGAAGCCTGTTGGGGGG CAAGGCTGCCCTGACCCTATCTGGGGCACAGCCTGAGGATGAGGCT GAGTACTACTGTGCCCTCTGGTACAGCAACCACTGGGTGTTTGGGG GGGGCACCAAACTGACAGTGTTGGAGAGCAAGTATGGCCCCCCCTG TCCTCCCTGTCCCTTTTGGGTGCTGGTGGTTGTGGGGGGGTGCTGG CCTGCTACAGCCTGCTGGTGACAGTGGCCTTCATCATCTTCTGGGTG AGAAGCAAGAGAAGCAGACTGCTGCACTCTGACTACATGAACATGA CCCCTAGAAGACCTGGCCCCACAAGAAAGCACTATCAGCCCTATGC CCCCCCTAGAGACTTTGCTGCCTACAGAAGCAGAGTGAAGTTCAGC AGATCTGCTGATGCCCCTGCCTATCAGCAAGGGCAGAATCAGCTGTA TAATGAGCTCAACCTGGGCAGAAGAGAGGAGTATGATGTGCTGGAC AAGAGAAGAGGCAGAGACCCTGAGATGGGGGGCAAGCCTAGAAGA AAGAACCCCCAAGAGGGCCTGTACAATGAGCTGCAAAAGGACAAG ATGGCTGAGGCCTACTCTGAGATTGGCATGAAGGGGGAGAGAAGAA GAGGCAAGGGCCATGATGGCCTGTACCAAGGCCTGAGCACAGCCAC CAAGGACACCTATGATGCCCTACATATGCAAGCTCTGCCCCCTAGAT GA |
| SEQ ID NO: 23 | HuCD28HR9 68 Amino acid sequence | MALPVTALLLPLALLLHAARPEVQLVESGGGLVQPGGSLRLSCAASGF TFSRYWMNWVRQAPGKGLEWVGEITPDSSTINYAPSLKGRFTISRDNA KNSLYLQMNSLRAEDTAVYYCARPYDYGAWFASWGQGTLVTVSSGG GGSGGGGSGGGGSGGGGSQAVVTQEPSLTVSPGGTVTLTCRSSTGAVT TSNYANWVQEKPDHLFTGLIGGTNKRAPGTPARFSGSLLGGKAALTLS GAQPEDEAEYYCALWYSNHWVFGGGTKLTVLIEVMYPPPYLDNEKSN GTIIHVKGKHLCPSPLFPGPSKPFWVLVVVGGVLACYSLLVTVAFIIFWV RSKRSRLLHSDYMNMTPRRPGPTRKHYQPYAPPRDFAAYRSRVKFSRS ADAPAYQQGQNQLYNELNLGRREEYDVLDKRRGRDPEMGGKPRRKN PQEGLYNELQKDKMAEAYSEIGMKGERRRGKGHDGLYQGLSTATKDT YDALHMQALPPR |
| SEQ ID NO: 24 | HuCD28HR9 68 Nucleotide sequence | ATGGCACTTCCTGTGACAGCCTTGTTACTACCTCTGGCCCTTCTTCTC CATGCTGCTAGACCTGAGGTGCAGCTGGTGGAGTCTGGGGGGGCC TGGTACAACCTGGGGGCTCTCTGAGACTGAGCTGTGCTGCCTCTGG CTTCACCTTCAGCAGATATTGGATGAACTGGGTGAGACAAGCCCCTG GCAAGGGCCTGGAGTGGGTGGGGGAGATCACCCCTGACAGCAGCA CCATCAACTATGCCCCTAGCCTGAAGGGCAGATTCACCATCAGCAGA GACAATGCCAAGAACAGCCTGTACCTGCAGATGAACAGCCTGAGAG CTGAGGACACAGCTGTGTACTATTGTGCTAGACCCTATGACTATGGG GCCTGGTTTGCTAGCTGGGGCCAAGGCACCCTTGTGACAGTCTCATC TGGGGGAGGGGGCAGTGGGGGGGGGGGCAGTGGGGGGGGGGGT CTGGGGGAGGGGGAGCCAAGCTGTGGTGACTCAAGAGCCAAGCC TGACAGTGAGCCCTGGGGGCACTGTGACCCTGACCTGCAGAAGCA GCACTGGGGCTGTGACCACAAGCAACTATGCCAACTGGGTGCAAGA GAAGCCTGACCACCTGTTTACTGGCCTGATTGGGGGCACCAATAAA AGAGCCCCTGGCACCCCTGCTAGATTCTCTGGCAGCCTCCTGGGGG GCAAGGCTGCCCTGACTCTGAGTGGGGCTCAGCCTGAGGATGAGGC TGAGTACTACTGTGCTCTCTGGTACAGCAACCACTGGGTGTTTGGGG GGGGCACCAAGCTTACAGTGCTGATTGAGGTGATGTACCCCCCCCC TACCTGGACAATGAGAAAAGCAATGGCACCATCATCCATGTGAAGG GCAAGCACCTGTGCCCTAGCCCCCTGTTTCCTGGCCCTAGCAAACCC TTCTGGGTGCTAGTGGTGGTGGGGGGGGTGCTGGCCTGCTACAGCC TGTTAGTAACAGTGGCCTTTATCATCTTCTGGGTGAGAAGCAAGAGA AGCAGACTGCTGCACTCTGACTACATGAACATGACCCCTAGAAGAC CTGGCCCCACAAGAAAGCACTATCAGCCCTATGCCCCCCCTAGAGAC TTTGCTGCCTACAGAAGCAGAGTGAAGTTCAGCAGATCTGCTGATG CCCCTGCCTATCAGCAAGGGCAGAATCAGCTGTATAATGAGCTCAAC CTGGGCAGAAGAGAGGAGTATGATGTGCTGGACAAGAGAAGAGGC AGAGACCCTGAGATGGGGGGCAAGCCTAGAAGAAAGAACCCCCAA GAGGGCCTGTACAATGAGCTGCAAAAGGACAAGATGGCTGAGGCCT ACTCTGAGATTGGCATGAAGGGGGAGAGAAGAAGAGGCAAGGGCC ATGATGGCCTGTACCAAGGCCTGAGCACAGCCACCAAGGACACCTA TGATGCACTTCACATGCAAGCCCTGCCCCCTAGATGA |
| SEQ ID NO: 25 | HuR968B Amino acid sequence | MALPVTALLLPLALLLHAARPEVQLVESGGGLVQPGGSLRLSCAASGF TFSRYWMNWVRQAPGKGLEWVGEITPDSSTINYAPSLKGRFTISRDNA KNSLYLQMNSLRAEDTAVYYCARPYDYGAWFASWGQGTLVTVSSGG GGSGGGGSGGGGSGGGGSQAVVTQEPSLTVSPGGTVTLTCRSSTGAVT TSNYANWVQEKPDHLFTGLIGGTNKRAPGTPARFSGSLLGGKAALTLS GAQPEDEAEYYCALWYSNHWVFGGGTKLTVLGGGGSIYIWAPLAGTC GVLLLSLVITLYCKRGRKKLLYIFKQPFMRPVQTTQEEDGCSCRFPEEEE GGCELRVKFSRSADAPAYQQGQNQLYNELNLGRREEYDVLDKRRGRD PEMGGKPRRKNPQEGLYNELQKDKMAEAYSEIGMKGERRRGKGHDG LYQGLSTATKDTYDALHMQALPPR |
| SEQ ID NO: 26 | HuR968B Nucleotide sequence | ATGGCCCTGCCTGTGACAGCCCTACTACTGCCCCTGGCCCTTCTGCT TCATGCTGCTAGACCTGAGGTGCAGCTGGTGGAATCTGGGGGGGC TTGGTTCAGCCTGGGGGCAGCCTGAGACTGAGCTGTGCTGCCTCTG GCTTCACCTTCAGCAGATATTGGATGAACTGGGTGAGACAAGCCCCT GGCAAGGGCCTGGAGTGGGTGGGGGAGATCACCCCTGACAGCAGC ACCATCAACTATGCCCCTAGCCTGAAGGGCAGATTCACCATCAGCAG AGACAATGCCAAGAACAGCCTGTACCTGCAGATGAACAGCCTGAGA GCTGAGGACACAGCTGTGTACTATTGTGCTAGACCCTATGACTATGG GGCCTGGTTTGCTAGCTGGGGCCAAGGCACCCTAGTAACAGTGTCAT CTGGGGGGGAGGATCTGGGGGGGGGGTTCTGGGGGGGGGGCT CTGGTGGGGGGGGTTCTCAAGCTGTGGTAACACAAGAGCCTAGCCT GACAGTGAGCCCTGGGGGCACAGTGACCCTGACCTGCAGAAGCAG CACTGGGGCTGTGACCACAAGCAACTATGCCAACTGGGTGCAAGAG AAGCCTGACCACCTGTTCACTGGCCTGATTGGGGGCACCAATAAGA GAGCACCTGGCACTCCTGCTAGATTTTCTGGCTCACTGCTGGGGGC AAGGCTGCCTTGACCCTTTCTGGGGCTCAGCCTGAGGATGAGGCTG AGTACTACTGTGCTCTCTGGTACAGCAACCACTGGGTGTTTGGGGGG GGCACCAAGCTGACAGTGCTGGGGGGGGGGTAGCATCTACATCT GGGCCCCCCTGGCTGGCACATGTGGGGTGCTGCTGCTGAGCCTGGT GATCACCCTGTACTGCAAGAGAGGCAGAAAGAAGCTGCTGTACATC TTCAAGCAGCCCTTCATGAGACCTGTGCAGACCACCCAAGAGGAGG ATGGCTGCAGCTGCAGATTCCCTGAGGAGGAGGAGGGGGGCTGTGA GCTGAGAGTGAAGTTCAGCAGATCTGCTGATGCCCCTGCCTATCAGC AAGGGCAGAATCAGCTGTATAATGAGCTCAACCTGGGCAGAAGAGA GGAGTATGATGTGCTGGACAAGAGAAGAGGCAGAGACCCTGAGATG GGGGGCAAGCCTAGAAGAAAGAACCCCCAAGAGGGCCTGTACAAT GAGCTGCAAAAGGACAAGATGGCTGAGGCCTACTCTGAGATTGGCA TGAAGGGGGAGAGAAGAAGAGGCAAGGGCCATGATGGCCTGTACC AAGGCCTGAGCACAGCCACCAAGGACACTTATGATGCCTTGCACAT GCAAGCCCTGCCTCCAAGATGA |
| SEQ ID NO: 31 | 8E5 CAR Amino acid sequence | MALPVTALLLPLALLLHAARPDIVMTQSPDSLAVSLGERATINCKSSQS LFNSGNQKNYLTWYQQKPGQPPKLLIYWASTRESGVPDRFSGSGSGTD FTLTISSLQAEDVAVYYCQNAYSFPYTFGGGTKLEIKRGGGGSGGGGSG GGGSQVQLQESGPGLIKPSQTLSLTCTVSGGSISSGYNWHWIRQPPGKG LEWIGYIHYTGSTNYNPALRSRVTISVDTSKNQFSLKLSSVTAADTAIYY CARIYNGNSFPYWGQGTTVTVSSTTTPAPRPPTPAPTIASQPLSLRPEAC RPAAGGAVHTRGLDFACDFWVLVVVGGVLACYSLLVTVAFIIFWVRSK RSRLLHSDYMNMTPRRPGPTRKHYQPYAPPRDFAAYRSRVKFSRSADA PAYQQGQNQLYNELNLGRREEYDVLDKRRGRDPEMGGKPQRRKNPQ EGLYNELQKDKMAEAYSEIGMKGERRRGKGHDGLYQGLSTATKDTYD ALHMQALPPR |
| SEQ ID NO: 32 | CD16 CAR Amino acid sequence | MALPVTALLLPLALLLHAARPGMRTEDLPKAVVFLEPQWYRVLEKDS VTLKCQGAYSPEDNSTQWFHNESLISSQASSYFIDAATVDDSGEYRCQT NLSTLSDPVQLEVHIGWLLLQAPRWVFKEEDPIHLRCHSWKNTALHKV TYLQNGKGRKYFHHNSDFYIPKATLKDSGSYFCRGLVGSKNVSSETVN ITITQGLAVSTISSFFPPGYQFWVLVVVGGVLACYSLLVTVAFIIFWVRS KRSRLLHSDYMNMTPRRPGPTRKHYQPYAPPRDFAAYRSRVKFSRSAD APAYQQGQNQLYNELNLGRREEYDVLDKRRGRDPEMGGKPRRKNPQ EGLYNELQKDKMAEAYSEIGMKGERRRGKGHDGLYQGLSTATKDTYD ALHMQALPPR |
| SEQ ID NO: 33 | Blue21 CAR Amino acid sequence | MALPVTALLLPLALLLHAARPDIVLTQSPPSLAMSLGKRATISCRASESV TILGSHLIHWYQQKPGQPPTLLIQLASNVQTGVPARFSGSGSRTDFTLTI DPVEEDDVAVYYCLQSRTIPRTFGGGTKLEIKGSTSGSGKPGSGEGSTK GQIQLVQSGPELKKPGETVKISCKASGYTFTDYSINWVKRAPGKGLKW MGWINTETREPAYAYDFRGRFAFSLETSASTAYLQINNLKYEDTATYFC ALDYSYAMDYWGQGTSVTVSSAAATTTPAPRPPTPAPTIASQPLSLRPE ACRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAPA YQQGQNQLYNELNLGRREEYDVLDKRRGRDPEMGGKPRRKNPQEGL YNELQKDKMAEAYSEIGMKGERRRGKGHDGLYQGLSTATKDTYDAL HMQALPPR |
| SEQ ID NO: 34 | Amino acid sequence of HB37A6 VH | EVQLLDSGGGLVQPGGSLRLSCAASGFTFSSYVMSWVRQAPGKGLNW VSTISHSGGSTYYADSVKGRFTISRDNSKNTLYLQMNSLRAEDTAVYYC AIDAPYYDILTGYRYWGQGTLVTVSS |
| SEQ ID NO: 35 | HB37A6 VL Amino acid sequence | DIQMTQSPSTLSASVGDRVTITCRASQSISSWLAWYQQKPGKAPKLLIY KASSLESGVPSRFSGSGSGTEFTLTISSLQPDDFATYYCQQYNSYSYTFG QGTKLEIK |
| SEQ ID NO: 36 | Hz69H9 VH Amino acid sequence | QVQLVQSGAEVKKPGSSVKVSCKASGYSFSSYNIHWVRQAPGQGLEW MGYIAPFQGDSRYNQKFKGRVTITADKSTSTAYMELSSLRSEDTAVYYC ARLNRGNSLDYWGQGTLVTVSS |
| SEQ ID NO: 37 | Hz69H9 VL Amino acid sequence | DIVMTQSPDSLAVSLGERATINCKSSQSLFNSGNQRNYLTWYQQKPGQ PPKLLIYWASTRESGVPDRFSGSGSGTDFTLTISSLQAEDVAVYYCQNN YIYPLTFGGGTKVEIK |
| SEQ ID NO: 38 | Amino acid sequence of WT IgG1 heavy chain constant region | |
| SEQ ID NO: 39 | Amino acid sequence of P329G IgG1 heavy chain constant region | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQ DWLNGKEYKCKVSNKALGAPIEKTISKAKGQPREPQVYTLPPSRDELT KNQVSLTCLVKGFYPSDIAVEWESNGQPENNYKTTPPVLDSDGSFFLYS KLTVDKSRWQQGNVFSCSVMHEALHNHYTQKSLSLSPGK |
| SEQ ID NO: 40 | Amino acid sequence of κ light chain constant region | |
| SEQ ID NO: 41 | WT antibody (HB37A6) Amino acid sequence | EVQLLDSGGGLVQPGGSLRLSCAASGFTFSSYVMSWVRQAPGKGLNW VSTISHSGGSTYYADSVKGRFTISRDNSKNTLYLQMNSLRAEDTAVYYC AIDAPYYDILTGYRYWGQGTLVTVSSASTKGPSVFPLAPSSKSTSGGTA ALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHE ALHNHYTQKSLSLSPGK DIQMTQSPSTLSASVGDRVTITCRASQSISSWLAWYQQKPGKAPKLLIY KASSLESGVPSRFSGSGSGTEFTLTISSLQPDDFATYYCQQYNSYSYTFG QGTKLEIKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGEC |
| SEQ ID NO: 42 | Hz3G3 VH Amino acid sequence | QVQLVQSGAEVKKPGASVKVSCKASGYSFTTYWMHWVRQAPGQGLE WMGLIDPSDSETRLNQKFKDRVTMTVDTSTSTVYMELSSLRSEDTAVY YCASNRWLLGWGQGTLVTVSS |
| SEQ ID NO: 43 | Hz3G3 VL Amino acid sequence | DIVMTQSPDSLAVSLGERATINCKSSQSLLNSGNQKNYLTWYQQKPGQ PPKLLIYWASTRESGVPDRFSGSGSGTDFTLTISSLQAEDVAVYYCQND YSYPLTFGQGTKLEIK |
| SEQ ID NO: 44 | Hz69H9-1.2 VH Amino acid sequence | QVQLVQSGAEVKKPGSSVKVSCKASGYSFSSYNIHWVRQAPGQGLEW MGYIAPFQGDARYNQKFKGRVTITADKSTSTAYMELSSLRSEDTAVYY CARLNRGQSLDYWGQGTLVTVSS |
| SEQ ID NO: 45 | Hz69H9-1.2 VL Amino acid sequence | DIVMTQSPDSLAVSLGERATINCKSSQSLFNAGNQRNYLTWYQQKPGQ PPKLLIYWASTRESGVPDRFSGSGSGTDFTLTISSLQAEDVAVYYCQNN YIYPLTFGGGTKVEIK |
| SEQ ID NO: 46 | Hz69H9-2.1 VH Amino acid sequence | QVQLVQSGAEVKKPGSSVKVSCKASGYSFSSYNIHWVRQAPGQGLEW MGYIAPFQGDARYNQKFKGRVTITADKSTSTAYMELSSLRSEDTAVYY CARLNRGNALDYWGQGTLVTVSS |
| SEQ ID NO: 47 | Hz69H9-2.1 VL Amino acid sequence | DIVMTQSPDSLAVSLGERATINCKSSQSLFQSGNQRNYLTWYQQKPGQ PPKLLIYWASTRESGVPDRFSGSGSGTDFTLTISSLQAEDVAVYYCQNN YIYPLTFGGGTKVEIK |
| SEQ ID NO: 48 | Hz69H9-SA VH Amino acid sequence | QVQLVQSGAEVKKPGSSVKVSCKASGYSFSSYNISWVRQAPGQGLEW MGYIAPFQGDARYNQKFKGRVTITADKSTSTAYMELSSLRSEDTAVYY CARLNRGNSLDYWGQGTLVTVSS |
| SEQ ID NO: 49 | Hz69H9-SA VL Amino acid sequence | DIVMTQSPDSLAVSLGERATINCKSSQSLFNSGNQRNYLTWYQQKPGQ PPKLLIYWASTRESGVPDRFSGSGSGTDFTLTISSLQAEDVAVYYCQNN YIYPLTFGGGTKVEIK |
| SEQ ID NO: 50 | Zmab VH Amino acid sequence | QVQLQQPGAELVRPGASVKLSCKASGYTFTSYWINWVKQRPGQGLE WIGNIYPSDSYTNYNQKFKDKATLTVDKSSSTAYMQLSSPTSEDSAVYY CTRSWRGNSFDYWGQGTTLTVSS |
| SEQ ID NO: 51 | Zmab VL Amino acid sequence | DIQMTQSPSSLTVTAGEKVTMSCKSSQSLLNSGNQRNYLTWYQQIPGQ PPKLLIYWSSTRGSGVPDRFTGSGSGTDFTLTISSVQAEDLAVYFCQNV YYYPFTFGSGTRLEVK |
| SEQ ID NO: 52 | PG-Fc Amino acid sequence | EVQLVESGGGLVQPGGSLRLSCAASGFTFSRYWMNWVRQAPGKGLE WVGEITPDSSTINYAPSLKGRFTISRDNAKNSLYLQMNSLRAEDTAVYY CARPYDYGAWFASWGQGTLVTVSSGGGGSGGGGSGGGGSGGGGSQA VVTQEPSLTVSPGGTVTLTCRSSTGAVTTSNYANWVQEKPDHLFTGLIG GTNKRAPGTPARFSGSLLGGKAALTLSGAQPEDEAEYYCALWYSNHW VFGGGTKLTVLESKYGPPCPPCPAPELLGGPSVFLFPPKPKDTLMISRTP EVTCVVVDVSHEDPEVKFNWYVDGVEVHNAKTKPREEQYNSTYRVV SVLTVLHQDWLNGKEYKCKVSNKALPAPIEKTISKAKGQPREPQVYTL PPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK |
| SEQ ID NO: 53 | CH2-PG Amino acid sequence | MALPVTALLLPLALLLHAARPAPELLGGPSVFLFPPKPKDTLMISRTPEV TCVVVDVSHEDPEVKFNWYVDGVEVHNAKTKPREEQYNSTYRVVSV LTVLHQDWLNGKEYKCKVSNKALGAPIEKTISKAKGGGGSGGGGSGG GGSGGGGSIYIWAPLAGTCGVLLLSLVITLYC |
| SEQ ID NO: 54 | Anti-PG antibody CDRH1 | RYWMN |
| SEQ ID NO: 55 | Anti-PG antibody CDR H2 | EITPDSSTINYAPSLKG |
| SEQ ID NO: 56 | Anti-PG antibody CDR H3 | PYDYGAWFAS |
| SEQ ID NO: 57 | Anti-PG antibody CDR L1 | RSSTGAVTTSNYAN |
| SEQ ID NO: 58 | Anti-PG antibody CDR L2 | GTNKRAP |
| SEQ ID NO: 59 | Anti-PG antibody CDR L3 | ALWYSNHWV |
| SEQ ID NO: 60 | HB37A6 CDR H1 | SYVMS |
| SEQ ID NO: 61 | HB37A6 CDR H2 | TISHSGGSTYYADSVKG |
| SEQ ID NO: 62 | HB37A6 CDR H3 | DAPYYDILTGYRY |
| SEQ ID NO: 63 | HB37A6 CDR L1 | RASQSISSWLA |
| SEQ ID NO: 64 | HB37A6 CDR L2 | KASSLES |
| SEQ ID NO: 65 | HB37A6 CDR L3 | QQYNSYSYT |
| SEQ ID NO: 66 | Hz69H9 CDR H1 | SYNIH |
| SEQ ID NO: 67 | Hz69H9 CDR H2 | YIAPFQGDSRYNQKFKG |
| SEQ ID NO: 68 | Hz69H9 CDR H3 | LNRGNSLDY |
| SEQ ID NO: 69 | Hz69H9 CDR L1 | KSSQSLFNSGNQRNYLT |
| SEQ ID NO: 70 | Hz69H9 CDR L2 | WASTRES |
| SEQ ID NO: 71 | Hz69H9 CDR L3 | QNNYIYPLT |
| SEQ ID NO: 72 | Hz69H9-1.2 CDR H1 | SYNIH |
| SEQ ID NO: 73 | Hz69H9-1.2 CDR H2 | YIAPFQGDARYNQKFKG |
| SEQ ID NO: 74 | Hz69H9-1.2 CDR H3 | LNRGQSLDY |
| SEQ ID NO: 75 | Hz69H9-1.2 CDR L1 | KSSQSLFNAGNQRNYLT |
| SEQ ID NO: 76 | Hz69H9-1.2 CDR L2 | WASTRES |
| SEQ ID NO: 77 | Hz69H9-1.2 CDR L3 | QNNYIYPLT |
| SEQ ID NO: 78 | Hz69H9-2.1 CDR H1 | SYNIH |
| SEQ ID NO: 79 | Hz69H9-2.1 CDR H2 | YIAPFQGDARYNQKFKG |
| SEQ ID NO: 80 | Hz69H9-2.1 CDR H3 | LNRGNALDY |
| SEQ ID NO: 81 | Hz69H9-2.1 CDR L1 | KSSQSLFQSGNQRNYLT |
| SEQ ID NO: 82 | Hz69H9-2.1 CDR L2 | WASTRES |
| SEQ ID NO: 83 | Hz69H9-2.1 CDR L3 | QNNYIYPLT |
| SEQ ID NO: 84 | Hz69H9-SA CDR H1 | SYNIS |
| SEQ ID NO: 85 | Hz69H9-SA CDR H2 | YIAPFQGDARYNQKFKG |
| SEQ ID NO: 86 | Hz69H9-SA CDR H3 | LNRGNSLDY |
| SEQ ID NO: 87 | Hz69H9-SA CDR L1 | KSSQSLFNSGNQRNYLT |
| SEQ ID NO: 88 | Hz69H9-SA CDR L2 | WASTRES |
| SEQ ID NO: 89 | Hz69H9-SA CDR L3 | QNNYIYPLT |
| SEQ ID NO: 90 | Hz3G3 CDR H1 | TYWMH |
| SEQ ID NO: 91 | Hz3G3 CDR H2 | LIDPSDSETRLNQKFKD |
| SEQ ID NO: 92 | Hz3G3 CDR H3 | NRWLLG |
| SEQ ID NO: 93 | Hz3G3 CDR L1 | KSSQSLLNSGNQKNYLT |
| SEQ ID NO: 94 | Hz3G3 CDR L2 | WASTRES |
| SEQ ID NO: 95 | Hz3G3 CDR L3 | QNDYSYPLT |

## Claims

1. A molecular switch-regulated chimeric antigen receptor (CAR) polypeptide, comprising:
(1) a humanized anti-P329G mutation scFv sequence, wherein the scFv sequence comprises the following sequences that are capable of specifically binding to an antibody Fc domain comprising a P329G mutation, but not capable of specifically binding to an unmutated parent antibody Fc domain:
(i) a heavy chain variable region, which comprises, according to the Kabat numbering,
(a) a heavy chain complementarity determining region CDR H1 shown in the amino acid sequence RYWMN (SEQ ID NO: 54), or a variant of the CDR H1 with no more than 2 amino acid changes or no more than 1 amino acid change;
(b) a CDR H2 shown in the amino acid sequence EITPDSSTINYAPSLKG (SEQ ID NO: 55), or a variant of the CDR H2 with no more than 2 amino acid changes or no more than 1 amino acid change; and
(c) a CDR H3 shown in the amino acid sequence PYDYGAWFAS (SEQ ID NO: 56), or a variant of the CDR H3 with no more than 2 amino acid changes or no more than 1 amino acid change; and
(ii) a light chain variable region, which comprises, according to the Kabat numbering,
(d) a light chain complementarity determining region (CDR L) 1 shown in the amino acid sequence RSSTGAVTTSNYAN (SEQ ID NO: 57), or a variant of the CDR L1 with no more than 2 amino acid changes or no more than 1 amino acid change;
(e) a CDR L2 shown in the amino acid sequence GTNKRAP (SEQ ID NO: 58), or a variant of the CDR L2 with no more than 2 amino acid changes or no more than 1 amino acid change; and
(f) a CDR L3 shown in the amino acid sequence ALWYSNHWV (SEQ ID NO: 59), or a variant of the CDR L3 with no more than 2 amino acid changes or no more than 1 amino acid change;
wherein the amino acid change is an addition, deletion or substitution of an amino acid;
(2) a hinge region/spacer region selected from:
(i) a (G₄S)ₙ, (SG₄)ₙ or G₄(SG₄)ₙ peptide linker, wherein "n" is an integer of 1 to 10, such as an integer of 2 to 4, for example, the sequences set forth in SEQ ID NO: 4 and SEQ ID NO: 5;
(ii) an IgG4 spacer region (SEQ ID NO: 6; ESKYGPPCPPCP), or a spacer region having at least 80% sequence identity thereto; and
(iii) a CD28 hinge region (SEQ ID NO: 7; IEVMYPPPYLDNEKSNGTIIHVKGKHLCPSPLFPGPSKP), or a spacer region having at least 80% sequence identity thereto;
(3) a transmembrane region (TM) selected from:
(i) a CD28 transmembrane domain or a variant thereof with 1-5 amino acid modifications, for example, the sequence set forth in SEQ ID NO: 8 or a variant thereof with 1-2 amino acid modifications; and
(ii) a CD8 transmembrane domain or a variant thereof with 1-5 amino acid modifications, for example, the sequence set forth in SEQ ID NO: 9 or a variant thereof with 1-2 amino acid modifications;
(4) a co-stimulatory signaling domain (CSD) selected from:
(i) a 4-1BB co-stimulatory domain or a variant thereof with 1-5 amino acid modifications, for example, the sequence set forth in SEQ ID NO: 11 or a variant thereof with 1-2 amino acid modifications; and
(ii) a CD28 co-stimulatory domain or a variant thereof with 1-5 amino acid modifications, for example, the sequence set forth in SEQ ID NO: 10 or a variant thereof with 1-2 amino acid modifications, e.g., the sequence set forth in SEQ ID NO: 12; and
(5) a stimulatory signaling domain (SSD), which is a CD3ζ signaling domain or a variant thereof with 1-10 amino acid modifications, for example, the sequence set forth in SEQ ID NO: 13 or a variant thereof with 1-10 or 1-5 amino acid modifications;
wherein preferably, the CAR polypeptide comprises:
(1) a humanized anti-P329G mutation scFv sequence, wherein the scFv sequence comprises the following sequences that are capable of specifically binding to an antibody Fc domain comprising a P329G mutation, but not capable of specifically binding to an unmutated parent antibody Fc domain:
(i) a heavy chain variable region, which comprises, according to the Kabat numbering,
(a) a CDR H1 shown in the amino acid sequence RYWMN (SEQ ID NO: 54);
(b) a CDR H2 shown in the amino acid sequence EITPDSSTINYAPSLKG (SEQ ID NO: 55); and
(c) a CDR H3 shown in the amino acid sequence PYDYGAWFAS (SEQ ID NO: 56); and
(ii) a light chain variable region, which comprises, according to the Kabat numbering,
(d) a CDR L1 shown in the amino acid sequence RSSTGAVTTSNYAN (SEQ ID NO: 57);
(e) a CDR L2 shown in the amino acid sequence GTNKRAP (SEQ ID NO: 58); and
(f) a CDR L3 shown in the amino acid sequence ALWYSNHWV (SEQ ID NO: 59);
for example, (i) a heavy chain variable region comprising the sequence of SEQ ID NO: 2 or a sequence having at least 90%, 91%, 92%, 93%, 94%, 95%, 96%, 97%, 98% or 99% identity thereto, and
(ii) a light chain variable region comprising the sequence of SEQ ID NO: 3 or a sequence having at least 90%, 91%, 92%, 93%, 94%, 95%, 96%, 97%, 98% or 99% identity thereto;
for example, (i) a heavy chain variable region comprising the sequence of SEQ ID NO: 2, and
(ii) a light chain variable region comprising the sequence of SEQ ID NO: 3;
(2) a hinge region/spacer region selected from:
(i) a (G₄S)ₙ, (SG₄)ₙ, (G₄S)ₙ or G₄(SG₄)ₙ peptide linker, wherein "n" is an integer of 1 to 10, such as an integer of 2 to 4, for example, the sequences set forth in SEQ ID NO: 4 and SEQ ID NO: 5;
(ii) an IgG4 spacer region (SEQ ID NO: 6; ESKYGPPCPPCP), or a spacer region having at least 90% sequence identity thereto; and
(iii) a CD28 hinge region (SEQ ID NO: 7; IEVMYPPPYLDNEKSNGTIIHVKGKHLCPSPLFPGPSKP), or a spacer region having at least 90% or at least 95% sequence identity thereto;
(3) a transmembrane region (TM) selected from:
(i) a CD28 transmembrane domain set forth in SEQ ID NO: 8 or a variant thereof with 1 amino acid modification; and
(ii) a CD8 transmembrane domain set forth in SEQ ID NO: 9 or a variant thereof with 1 amino acid modification;
(4) a co-stimulatory signaling domain (CSD) selected from:
(i) a 4-1BB co-stimulatory domain set forth in SEQ ID NO: 11 or a variant thereof with 1 amino acid modification; and
(ii) a CD28 co-stimulatory domain set forth in SEQ ID NO: 10 or a variant thereof with 1 amino acid modification, for example, the sequence set forth in SEQ ID NO: 12; and
(5) a stimulatory signaling domain (SSD), which is a CD3ζ signaling domain set forth in SEQ ID NO: 13 or a variant thereof with 1 amino acid modification;
wherein the amino acid modification is an addition, deletion or substitution of an amino acid.

2. The molecular switch-regulated CAR polypeptide according to claim 1, further comprising a signal peptide sequence located at the N-terminus, for example, the signal peptide sequence set forth in SEQ ID NO: 1,
wherein preferably, the molecular switch-regulated CAR polypeptide has the amino acid sequence set forth in SEQ ID NO: 21, 23 or 25.

3. The molecular switch-regulated CAR polypeptide according to claim 1 or 2, further comprising a membrane-proximal intracellular domain located between the transmembrane region (TM) and the co-stimulatory signaling domain (CSD), for example, a CD3ε chain membrane-proximal intracellular signaling domain, such as a CD3ε chain membrane-proximal intracellular signaling domain as set forth in SEQ ID NO: 14.

4. A nucleic acid molecule encoding the molecular switch-regulated CAR polypeptide according to any one of claims 1-3, wherein preferably, the nucleic acid molecule comprises the nucleotide sequence set forth in SEQ ID NO: 22, 24 or 26.

5. A vector, comprising the nucleic acid molecule according to claim 4, wherein, for example, the vector is selected from a DNA vector, an RNA vector, a plasmid, a lentiviral vector, an adenoviral vector, or a retroviral vector.

6. A cell, comprising the CAR polypeptide according to any one of claims 1-3, the nucleic acid according to claim 4, or the vector according to claim 5, wherein the cell is, for example, an immune effector cell; for example, the immune effector cell is a T cell; for example, the T cell is an autologous T cell or an allogeneic T cell; for example, the immune effector cell is prepared from a T cell isolated from a human PBMC.

7. A method for preparing the cell according to claim 6, comprising introducing the cell with the vector according to claim 5, for example, isolating a T cell from a human PBMC and transducing the isolated T cell with the vector according to claim 5.

8. A pharmaceutical combination, comprising:
(i) an immune effector cell (e.g., a T cell) expressing the molecular switch-regulated CAR polypeptide according to any one of claims 1-3, a nucleic acid molecule encoding the molecular switch-regulated CAR polypeptide according to any one of claims 1-3, the vector according to claim 5, or any combination thereof; and
(ii) an antibody or an antigen-binding fragment specifically binding to a CLDN18.2 molecule and comprising a P329G mutation (also referred to as a P329G mutated antibody), wherein, for example, the P329G mutated antibody comprises a mutated Fc domain, wherein the amino acid at position P329 according to the EU numbering is mutated to glycine (G), and the binding of the mutated Fc domain to an Fcy receptor is reduced compared to the binding of an unmutated parent antibody Fc domain to the Fcy receptor;
as well as, optionally, a pharmaceutically acceptable supplementary material.

9. The pharmaceutical combination according to claim 8, wherein the antibody or the antigen-binding fragment specifically binding to the CLDN18.2 molecule comprises a heavy chain variable region and a light chain variable region, wherein
(a) the heavy chain variable region comprises, according to the Kabat numbering, a CDR H1 shown in the amino acid sequence SYVMS (SEQ ID NO: 60), or a variant of the CDR H1 with no more than 2 amino acid changes or no more than 1 amino acid change; a CDR H2 shown in the amino acid sequence TISHSGGSTYYADSVKG (SEQ ID NO: 61), or a variant of the CDR H2 with no more than 2 amino acid changes or no more than 1 amino acid change; and a CDR H3 shown in the amino acid sequence DAPYYDILTGYRY (SEQ ID NO: 62), or a variant of the CDR H3 with no more than 2 amino acid changes or no more than 1 amino acid change; and the light chain variable region comprises, according to the Kabat numbering, a CDR L1 shown in the amino acid sequence RASQSISSWLA (SEQ ID NO: 63), or a variant of the CDR L1 with no more than 2 amino acid changes or no more than 1 amino acid change; a CDR L2 shown in the amino acid sequence KASSLES (SEQ ID NO: 64), or a variant of the CDR L2 with no more than 2 amino acid changes or no more than 1 amino acid change; and a CDR L3 shown in the amino acid sequence QQYNSYSYT (SEQ ID NO: 65), or a variant of the CDR L3 with no more than 2 amino acid changes or no more than 1 amino acid change;
(b) the heavy chain variable region comprises, according to the Kabat numbering, a CDR H1 shown in the amino acid sequence SYNIH (SEQ ID NO: 66), or a variant of the CDR H1 with no more than 2 amino acid changes or no more than 1 amino acid change; a CDR H2 shown in the amino acid sequence YIAPFQGDSRYNQKFKG (SEQ ID NO: 67), or a variant of the CDR H2 with no more than 2 amino acid changes or no more than 1 amino acid change; and a CDR H3 shown in the amino acid sequence LNRGNSLDY (SEQ ID NO: 68), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 69), or a variant of the CDR L1 with no more than 2 amino acid changes or no more than 1 amino acid change; a CDR L2 shown in the amino acid sequence WASTRES (SEQ ID NO: 70), or a variant of the CDR L2 with no more than 2 amino acid changes or no more than 1 amino acid change; and a CDR L3 shown in the amino acid sequence QNNYIYPLT (SEQ ID NO: 71), or a variant of the CDR L3 with no more than 2 amino acid changes or no more than 1 amino acid change;
(c) the heavy chain variable region comprises, according to the Kabat numbering, a CDR H1 shown in the amino acid sequence SYNIH (SEQ ID NO: 72), or a variant of the CDR H1 with no more than 2 amino acid changes or no more than 1 amino acid change; a CDR H2 shown in the amino acid sequence YIAPFQGDARYNQKFKG (SEQ ID NO: 73), or a variant of the CDR H2 with no more than 2 amino acid changes or no more than 1 amino acid change; and a CDR H3 shown in the amino acid sequence LNRGQSLDY (SEQ ID NO: 74), or a variant of the CDR H3 with no more than 2 amino acid changes or no more than 1 amino acid change; and the light chain variable region comprises, according to the Kabat numbering, a CDR L1 shown in the amino acid sequence KSSQSLFNAGNQRNYLT (SEQ ID NO: 75), or a variant of the CDR L1 with no more than 2 amino acid changes or no more than 1 amino acid change; a CDR L2 shown in the amino acid sequence WASTRES (SEQ ID NO: 76), or a variant of the CDR L2 with no more than 2 amino acid changes or no more than 1 amino acid change; and a CDR L3 shown in the amino acid sequence QNNYIYPLT (SEQ ID NO: 77), or a variant of the CDR L3 with no more than 2 amino acid changes or no more than 1 amino acid change;
(d) the heavy chain variable region comprises, according to the Kabat numbering, a CDR H1 shown in the amino acid sequence SYNIH (SEQ ID NO: 78), or a variant of the CDR H1 with no more than 2 amino acid changes or no more than 1 amino acid change; a CDR H2 shown in the amino acid sequence YIAPFQGDARYNQKFKG (SEQ ID NO: 79), or a variant of the CDR H2 with no more than 2 amino acid changes or no more than 1 amino acid change; and a CDR H3 shown in the amino acid sequence LNRGNALDY (SEQ ID NO: 80), or a variant of the CDR H3 with no more than 2 amino acid changes or no more than 1 amino acid change; and the light chain variable region comprises, according to the Kabat numbering, a CDR L1 shown in the amino acid sequence KSSQSLFQSGNQRNYLT (SEQ ID NO: 81), or a variant of the CDR L1 with no more than 2 amino acid changes or no more than 1 amino acid change; a CDR L2 shown in the amino acid sequence WASTRES (SEQ ID NO: 82), or a variant of the CDR L2 with no more than 2 amino acid changes or no more than 1 amino acid change; and a CDR L3 shown in the amino acid sequence QNNYIYPLT (SEQ ID NO: 83), or a variant of the CDR L3 with no more than 2 amino acid changes or no more than 1 amino acid change;
(e) the heavy chain variable region comprises, according to the Kabat numbering, a CDR H1 shown in the amino acid sequence SYNIS (SEQ ID NO: 84), or a variant of the CDR H1 with no more than 2 amino acid changes or no more than 1 amino acid change; a CDR H2 shown in the amino acid sequence YIAPFQGDARYNQKFKG (SEQ ID NO: 85), or a variant of the CDR H2 with no more than 2 amino acid changes or no more than 1 amino acid change; and a CDR H3 shown in the amino acid sequence LNRGNSLDY (SEQ ID NO: 86), or a variant of the CDR H3 with no more than 2 amino acid changes or no more than 1 amino acid change; and the light chain variable region comprises, according to the Kabat numbering, a CDR L1 shown in the amino acid sequence KSSQSLFNSGNQRNYLT (SEQ ID NO: 87), or a variant of the CDR L1 with no more than 2 amino acid changes or no more than 1 amino acid change; a CDR L2 shown in the amino acid sequence WASTRES (SEQ ID NO: 88), or a variant of the CDR L2 with no more than 2 amino acid changes or no more than 1 amino acid change; and a CDR L3 shown in the amino acid sequence QNNYIYPLT (SEQ ID NO: 89), or a variant of the CDR L3 with no more than 2 amino acid changes or no more than 1 amino acid change; or
(f) the heavy chain variable region comprises, according to the Kabat numbering, a CDR H1 shown in the amino acid sequence TYWMH (SEQ ID NO: 90), or a variant of the CDR H1 with no more than 2 amino acid changes or no more than 1 amino acid change; a CDR H2 shown in the amino acid sequence LIDPSDSETRLNQKFKD (SEQ ID NO: 91), or a variant of the CDR H2 with no more than 2 amino acid changes or no more than 1 amino acid change; and a CDR H3 shown in the amino acid sequence NRWLLG (SEQ ID NO: 92), or a variant of the CDR H3 with no more than 2 amino acid changes or no more than 1 amino acid change; and the light chain variable region comprises, according to the Kabat numbering, a CDR L1 shown in the amino acid sequence KSSQSLLNSGNQKNYLT (SEQ ID NO: 93), or a variant of the CDR L1 with no more than 2 amino acid changes or no more than 1 amino acid change; a CDR L2 shown in the amino acid sequence WASTRES (SEQ ID NO: 94), or a variant of the CDR L2 with no more than 2 amino acid changes or no more than 1 amino acid change; and a CDR L3 shown in the amino acid sequence QNDYSYPLT (SEQ ID NO: 95), or a variant of the CDR L3 with no more than 2 amino acid changes or no more than 1 amino acid change;
wherein the amino acid change is an addition, deletion or substitution of an amino acid;
for example,
(a) the heavy chain variable region comprises, according to the Kabat numbering, a CDR H1 shown in the amino acid sequence SYVMS (SEQ ID NO: 60); a CDR H2 shown in the amino acid sequence TISHSGGSTYYADSVKG (SEQ ID NO: 61); and a CDR H3 shown in the amino acid sequence DAPYYDILTGYRY (SEQ ID NO: 62); and the light chain variable region comprises, according to the Kabat numbering, a CDR L1 shown in the amino acid sequence RASQSISSWLA (SEQ ID NO: 63); a CDR L2 shown in the amino acid sequence KASSLES (SEQ ID NO: 64); and a CDR L3 shown in the amino acid sequence QQYNSYSYT (SEQ ID NO: 65);
(b) the heavy chain variable region comprises, according to the Kabat numbering, a CDR H1 shown in the amino acid sequence SYNIH (SEQ ID NO: 66); a CDR H2 shown in the amino acid sequence YIAPFQGDSRYNQKFKG (SEQ ID NO: 67); and a CDR H3 shown in the amino acid sequence LNRGNSLDY (SEQ ID NO: 68); and the light chain variable region comprises, according to the Kabat numbering, a CDR L1 shown in the amino acid sequence KSSQSLFNSGNQRNYLT (SEQ ID NO: 69); a CDR L2 shown in the amino acid sequence WASTRES (SEQ ID NO: 70); and a CDR L3 shown in the amino acid sequence QNNYIYPLT (SEQ ID NO: 71);
(c) the heavy chain variable region comprises, according to the Kabat numbering, a CDR H1 shown in the amino acid sequence SYNIH (SEQ ID NO: 72); a CDR H2 shown in the amino acid sequence YIAPFQGDARYNQKFKG (SEQ ID NO: 73); and a CDR H3 shown in the amino acid sequence LNRGQSLDY (SEQ ID NO: 74); and the light chain variable region comprises, according to the Kabat numbering, a CDR L1 shown in the amino acid sequence KSSQSLFNAGNQRNYLT (SEQ ID NO: 75); a CDR L2 shown in the amino acid sequence WASTRES (SEQ ID NO: 76); and a CDR L3 shown in the amino acid sequence QNNYIYPLT (SEQ ID NO: 77);
(d) the heavy chain variable region comprises, according to the Kabat numbering, a CDR H1 shown in the amino acid sequence SYNIH (SEQ ID NO: 78); a CDR H2 shown in the amino acid sequence YIAPFQGDARYNQKFKG (SEQ ID NO: 79); and a CDR H3 shown in the amino acid sequence LNRGNALDY (SEQ ID NO: 80); and the light chain variable region comprises, according to the Kabat numbering, a CDR L1 shown in the amino acid sequence KSSQSLFQSGNQRNYLT (SEQ ID NO: 81); a CDR L2 shown in the amino acid sequence WASTRES (SEQ ID NO: 82); and a CDR L3 shown in the amino acid sequence QNNYIYPLT (SEQ ID NO: 83);
(e) the heavy chain variable region comprises, according to the Kabat numbering, a CDR H1 shown in the amino acid sequence SYNIS (SEQ ID NO: 84); a CDR H2 shown in the amino acid sequence YIAPFQGDARYNQKFKG (SEQ ID NO: 85); and a CDR H3 shown in the amino acid sequence LNRGNSLDY (SEQ ID NO: 86); and the light chain variable region comprises, according to the Kabat numbering, a CDR L1 shown in the amino acid sequence KSSQSLFNSGNQRNYLT (SEQ ID NO: 87); a CDR L2 shown in the amino acid sequence WASTRES (SEQ ID NO: 88); and a CDR L3 shown in the amino acid sequence QNNYIYPLT (SEQ ID NO: 89); or
(f) the heavy chain variable region comprises, according to the Kabat numbering, a CDR H1 shown in the amino acid sequence TYWMH (SEQ ID NO: 90); a CDR H2 shown in the amino acid sequence LIDPSDSETRLNQKFKD (SEQ ID NO: 91); and a CDR H3 shown in the amino acid sequence NRWLLG (SEQ ID NO: 92); and the light chain variable region comprises, according to the Kabat numbering, a CDR L1 shown in the amino acid sequence KSSQSLLNSGNQKNYLT (SEQ ID NO: 93); a CDR L2 shown in the amino acid sequence WASTRES (SEQ ID NO: 94); and a CDR L3 shown in the amino acid sequence QNDYSYPLT (SEQ ID NO: 95);
for example,
(a) the heavy chain variable region comprises the sequence of SEQ ID NO: 34 or a sequence having at least 90%, 91%, 92%, 93%, 94%, 95%, 96%, 97%, 98% or 99% identity thereto, and the light chain variable region comprises the sequence of SEQ ID NO: 35 or a sequence having at least 90%, 91%, 92%, 93%, 94%, 95%, 96%, 97%, 98% or 99% identity thereto;
(b) the heavy chain variable region comprises the sequence of SEQ ID NO: 36 or a sequence having at least 90%, 91%, 92%, 93%, 94%, 95%, 96%, 97%, 98% or 99% identity thereto, and the light chain variable region comprises the sequence of SEQ ID NO: 37 or a sequence having at least 90%, 91%, 92%, 93%, 94%, 95%, 96%, 97%, 98% or 99% identity thereto;
(c) the heavy chain variable region comprises the sequence of SEQ ID NO: 44 or a sequence having at least 90%, 91%, 92%, 93%, 94%, 95%, 96%, 97%, 98% or 99% identity thereto, and the light chain variable region comprises the sequence of SEQ ID NO: 45 or a sequence having at least 90%, 91%, 92%, 93%, 94%, 95%, 96%, 97%, 98% or 99% identity thereto;
(d) the heavy chain variable region comprises the sequence of SEQ ID NO: 46 or a sequence having at least 90%, 91%, 92%, 93%, 94%, 95%, 96%, 97%, 98% or 99% identity thereto, and the light chain variable region comprises the sequence of SEQ ID NO: 47 or a sequence having at least 90%, 91%, 92%, 93%, 94%, 95%, 96%, 97%, 98% or 99% identity thereto;
(e) the heavy chain variable region comprises the sequence of SEQ ID NO: 48 or a sequence having at least 90%, 91%, 92%, 93%, 94%, 95%, 96%, 97%, 98% or 99% identity thereto, and the light chain variable region comprises the sequence of SEQ ID NO: 49 or a sequence having at least 90%, 91%, 92%, 93%, 94%, 95%, 96%, 97%, 98% or 99% identity thereto; or
(f) the heavy chain variable region comprises the sequence of SEQ ID NO: 42 or a sequence having at least 90%, 91%, 92%, 93%, 94%, 95%, 96%, 97%, 98% or 99% identity thereto, and the light chain variable region comprises the sequence of SEQ ID NO: 43 or a sequence having at least 90%, 91%, 92%, 93%, 94%, 95%, 96%, 97%, 98% or 99% identity thereto;
for example,
(a) the heavy chain variable region comprises the sequence of SEQ ID NO: 34, and the light chain variable region comprises the sequence of SEQ ID NO: 35;
(b) the heavy chain variable region comprises the sequence of SEQ ID NO: 36, and the light chain variable region comprises the sequence of SEQ ID NO: 37;
(c) the heavy chain variable region comprises the sequence of SEQ ID NO: 44, and the light chain variable region comprises the sequence of SEQ ID NO: 45;
(d) the heavy chain variable region comprises the sequence of SEQ ID NO: 46, and the light chain variable region comprises the sequence of SEQ ID NO: 47;
(e) the heavy chain variable region comprises the sequence of SEQ ID NO: 48, and the light chain variable region comprises the sequence of SEQ ID NO: 49; or
(f) the heavy chain variable region comprises the sequence of SEQ ID NO: 42, and the light chain variable region comprises the sequence of SEQ ID NO: 43;
for example, the antibody is an IgG1, IgG2, IgG3 or IgG4 antibody, preferably an IgG1 or IgG4 antibody, more preferably an IgG1 antibody;
for example, the antigen-binding fragment is a Fab, a Fab', a F(ab')₂, an Fv, a single-chain Fv, a single-chain Fab, or a diabody.

10. The pharmaceutical combination according to claim 8 or 9, wherein the mutated Fc domain is a mutated Fc domain of an IgG1, IgG2, IgG3 or IgG4 antibody, preferably a mutated Fc domain of an IgG1 or IgG4 antibody, more preferably a mutated Fc domain of an IgG1 antibody;
for example, the antibody or the antigen-binding fragment comprises the heavy chain constant region sequence set forth in SEQ ID NO: 39 or a sequence having at least 90%, 91%, 92%, 93%, 94%, 95%, 96%, 97%, 98% or 99% identity thereto, wherein the amino acid at position P329 according to the EU numbering is mutated to G;
for example, the antibody or the antigen-binding fragment comprises the heavy chain constant region sequence set forth in SEQ ID NO: 39 or a sequence having at least 90%, 91%, 92%, 93%, 94%, 95%, 96%, 97%, 98% or 99% identity thereto, wherein the amino acid at position P329 according to the EU numbering is mutated to G; and the light chain constant region sequence set forth in SEQ ID NO: 40 or a sequence having at least 90%, 91%, 92%, 93%, 94%, 95%, 96%, 97%, 98%, or 99% identity thereto.

11. The pharmaceutical combination according to any one of claims 8-10, wherein
(i) the immune effector cell is a T cell expressing the molecular switch-regulated CAR polypeptide according to any one of claims 1-3 prepared from an autologous T cell or an allogeneic T cell, for example, the immune effector cell is a T cell expressing the molecular switch-regulated CAR polypeptide according to any one of claims 1-3 prepared from a T cell isolated from a human PBMC;
(ii) the P329G mutated antibody is an HB37A6 PG Ab, an Hz69H9 PG Ab, an Hz69H9-1.2 PG Ab, an Hz69H9-2.1 PG Ab, an Hz69H9-SA PG Ab, and/or an Hz3G3 PG Ab.

12. The pharmaceutical combination according to any one of claims 8-11, wherein
(i) is administered intravenously at a dose of 1 × 10⁶-1 × 10¹² immune effector cells, preferably 5 × 10⁶-1 × 10¹¹ immune effector cells, more preferably 1 × 10⁷-1 × 10¹⁰ immune effector cells, such as 5 × 10⁷ immune effector cells, 2.5 × 10⁸ immune effector cells, 7.5 × 10⁸ immune effector cells, or 1.25 × 10⁹ immune effector cells, either in a single administration or in multiple administrations; and
(ii) is administered in the form of a dose unit of 0.1-10 mg/kg, preferably 0.3 mg/kg, 0.5 mg/kg, 1 mg/kg, 3 mg/kg, 5 mg/kg, 7 mg/kg, or 9 mg/kg, preferably as a parenteral dosage form, more preferably as an intravenous dosage form.

13. The pharmaceutical combination according to any one of claims 8-12, wherein (i) and (ii) are administered separately, simultaneously, or sequentially; for example, (i) is administered intravenously on the first day, (ii) is administered on the second day, and then (ii) is administered multiple times at a certain frequency, while an *in-vivo* concentration of (i) and a desired therapeutic efficacy endpoint are monitored to determine whether to administer (i) multiple times; or (ii) is administered on the first day, (i) is administered intravenously on the second day, and then (ii) is administered multiple times at a certain frequency, while an *in-vivo* concentration of
(i) and a desired therapeutic efficacy endpoint are monitored to determine whether to administer (i) multiple times;
for example, (i) and (ii) are each administered once, and then (ii) is administered multiple times at a frequency of once every 3-4 days, once a week, once every two weeks, once every three weeks, or once every four weeks, while the *in-vivo* concentration of (i) and the desired therapeutic efficacy endpoint are detected to determine whether to administer (i) multiple times.

14. Use of the pharmaceutical combination according to any one of claims 8-13 in the treatment of a disease related to CLDN18.2 in a subject, comprising administering to the subject a therapeutically effective amount of the pharmaceutical combination according to any one of claims 8-13, wherein preferably, the disease related to CLDN18.2 is, for example, a cancer expressing or overexpressing CLDN 18.2, such as a CLDN18.2-positive solid tumor.

15. Use of the pharmaceutical combination according to any one of claims 8-13 in the preparation of a medicament for treating a disease related to CLDN18.2, wherein the disease related to CLDN18.2 is, for example, a cancer expressing or overexpressing CLDN 18.2, such as a CLDN18.2-positive solid tumor.

16. A method for treating a disease related to CLDN18.2, comprising administering to a subject a therapeutically effective amount of the pharmaceutical combination according to any one of claims 8-13, wherein the disease related to CLDN18.2 is, for example, a cancer expressing or overexpressing CLDN 18.2, such as a CLDN18.2-positive solid tumor.

17. A kit of parts, comprising the pharmaceutical combination according to any one of claims 8-13, wherein preferably, the kit of parts is in the form of a pharmaceutical dose unit.

18. A pharmaceutical complex, formed by
(i) an immune effector cell (e.g., a T cell) expressing the molecular switch-regulated CAR polypeptide according to any one of claims 1-3; and
(ii) an antibody or an antigen-binding fragment specifically binding to a CLDN18.2 molecule and comprising a P329G mutation (also referred to as a P329G mutated antibody), wherein, for example, the P329G mutated antibody comprises a mutated Fc domain, wherein the amino acid at position P329 according to the EU numbering is mutated to glycine (G), and the binding of the mutated Fc domain to an Fcy receptor is reduced compared to the binding of an unmutated parent antibody Fc domain to the Fcy receptor;
wherein the complex is formed by binding of the humanized anti-P329G mutation scFv sequence in the extracellular domain of the CAR polypeptide in the immune effector cell to the Fc domain of the P329G mutated antibody;
for example, the immune effector cell is a T cell expressing the molecular switch-regulated CAR polypeptide according to any one of claims 1-3 prepared from an autologous T cell or an allogeneic T cell, e.g., the immune effector cell is a T cell expressing the molecular switch-regulated CAR polypeptide according to any one of claims 1-3 prepared from a T cell isolated from a human PBMC;
for example, the P329G mutated antibody is an HB37A6 PG Ab, an Hz69H9 PG Ab, an Hz69H9-1.2 PG Ab, an Hz69H9-2.1 PG Ab, an Hz69H9-SA PG Ab, and/or an Hz3G3 PG Ab.

19. Use of the pharmaceutical complex according to claim 18 in the treatment of a disease related to CLDN18.2 in a subject, wherein preferably, the disease related to CLDN18.2 is, for example, a cancer expressing or overexpressing CLDN 18.2, such as a CLDN18.2-positive solid tumor.
